(19) Europäisches Patentamt
European Patent Office
Office européen des brevets

(11) **EP 3 782 609 B1**

(12) **EUROPEAN PATENT SPECIFICATION**

(45) Date of publication and mention of the grant of the patent:
**01.11.2023 Bulletin 2023/44**

(21) Application number: **20176250.7**

(22) Date of filing: **25.05.2020**

(51) International Patent Classification (IPC):
*A61K 9/14* (2006.01)    *A61K 9/16* (2006.01)
*A61K 36/63* (2006.01)    *A61K 36/258* (2006.01)
*A61K 36/484* (2006.01)    *A61K 36/539* (2006.01)

(52) Cooperative Patent Classification (CPC):
**A61K 36/258; A61K 9/1623; A61K 9/1682;**
**A61K 9/1694; A61K 36/484; A61K 36/539;**
**A61K 36/63;** A61K 2236/00

(54) **PELLET FORMULATION CONTAINING SINGLE OR COMPLEX HERBAL EXTRACT AT HIGH CONCENTRATION AND MANUFACTURING METHOD THEREFOR**

PELLETFORMULIERUNG MIT EINZELNEM ODER KOMPLEXEM KRÄUTEREXTRAKT IN HOHER KONZENTRATION UND HERSTELLUNGSVERFAHREN DAFÜR

FORMULATION DE PASTILLES CONTENANT UN EXTRAIT D'HERBES SIMPLE OU COMPLEXE À HAUTE CONCENTRATION ET SON PROCÉDÉ DE FABRICATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.08.2019 KR 20190102483**

(43) Date of publication of application:
**24.02.2021 Bulletin 2021/08**

(73) Proprietor: **Ku, Tae Hun**
**Busan 46232 (KR)**

(72) Inventors:
• **KU, Tae Hun**
  **Busan 46232 (KR)**
• **YOON, Chil-Surk**
  **Uiwang-si Gyeonggi-do 16042 (KR)**

(74) Representative: **Plougmann Vingtoft a/s**
**Strandvejen 70**
**2900 Hellerup (DK)**

(56) References cited:
**WO-A1-2008/035354    CN-A- 107 744 510**
**CN-A- 109 925 296    KR-A- 20070 118 020**
**US-A1- 2018 064 156**

• **LUCIMARA BENELLI ET AL: "Fluid bed drying and agglomeration of phytopharmaceutical compositions", POWDER TECHNOLOGY, vol. 273, 1 March 2015 (2015-03-01), pages 145-153, XP055748579, Basel (CH) ISSN: 0032-5910, DOI: 10.1016/j.powtec.2014.12.022**
• **GYU-HEE LEE ET AL: "Production of Spherical Granule from Viscous Red Ginseng Extracts for Improving Product Fluency and Preservation and Its Physicochemical Properties", JOURNAL OF FOOD SCIENCE, vol. 74, no. 9, 1 November 2009 (2009-11-01), pages E519-E525, XP055748581, US ISSN: 0022-1147, DOI: 10.1111/j.1750-3841.2009.01361.x**
• **Lucimara Benelli ET AL: "Fluid bed drying and agglomeration of phytopharmaceutical compositions", Powder Technology, vol. 273, 1 March 2015 (2015-03-01), pages 145-153, XP055748580, Basel (CH) ISSN: 0032-5910, DOI: 10.1016/j.powtec.2014.12.022**

Remarks:
The file contains technical information submitted after the application was filed and not included in this specification

**Description**

BACKGROUND OF THE INVENTION

1. Field of the invention

The present disclosure relates to a method for manufacturing a pellet formulation containing a single or complex herbal extract.

2. Description of the Prior Art

Herbal medicines are drugs that are mainly prepared from plant medicinal herbs according to the principles of oriental medicine and prescription drugs that are formulated by combining plant medicinal herbs to be suitable for the treatment of disease symptoms. Herbal medicines are largely classified according to the formulating manner into: decoctions by a medicinal-herbs-in-package type of prescription, which is most general; single herbal extracts obtained by extracting, concentrating, and drying one kind of medicinal herb and then adding a plurality of excipients thereto, followed by granulation or powdering, for convenient dosing and long-term storage through change of a formulation; mixed herbal extracts made of single herbal extracts corresponding to constituent medicinal herbs of a prescription; and a complex herbal extract obtained by mixing, extracting, concentrating, and drying together constituent medicinal herbs of a prescription and adding a plurality of excipients thereto, followed by granulation or powdering. The complex herbal extracts may be differentiated by *gamibang* in which important constitutional medicinal herbs are added or removed.

In western medicine, a patient can receive prescribed drugs in a pharmacy immediately after receiving a prescription from a doctor. In oriental medicine, a patient usually receives drugs 3-5 days after the treatment since a prescription established after the treatment is usually passed through a common decocting room, and the drugs have low convenience in carrying and dosing.

Therefore, some measures for modernizing the prescriptions of herbal medicines and the manufacturing process of formulations, providing methods for drug efficacy enhancement through the adjustment of the contents of marker components contained in medicinal herbs, and offering the convenience in carrying and dosing have been recently proposed, but such measures are still insufficient, and thus much development is urgently required.

Spray drying, which is a physical method for making extract solid powders, is a drying method in which a liquid sample is dried over high-temperature hot air while being sprayed as atomized liquid droplets with a size of several tens to several hundreds of micrometers (10-200 $\mu$m), and thus made into a powder product, wherein the time of drying is approximately 5-30 s. Spray drying accounts for 80% or more of powdering methods. However, the spray-dried powder has problems of causing a lot of dust, being poor in flowability, being prone to moisture absorption or oxidation, and sticking together to forming a mass, during a subsequent treatment process or in handling by a user. In particular, plant extract solid powders have very high hygroscopicity due to a high content of carbohydrates containing sugar components.

The extract powders dried by hot-air spray drying are produced by necessarily adding a drying aid agents in the range of 30-60% due to the characteristics of plant extracts. Additionally, 10-40% of excipients are further added in the manufacture of microgranules, granules, and pellets using the extract powders as base materials, resulting in a long processing time, low content of marker components, and especially many difficulties in handling raw materials due to hygroscopicity in the summer season.

Glycyrrhizae Radix, Notopterygii Rhizoma, Lonicerae Flos, Platycodi Radix, Jujubae Fructus, Thujae Resina, Rehmanniae Radix, Anemarrhenae Rhizoma, Fraxini Cortex, and the like are medicinal herbs that are difficult to formulate with extracted solid powders due to high sugar contents thereof.

In spray drying, from the industrial aspect of securing economic efficiency in the production of plant extract powders, flowability and stickiness of dried powders are important factors, and these factors influence efficient productivity of the dry powders and ease of packaging and handling thereof. Especially, various saccharide components (glucose, fructose, sucrose, etc.) and various organic acids contained at high content in plant material extracts, have low molecular weights, and thus such components are serious obstacles to spray drying since they become sticky in spray drying due to low glass transition temperature (Tg) characteristics thereof. Therefore, a drying aid agent with a high glass transition temperature is essentially added in spray drying of plant extracts.

Commercial powders are usually produced by adding, as drying aid agents, carbohydrates with a high molecular weight, i.e., various starches, modified starches, dextrins, solid corn syrups, gum arabic, cyclodextrin, and the like, in the range of 30-70% relative to the dried solid content to increase glass transition temperatures. While DE 36 maltodextrin can be used as a drying aid agent to increase the drying temperature to 100°C, DE 5 maltodextrin can be used to increase the drying temperature to 188°C, thereby improving production efficiency.

To manufacture dry powders with an increased extract powder content, the prior art has suggested a method of extremely restricting the amount of an excipient, but such a method cannot increase the drying temperature relevant to the glass transition temperature in spray drying, resulting in extremely poor production efficiency, and thus is impossible to utilize industrially. As a result, the produced extract dry powders may necessarily have an excipient content in the range of 30-60%.

In addition, *Zizyphus jujuba, Rhemannia glutinosa, Panax ginseng,* or a red ginseng concentrate powder having a high sugar content easily deteriorates in quality stability at room temperature due to hygroscopicity thereof, and has very low powder flowability due to high hygroscopicity, so that it is very difficult to conduct tableting and capsule filling in the productization procedure. The products manufactured by adding other excipients in large quantities in order to overcome these have a disadvantage of low content of active ingredients.

As for the pellets manufactured by extrusion and spheronization (E/S), which are favorably applied to the manufacturing method for spherical pellets in western medicines, an increased content of water used in the mixing of materials increases dampening mass characteristics, i.e., compressed plastic mass formability, with the result that a flow passing through a die for extrusion is compressed, and thus particles stick to cylindrical rods. The pellets manufactured of microcrystalline cellulose and lactose have a hardness in the range of 0.48-0.73 kgf. In the pellets manufactured of microcrystalline cellulose and lactose, the lactose acts as a binder when absorbing moisture, and therefore, the reduction of the lactose content results in a decrease in hardness of the pellets. The friability decreases as the density of the pellets increases, and the friability decreases as the compressive pressure increases. It is known that a friability of 0.71% or less is appropriate.

In E/S, the friability decreases as the compressive pressure increases. The screw pressure in extrusion is higher than those in the other methods, and thus the pellets manufactured by extrusion tend to show increased hardness and density, decreased friability, and somewhat delayed dissolution rates. According to another report, sugar spheres with a diameter of 0.5-0.6 mm had a hardness of 0.09 kgf$\pm$ 0.04 and a friability of 3.72%$\pm$0.06, showing a very high level of friability. The appropriate level of friability was 1.7% or less in this report.

In general, the friability rate is determined by measuring the weight of the powder separated after rotation at a rate of 100-200 rpm for 5-10 min in a friability rate tester. However, in the present disclosure, the spray pressure of a preparation solution is in the range of 2.0-4.0 kg/cm$^2$ in an actual fluidized bed process, and thus seeds are placed under very highly severe conditions. As a result, small broken particles of the seeds stick to external portions of final products, or pellets are broken in half during the fluidized bed process, causing fatal damage to appearance quality of finished products.

When spherical pellets of starch-containing sugar spheres and cellulose are used as seeds, the seeds undergo friction and collisions by the spray air pressure during the process in a fluidized bed apparatus, and the seeds are broken due to weak hardness, resulting in an increase in friability. Moreover, the use of extract dry powders has a problem in that the contents of herbal extract solids or marker components contained in final products get gradually smaller since various kinds of excipients are added in the manufacture of powders in a spray drier, the preparation of seeds, and the preparation of a preparation solution for fluidized bed spraying.

When, for use as basic seeds in a fluidized bed apparatus, plant herbal extract dry powders, extract concentrates, and excipients are mixed, and made into an indeterminate form of granular pellets by using a high shear mixing and granulator or vertical granulator or made into granules by using a conventional extruder, the resultant extrudate, unlike an extrudate for western medicines, has very strong stickiness and thus is lengthily extruded, like noodles, without being naturally easily cut. When such an extrudate is ground to be made into spherical pellets in a spheronizer, the spherical pellets are made into a rectangular cylindrical shape with both edges worn. In cases where such pellets are used as seeds in a fluidized bed apparatus, the final shape is impossible to spheronize regardless of coating with the same kind of extract.

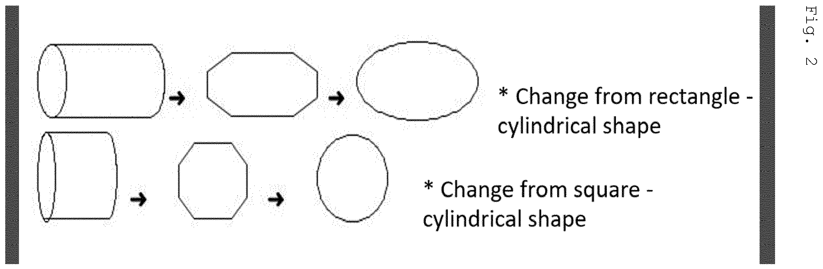

Therefore, when the resultant product formed in the form of rod-like granules or linear granules is ground in a spheronizer according to a conventional pellet manufacturing method, complete spherical pellets cannot be produced as shown in the examples of shape of FIG. 2. When such pellets are introduced into a fluidized bed apparatus and an extract is sprayed and coated, final products have non-uniform shapes, deteriorating in marketability. Moreover, the rod-like granules and the like manufactured by a high shear mixing and granulator or vertical granulator have weak hardness, and thus seeds are broken in the subsequent fluidized bed coating process, and granules with small broken particles sticking thereto result in a lack of uniformity of the entire appearance and marketability.

In oriental medicine, as for single herbal medicines considering the presence of an aroma component according to the characteristics of raw materials and complex herbal medicines having a high content of an aromatic single herbal medicine, no consideration was given to the presence or absence of the aroma component in the final formulation or the transfer rate of the aroma component to the final formulation in the conventional art. Herbal decoction extracts are medicines with high stability since the experience of dosing is ensured for a long period of time, and according to the Qi-taste theory, which is considered as the basis of oriental medicine theories, there exists justification that aroma or smell components should be contained in final products, and the efficacy of the herbal decoction extracts has been proved in even modern aroma therapy using aromas.

The conventional pellet formulation manufacturing methods did not consider physical characteristics, such as the content of sugar contained in an extract and viscosity of the extract, and thus, the viscosity continuously increases and heat is generated during the manufacture of pellets by common stirring and extrusion. As a result, a kneaded product that has not been extruded is hardened to cause instant mechanical resistance, and thus a screw may be stopped and screen holes may be clogged, and at last, a granulator may become inoperable.

## SUMMARY OF THE INVENTION

The present inventors made an effort to develop a method for manufacturing a pellet formulation containing a single or complex herbal extract at a high concentration. As a result, the present inventors developed: a method for manufacturing a pellet formulation, which contains a single or complex herbal extract at a high concentration and allows the identification of the contents of extract solids and marker components, by segmenting and classifying characteristics of original material extracts on the basis of the sugar content and optimizing a variety of associated factors; and a method for manufacturing a pellet formulation containing a capsule having an essential oil encapsulated therein, and as a result, completed the present disclosure.

Therefore, an aspect of the present disclosure is to provide a method for manufacturing a pellet formulation containing a single or complex herbal extract. The invention is set out in the appended set of claims.

In accordance with an aspect of the present disclosure, there is provided a method for manufacturing a pellet formulation containing a single or complex herbal extract, the method comprising:

> (a) preparing a concentrate for seeds and a concentrate for fluidized bed coating from a single or complex herbal extract, wherein the concentrate for fluidized bed coating having a lower sugar content than the concentrate for seeds;
> (b) molding a seed by mixing the concentrate for seed and an excipient; and
> (c) coating surfaces of the seeds by mixing the concentrate for fluidized bed coating and an excipient,

and wherein the excipient is at least one kind selected from the group consisting of precipitated calcium carbonate (precipitated $CaCO_3$), magnesium oxide (MgO), calcium silicate ($CaSiO_3$), magnesium silicate ($3MgSiO_3 \cdot 5H_2O$), silicon dioxide ($SiO_2$), titanium dioxide ($TiO_2$), bentonite, kaolin, talc, CMC-Ca, casein, dextrin, hydroxypropyl methylcellulose (HPMC), pullulan, microcrystalline cellulose (MCC), lactose, stearic acid-magnesium (stearate-Mg), stearic acid-calcium (stearate-Ca), PEG 6000, beta-cyclodextrin powder, zein powder, starch sodium octenyl succinate (SSOC), and sucrose fatty acid esters.

The present inventors made an effort to develop a method for manufacturing a pellet formulation containing a single or complex herbal extract at a high concentration. As a result, the present inventors developed a method for manufacturing a pellet formulation, which contains a single or complex herbal extract at a high concentration and allows the identification of the contents of extract solids and marker components, by segmenting and classifying characteristics of original material extracts on the basis of the sugar content and optimizing a variety of associated factors.

Hereinafter, a method of the present disclosure for manufacturing a pellet formulation containing a single or complex herbal extract at a high concentration will be described by steps.

(a) Preparing a concentrate for seeds and a concentrate for fluidized bed coating from a single or complex herbal extract.

A method for manufacturing a pellet formulation of the present disclosure is largely composed of a step for preparing a concentrate for seeds and a concentrate for fluidized bed coating, molding seeds and coating surfaces of the seeds.

As used herein, the term seed refers to a small particle that is a core of a pellet in the manufacture of a pellet formulation of the present disclosure. While the seed is coated with an extract, an excipient, and the like, the particle is grown to a desired size, so that a pellet is manufactured.

For the preparation of seeds and the coating of seeds, a single or complex herbal extract is concentrated into a concentrate for seeds and a concentrate for fluidized bed coating under different concentration conditions, respectively.

As used herein, the term "single herbal medicine" refers to one kind of medicinal herb, including (i) plant herbs and (ii) roots, barks, flowers, fruits, seeds, or rhinoceros horns, which are natural products that are used *per se* as a drug product or used as a raw material for medicines.

The single herbal medicine in the present disclosure includes Puerariae Radix, Chrysanthemi Flos, Glycyrrhizae Radix, Notopterygii Rhizoma, Zingiberis Rhizoma Siccus, Cinnamomi Ramulus, Agastachis Herba, Trichosanthis Semen, Lonicerae Flos, Platycodi Radix, Angelicae Gigantis Radix, Jujubae Fructus, Rhei Rhizoma, Persicae Semen, Angelicae Pubescentis Radix, Ephedrae Herba, Viticis Fructus, Liriopes Radix, Hordei Fructus Germiniatus, Moutan Cortex, Aucklandiae Radix, Menthae Herba, Pinelliae Rhizoma, Saposhnikovia Radix, Thujae Resina, Atractylodis Rhizoma Alba, Poria (Hoelen), Amomi Fuctus, Crataegii Fructus, Scirpi Rhizoma, Mori Radicis Cortex, Zingiberis Rhizoma Recens, Rehmanniae Radix, Asari Herba Cum Radix, Rehmanniae Radix Preparat, Cimicifugae Rhizoma, Bupleuri Radix, Massa Medicata Fermentata, Zedoariae Rhizoma, Forsythiae Fructus, Schizandrae Fructus, Cinnamomi Cortex Spissus, Ginseng Radix, Artemisiae Capillaris Herba, Perilla Folium, Paeoniae Radix, Peucedani Radix, Fritillariae Thunbergii Bulbus, Aurantii Fructus Pericarpium, Anemarrhenae Rhizoma, Aurantii Immaturus Fructus, Fraxini Cortex, Atractylodis Rhizoma, Ligustici Rhizoma, Gastrodiae Rhizoma, Citrii Unshiu Immaturi Pericarpium, Gardeniae Fructus, Alismatis Rhizoma, Armeniacae Semen, Cyperi Rhizoma, Schizonepetae Spica, Scutellariae Radix, Astragali Radix, Coptidis Rhizoma, Phellodendri Cortex, and Magnoliae Cortex, but is not limited thereto.

As used herein, the term "complex herbal medicine" is composed of one or more kinds of medicinal herbs, and the complex herbal medicine contains individual medicinal compositions by an herbal medicine manufacturing instruction or a prescription. The complex herbal medicine may include a combination of single herbal medicines and individual medicinal compositions in any appropriate manner, such as a combination of one or more kinds of single herbal medicines and a combination of one or more kinds of single herbal medicines and an individual medicinal composition by one or more prescriptions.

The individual medicinal composition in a prescription, as a complex herbal medicine in the present disclosure, includes *Gamisoyosan, Galgeun-tang, Galgeunhaegi-tang, Gumigangh wal-tang, Dangguiyukhwang-tang, Dashiho-tang, Daecheongryong-tang, Daehwajungeum, Daehwangmokdanpi-tang, Doinseunggi-tang, Mahwang-tang, Banhabaekchulcheonma-tang, Banhasasim-tang, Banhahubak-tang, Baekchul-tang, Bojungikgi-tang, Bohe-tang, Bokryeongbosim-tang, Bulhwangeumjeonggisan, Samsoum, Samchulgeonbi-tang, Saengmaeksan, Sosiho-tang, Yeongyangjihwang-tang, Palmul-tang, and Pyeongwisan,* but is not limited thereto.

In the present disclosure, step (a) may further include an extraction step for obtaining the single or complex herbal extract. The extraction may employ various extraction methods commonly known in the art.

As used herein, the term "extract" refers to a solvent crude extract, a particular solvent-soluble extract (solvent fraction), and a solvent fraction of a solvent crude extract, for the single or complex herbal medicine.

The extract may be a crude extract obtained by subjecting a single herbal medicine and/or a complex herbal medicine to extraction with at least one solvent selected from the group consisting of water and a straight-chain or branched alcohol having 1 to 4 carbon atoms, and for example, the extract may be a crude extract obtained by extraction with water as a solvent.

When a mixture of water and an alcohol is used as a solvent used in the preparation of the crude extract of the present disclosure, an aqueous solution of a straight-chain or branched alcohol having 1 to 4 carbon atoms may be used in 10 or more and less than 100 %(v/v), 20 or more and less than 100 %(v/v), 30 or more and less than 100 %(v/v), 40 or more and less than 100 % (v/v), 50 or more and less than 100 %(v/v), 60 or more and less than 100 %(v/v), or 70 or more and less than 100 % (v/v) .

In addition, the aqueous solution of an alcohol may be one kind selected from the group consisting of an aqueous solution of methanol, an aqueous solution of ethanol, an aqueous solution of propanol, and an aqueous solution of butanol.

The extract according to the present disclosure may be a solvent fraction obtained by fractionating the solvent crude extract with an additional solvent, and for example, may be a solvent fraction of the solvent crude extract, obtained by using at least one kind of solvent selected from the group consisting of ethyl ether, ethyl acetate, and butanol.

For example, the extract may be a solvent fraction obtained by fractionating a solvent crude extract with at least one kind of solvent selected from the group consisting of ethyl ether, ethyl acetate, and butanol, the solvent crude extract being obtained by subjecting the single herbal medicine and/or complex herbal medicine to extraction with at least one solvent selected from the group consisting of water and a straight-chain or branched alcohol having 1 to 4 carbon atoms.

According to the present disclosure, the extract may be obtained by adding water as a solvent to a single herbal medicine and/or complex herbal medicine and immersing the medicine in the water.

As used herein, the term "extract solid" refers to a solid material contained in the extract, and the extract solid may be obtained by drying, for example, spray-drying the extract, but is not limited thereto.

According to the present disclosure, the method may further include, before step (a), a step of measuring the sugar content of the single herbal medicine or complex herbal medicine.

A single herbal medicine or complex herbal medicine is sorted according to the sugar content of a 10-fold water extract of the single herbal medicine or complex herbal medicine, and then a concentrate for seeds and a concentrate for fluidized bed coating having a lower sugar content than the concentrate for seeds are prepared.

When a 10-fold water extract of the single or complex herbal medicine has a sugar content of 5.5 or more and less than 10.0 Brix, the concentrate for seeds in step (a) may be prepared by concentrating the single or complex herbal extract to a sugar content of 45-65 Brix.

When a 10-fold water extract of the single or complex herbal medicine has a sugar content of 5.5 or more and less than 10.0 Brix, the concentrate for fluidized bed coating in step (a) may be prepared by concentrating the single or complex herbal extract to a sugar content of 15-35 Brix.

According to the present disclosure, the single herbal medicine, of which a 10-fold water extract has a sugar content of 5.5 or more and less than 10.0 Brix, may include Glycyrrhizae Radix, Notopterygii Rhizoma, Lonicerae Flos, Platycodi Radix, Angelicae Gigantis Radix, Jujubae Fructus, Moutan Cortex, Saposhnikovia Radix, Thujae Resina, Atractylodis Rhizoma Alba, Crataegii Fructus, Rehmanniae Radix, Ginseng Radix, Anemarrhenae Rhizoma, Fraxini Cortex, and Scutellariae Radix, and the complex herbal medicine, of which a 10-fold water extract has a sugar content of 5.5 or more and less than 10.0 Brix, may include *Gumiganghwal-tang* and *Yeongyangjihwang-tang,* but are not limited thereto.

When a 10-fold water extract of the single or complex herbal medicine has a sugar content of 3.0 or more and less than 5.5 Brix, the concentrate for seeds in step (a) may be prepared by concentrating the single or complex herbal extract to a sugar content of 40-60 Brix.

When a 10-fold water extract of the single or complex herbal medicine has a sugar content of 3.0 or more and less than 5.5 Brix, the concentrate for fluidized bed coating in step (a) may be prepared by concentrating the single or complex herbal extract to a sugar content of 25-45 Brix.

According to the present disclosure, the single herbal medicine, of which a 10-fold water extract has a sugar content of 3.0 or more and less than 5.5 Brix, may include Angelicae Pubescentis Radix, Ephedrae Herba, Liriopes Radix, Aucklandiae Radix, Zingiberis Rhizoma Recens, Cimicifugae Rhizoma, Schizandrae Fructus, Paeoniae Radix, Aurantii Fructus Pericarpium, Atractylodis Rhizoma, Gastrodiae Rhizoma, Citrii Unshiu Immaturi Pericarpium, Gardeniae Fructus, Astragali Radix, and Coptidis Rhizoma, and the complex herbal medicine, of which a 10-fold water extract has a sugar content of 3.0 or more and less than 5.5 Brix, may include Galgeun-tang, Dangguiyukhwang-tang, Daehwajungeum, Mahwang-tang, Banhasasim-tang, Baekchul-tang, Bojungikgi-tang, Bohe-tang, Bulhwangeumjeonggisan, Samsoum, Samchulgeonbi-tang, Saengmaeksan, Sosiho-tang, Palmul-tang, and Pyeongwisan, but are not limited thereto.

When a 10-fold water extract of the single or complex herbal medicine has a sugar content of 0.01 or more and less than 3.0 Brix, the concentrate for seeds in step (a) may be prepared by concentrating the single or complex herbal extract to a sugar content of 30-60 Brix.

When a 10-fold water extract of the single or complex herbal medicine has a sugar content of 0.01 or more and less than 3.0 Brix, the concentrate for fluidized bed coating in step (a) may be prepared by concentrating the single or complex herbal extract to a sugar content of 20-35 Brix.

According to the present disclosure, the single herbal medicine, of which a 10-fold water extract has a sugar content of 0.01 or more and less than 3.0 Brix, may include Puerariae Radix, Chrysanthemi Flos, Zingiberis Rhizoma Siccus, Cinnamomi Ramulus, Agastachis Herba, Trichosanthis Semen, Rhei Rhizoma, Persicae Semen, Viticis Fructus, Hordei Fructus Germiniatus, Menthae Herba, Pinelliae Rhizoma, Poria(Hoelen, Amomi Fuctus, Scirpi Rhizoma, Mori Radicis Cortex, Asari Herba Cum Radix, Bupleuri Radix, Massa Medicata Fermentata, Zedoariae Rhizoma, Forsythiae Fructus, Cinnamomi Cortex Spissus, Artemisiae Capillaris Herba, Perilla Folium, Peucedani Radix, Fritillariae Thunbergii Bulbus, Aurantii Immaturus Fructus, Ligustici Rhizoma, Alismatis Rhizoma, Armeniacae Semen, Cyperi Rhizoma, Schizonepetae Spica, Phellodendri Cortex , and Magnoliae Cortex, and the complex herbal medicine, of which a 10-fold water extract has a sugar content of 0.01 or more and less than 3.0 Brix, may include Gamisoyosan, *Galgeunhaegi-tang, Dashiho-tang, Daecheongryong-tang, Daehwangmokdanpi-tang, Doinseunggi-tang, Banhabaekchulcheonma-tang, Banhahubak-tang,* and *Bokryeongbosim-tang,* but are not limited thereto.

In oriental medicine, the presence of aroma components is important according to characteristics of raw materials, but in some cases, a significant amount of aroma components are volatilized during extraction and concentration, with the result that the extract and concentrate thus obtained may contain few aroma components.

In order to prevent the loss of aroma components contained in raw materials, when the single or complex herbal extract has aromaticity, the method for manufacturing a pellet formulation of the present disclosure may further include: a step of obtaining an essential oil volatilized during extraction and concentration; and a step of encapsulating the essential oil.

The essential oil may be obtained by subjecting the single or complex herbal medicine to extraction and concentration in an extractor and concentrator equipped with essential oil encapsulating, condensing, and separating devices, and the obtained essential oil component may be encapsulated using alginic acid or beta-cyclodextrin, and added to the concentrate for fluidized bed coating in step (c).

The aromatic single herbal medicine may include Nardostachyos Rhizoma, Dalbergiae Odoriferae Lignum, Osterici Radix, Agastachis Herba, Cinnamomi Ramulus, Aucklandiae Radix, Saposhnikovia Radix, Alpiniae Japonicae Semen, Menthae Herba, Santali Albi Lignum, Moschi Moschus, Thymi Herba, Styrax Liquides, Lysimachiae Foeni-graeci Herba, Olibanum, Cinnamomi Cortex Spissus, Myristicae Semen powder, Pterocarpi Lignum, Syzygii Flos, Atractylodis Rhizoma, Aquilariae Resinatum Lignum, Helenii Radix, Pini Semen, and Sesami Oleum, but is not limited thereto.

(b) <u>Molding seeds</u>

Next, the concentrate for seeds and an excipient are mixed to mold seeds.

The excipient is at least one kind selected from the group consisting of precipitated calcium carbonate (precip-

itated CaCO$_3$), magnesium oxide (MgO), calcium silicate (CaSiO$_3$), magnesium silicate (3MgSiO$_3$·5H$_2$O), silicon dioxide (SiO$_2$), titanium dioxide (TiO$_2$), bentonite, kaolin, talc, CMC-Ca, casein, dextrin, hydroxypropyl methylcellulose (HPMC), pullulan, microcrystalline cellulose (MCC), lactose, stearic acid-magnesium (stearate-Mg), stearic acid-calcium (stearate-Ca), PEG 6000, beta-cyclodextrin powder, zein powder, starch sodium octenyl succinate (SSOC), and sucrose fatty acid ester.

According to the present disclosure, when a 10-fold water extract of the single or complex herbal medicine has a sugar content of 5.5 or more and less than 10.0 Brix in step (a), a concentrate for seeds, which contains 35-55 wt% of single or complex herbal extract solids relative to the total dry weight of the seeds, may be mixed with the balance of an excipient. The excipient may contain, relative to the total weight of excipients: 74-83 wt% of at least one kind of release agent selected from the group consisting of microcrystalline cellulose, lactose, stearic acid-magnesium, stearic acid-calcium, PEG 6000, beta-cyclodextrin powder, zein powder, starch sodium octenyl succinate; 15-25 wt% of at least one kind of mineral additive selected from the group consisting of precipitated calcium carbonate, magnesium oxide, calcium silicate, magnesium silicate, silicon dioxide, titanium dioxide, bentonite, kaolin, and talc; and 1-2 wt% of sucrose fatty acid ester as an emulsifier.

According to the present disclosure, when a 10-fold water extract of the single or complex herbal medicine has a sugar content of 3.0 or more and less than 5.5 Brix in step (a), a concentrate for seeds, which contains 45-65 wt% of single or complex herbal extract solids relative to the total dry weight of the seeds, may be mixed with the balance of an excipient. The excipient may contain, relative to the total weight of excipients: 78-88 wt% of at least one kind of release agent selected from the group consisting of microcrystalline cellulose, lactose, stearic acid-magnesium, stearic acid-calcium, PEG 6000, beta-cyclodextrin powder, zein powder, starch sodium octenyl succinate; 10-20 wt% of at least one kind of mineral additive selected from the group consisting of precipitated calcium carbonate, magnesium oxide, calcium silicate, magnesium silicate, silicon dioxide, titanium dioxide, bentonite, kaolin, and talc; and 1-2 wt% of sucrose fatty acid ester as an emulsifier.

According to the present disclosure, when a 10-fold water extract of the single or complex herbal medicine has a sugar content of 0.01 or more and less than 3.0 Brix in step (a), a concentrate for seeds, which contains 50-70 wt% of single or complex herbal extract solids relative to the total dry weight of the seeds, may be mixed with the balance of an excipient. The excipient may contain, relative to the total weight of excipients: 83-94 wt% of at least one kind of release agent selected from the group consisting of microcrystalline cellulose, lactose, stearic acid-magnesium, stearic acid-calcium, PEG 6000, beta-cyclodextrin powder, zein powder, starch sodium octenyl succinate; 5-15 wt% of at least one kind of mineral additive selected from the group consisting of precipitated calcium carbonate, magnesium oxide, calcium silicate, magnesium silicate, silicon dioxide, titanium dioxide, bentonite, kaolin, and talc; and 1-2 wt% of sucrose fatty acid ester as an emulsifier.

The step of mixing the concentrate for seeds and the excipient to mold seeds may include: a step of passing a mixture of the concentrate for seeds and the excipient through an extruder to cut an extrudate; and introducing the cut extrudate into a spheronizer to grind the cut extrudate.

Since the concentrate of the single or complex herbal extract usually has a high sugar content in the range of 20-70 Brix, a kneaded product obtained by mixing the extract with an excipient has increased stickiness due to sugar components. While the kneaded product is re-mixed and compressed by the rotation of a screw inside a cylinder of an extruder, the kneaded product has rapidly increased viscosity and generates heat due to friction inside the cylinder, causing the hardening of the contents, with the result screen holes may be clogged, or the kneaded product is lengthily extruded and thus are not easily cut.

In order to solve such problems, in the present disclosure, the mixture of the concentrate for seeds and the excipient is allowed to pass through an extruder equipped with an extrusion part disc having a hole length of 1.0-2.0 cm, 1.0-1.9 cm, 1.0-1.8 cm, 1.0-1.7 cm, 1.0-1.6 cm, 1.1-2.0 cm, 1.1-1.9 cm, 1.1-1.8 cm, 1.1-1.7 cm, 1.1-1.6 cm, 1.3-2.0 cm, 1.3-1.9 cm, 1.3-1.8 cm, 1.3-1.7 cm, 1.3-1.6 cm, 1.4-2.0 cm, 1.4-1.9 cm, 1.4-1.8 cm, 1.4-1.7 cm or 1.4-1.6 cm and a hole diameter of 1.0-2.0 mm, 1.0-1.9 mm, 1.0-1.8 mm, 1.0-1.7 mm, 1.0-1.6 mm, 1.1-2.0 mm, 1.1-1.9 mm, 1.1-1.8 mm, 1.1-1.7 mm, 1.1-1.6 mm, 1.3-2.0 mm, 1.3-1.9 mm, 1.3-1.8 mm, 1.3-1.7 mm, 1.3-1.6 mm, 1.4-2.0 mm, 1.4-1.9 mm, 1.4-1.8 mm, 1.4-1.7 mm, or 1.4-1.6 mm, and for example, an extrusion part disc having a hole length of 1.5 cm and a hole diameter of 1.5 mm, to thereby cut the mixture into an extrudate equal in diameter and length.

The cut extrudate is introduced into a spheronizer, followed by grinding, thereby manufacturing spherical pellets usable as basic seeds for the pellet formulation of the present disclosure. The seeds are used as cores for the pellet formulation of the present disclosure.

(c) Coating surfaces of the seeds

In the present step, the seeds produced in step (b) are introduced into a fluidized bed granulator to be fluidized, and a mixture of the concentrate for fluidized bed coating and an excipient is sprayed to grow the seeds into a pellet formulation.

According to the present disclosure, the seeds are introduced into the fluidized bed granulator, and then the mixture of the liquid-concentrate for fluidized bed coating and the excipient may be sprayed from the bottom portion of the fluidized bed granulator in a bottom-spray manner or from the top portion of the fluidized bed granulator in a top-spray manner. For example, the mixture may be sprayed from the bottom portion of the fluidized bed granulator in a bottom-spray manner.

In the fluidized bed coating process, when a preparation solution is sprayed, sugar components are concentrated through the volatilization of moisture, causing a sharp increase in stickiness, resulting in the agglomeration of pellet particles (aggregate formation). Therefore, the preparation solution to be sprayed is required to have appropriate viscosity and sticking strength, retain uniform spreadability on the particle surface, and prevent agglomeration by mitigating the sticking strength of sugar components. In the present disclosure, the sugar content of the concentrate used for fluidized bed coating is differently adjusted according to characteristics of medicinal herbs, and the kind of the excipient used and the additional amount thereof are adjusted, so that the concentrate is promptly dried and does not have excessive sticking strength by sugar components even though the liquid is concentrated.

According to the present disclosure, when a 10-fold water extract of the single or complex herbal medicine has a sugar content of 5.5 or more and less than 10.0 Brix in step (a), the excipient mixed with the concentrate for fluidized bed coating may be used in 10-15 wt% relative to single or complex herbal extract solids, which are contained in the concentrate for fluidized bed coating. The excipient may contain, relative to the total weight thereof: 40-50 wt% of at least one kind selected from the group consisting of precipitated calcium carbonate, stearic acid-magnesium, and PEG 6000; 5-15 wt% of at least one kind selected from the group consisting of HPMC, pullulan, and glutinous rice starch; 35-45 wt% of starch sodium octenyl succinate; and 2-8 wt% of sucrose fatty acid ester.

According to the present disclosure, when a 10-fold water extract of the single or complex herbal medicine has a sugar content of 3.0 or more and less than 5.5 Brix in step (a), the excipient mixed with the concentrate for fluidized bed coating may be used in 5-10 wt% relative to single or complex herbal extract solids, which are contained in the concentrate for fluidized bed coating. The excipient may contain, relative to the total weight thereof: 15-25 wt% of at least one kind selected from the group consisting of precipitated calcium carbonate, stearic acid-magnesium, and PEG 6000; 25-35 wt% of at least one kind selected from the group consisting of HPMC, pullulan, and glutinous rice starch; 35-45 wt% of starch sodium octenyl succinate; and 2-8 wt% of sucrose fatty acid ester.

According to the present disclosure, when a 10-fold water extract of the single or complex herbal medicine has a sugar content of 0.01 or more and less than 3.0 Brix in step (a), the excipient mixed with the concentrate for fluidized bed coating may be used in 3-8 wt% relative to single or complex herbal extract solids, which are contained in the concentrate for fluidized bed coating. The excipient may contain, relative to the total weight thereof: 5-15 wt% of at least one kind selected from the group consisting of precipitated calcium carbonate, stearic acid-magnesium, and PEG 6000; 50-70 wt% of at least one kind selected from the group consisting of HPMC, pullulan, and glutinous rice starch; 35-45 wt% of starch sodium octenyl succinate; and 2-8 wt% of sucrose fatty acid ester.

According to the present disclosure, the method may further include, after step (c), a step of coating a surface of the pellet formulation produced in step (c).

In the coating step, various moisture-proof coating materials known in the art may be selected and used.

The moisture-proof coating material includes starch, gelatin, natural edible dyes, artificial edible dyes, cellulose, biodegradable polymers, biodegradable oligomers, emulsifying wax, shellac, flavoring agents, hydrophobic agents or hydrophilic agents, lipids, proteins, and minerals. As the moisture-proof coating material, at least one kind selected from the group consisting of hydroxy propyl methylcellulose, hydroxypropyl cellulose, ethylcellulose, methylcellulose, titanium dioxide, polyethylene glycol, triethyl citrate, triacetin, dibutyl sebacate, polymethacrylate, cellulose acetate phthalate, carnauba wax, candelilla wax, petroleum wax, beeswax, and hydrogenated fat may be used, and for example, shellac or ethyl cellulose may be used, but is not limited thereto.

The moisture-proof coating material may be used in 1-5 wt%, 1-4 wt%, or 1-3 wt%, and for example 2 wt%, relative to the sum of the weight of the seeds introduced into the fluidized bed granulator and the dry weight of the mixture of the concentrate for fluidized bed coating and the excipient, which has been sprayed on the surfaces of the seeds, in step (c).

The surface of the pellet formulation produced in step (c) of the present disclosure is coated with a moisture-proof material, thereby improving stability in storage and safety in the distribution procedure.

According to the present disclosure, when the pellet formulation is manufactured by the method of the present disclosure, the contents of solids and marker components in herbal extracts can be monitored. Therefore, an herbal medicinal prescription can be easily configured by combining pellet formulations manufactured by the method of the present disclosure, with the results that the content of the medicine contained in finally administered extract solids can be easily adjusted, and thus by raising or lowering the content of a particular marker component in the herbal medicinal prescription, a prescription can be easily adjusted in response to the degrees of personal disease symptoms and the personal constitution. Furthermore, in the event of side effects, the accurate understanding of causes thereof and the derivation of remedies are easily attained.

The following aspects are not according to the invetion and are present for illustration purposes only. The present disclosure provides a pellet formulation containing a single or complex herbal extract, the pellet formulation being manufactured by the method of the present disclosure.

Since the pellet formulation containing a single or complex herbal extract of the present disclosure is manufactured by the manufacturing method of the present disclosure, the descriptions of overlapping contents therebetween will be omitted to avoid excessive complication of the specification due to repetitive descriptions thereof.

The pellet formulation manufactured by the method of the present disclosure may have a spherical or oval shape, and for example, a spherical shape.

The pellet formulation containing a single or complex herbal extract, manufactured by the method of the present disclosure, contains 55-95 wt% of extract solids in an extract, which is an active ingredient showing a pharmaceutical effect.

Specifically, a pellet formulation manufactured using a single or complex herbal extract contains 55-80 wt% of extract solids of a single or complex herbal medicine when a 10-fold water extract of the single or complex medicine has a sugar content of 5.5 or more and less than 10.0 Brix; a pellet formulation manufactured using a single or complex herbal extract contains 60-90 wt% of extract solids of a single or complex herbal medicine when a 10-fold water extract of the single or complex medicine has a sugar content of 3.0 or more and less than 5.5 Brix; and a pellet formulation manufactured using a single or complex herbal extract contains 70-95 wt% of extract solids of a single or complex herbal medicine when a 10-fold water extract of the single or complex medicine has a sugar content of 0.01 or more and less than 3.0 Brix.

The pellet formulation of the present disclosure can be utilized for a pharmaceutical composition, a food composition, a health functional food, an herbal medicinal product, and various basic processed products.

The pellet formulation of the present disclosure may be used as a non-prescription drug purchasable without a prescription, a botanical drug, an herbal medicine, a homeopathic agent supplier, or a supplement, and may be contained as a nutritional component in a food, a functional food, a beverage, bar, a food additive, a medicinal food, a dietary supplement, or an herb product.

The present disclosure relates to a method for manufacturing a pellet formulation containing a single or complex herbal extract. The invention is set out in the appended set of claims. According to the present disclosure, a method for manufacturing a pellet formulation, which has a high content of extract solids and attain improved production efficiency, was completed by segmenting and classifying characteristics of original material extracts on the basis of the sugar content and optimizing a variety of associated factors in the manufacture of a pellet formulation through the preparation of seeds and a fluidized bed coating process for coating the prepared seeds.

When a pellet formulation is manufactured using the manufacturing method of the present disclosure, the contents of solids and marker components in herbal extracts can be monitored, and an herbal medicinal prescription can be easily configured by combining the manufactured pellet formulations. Furthermore, the content of the extract solids administered can be easily adjusted, and thus by raising or lowering the content of a particular marker component in the herbal medicinal prescription, a prescription can be easily adjusted in response to the degrees of personal disease symptoms and the personal constitution, and in the event of side effects, the accurate understanding of causes thereof and the derivation of remedies are easily attained.

BRIEF DESCRIPTION OF THE DRAWINGS

The above and other aspects, features and advantages of the present disclosure will be more apparent from the following detailed description taken in conjunction with the accompanying drawings, in which:

FIG. 1A illustrates an image showing that spherical pellets of starch-containing sugar spheres and cellulose are used as seeds to manufacture pellets;

FIG. 1B illustrates an image showing pellets manufactured by a method for manufacturing a pellet formulation of the present disclosure;

FIG. 2 is an exemplary diagram showing extrudates used in the preparation of seeds and pellet shapes after grinding;

FIG. 3 shows an extruder die mold part of an extruder used in the method for manufacturing a pellet formulation of the present disclosure;

FIG. 4A illustrates images showing seeds produced by an extruder equipped with a conventional dome-type screen and a shape of pellets manufactured using the seeds;

FIG. 4B illustrates images showing seeds produced by an extruder equipped with a mold type extruder die used in the present disclosure and a shape of pellets manufactured using the seeds;

FIG. 4C illustrates an image showing shapes of final spherical pellets completed after fluidized bed coating of seeds passing through extrusion holes with diameters of 1.5, 1.2, and 1 mm;

FIG. 5 illustrates images showing color coating of surfaces of finished spherical pellets;

FIG. 6 illustrates images showing a slurry layer obtained by collecting an essential oil of an aromatic single herbal medicine;

FIG. 7 illustrates images showing spherical pellets manufactured using single herbal medicines classified as high-sugar-content medicines;

FIG. 8 illustrates images showing spherical pellets manufactured using single herbal medicines classified as medium-sugar-content medicines;

FIG. 9 illustrates images showing spherical pellets manufactured using single herbal medicines classified as low-sugar-content medicines;

FIG. 10 illustrates images showing spherical pellets manufactured using complex herbal medicines; and

FIG. 11 shows chromatogram results analyzing ephedrine contained in spherical pellets of *Mahwang-tang*.

## DETAILED DESCRIPTION OF THE EXEMPLARY EMBODIMENTS

Hereinafter, the present disclosure will be described in more detail with reference to examples.

## EXAMPLES

Throughout the present specification, the term "%" used to express the concentration of a specific material, unless otherwise particularly stated, refers to (wt/wt)% for solid/solid, (wt/vol)% for solid/liquid, and (vol/vol)% for liquid/liquid.

**Comparative Example 1: Comparison with conventional method**

**1.1. Comparison with case where spherical pellets of sugar spheres and cellulose are used as seeds**

Comparison was made between the seeds manufactured by the method of the present disclosure and the seeds employing spherical pellets of starch-containing sugar spheres and cellulose, disclosed in Korean Patent Application No. 10-2004-0075804, "Preparation containing plant extracts and manufacturing method therefor" and Korean Patent Application No. 10-2011-0105351, "pellet formulation containing medicinal herbal extract and having improved hygroscopic properties and manufacturing method therefor".

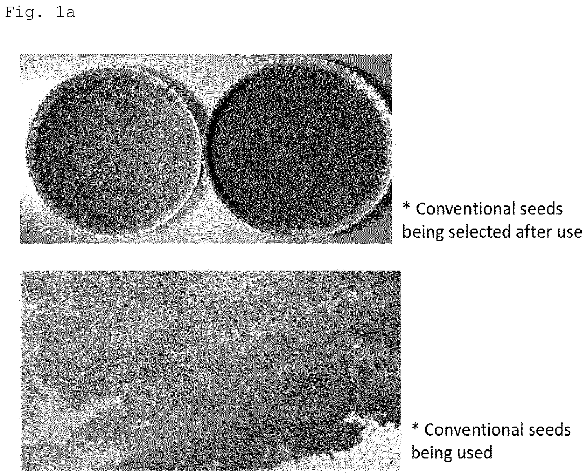

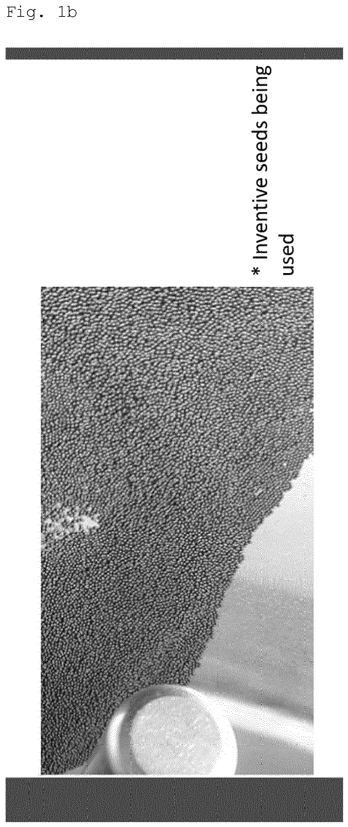

When the spherical pellets of starch-containing sugar spheres and cellulose are used as seeds, the seeds undergo friction and collisions by spray air pressure during the process in a fluidized bed apparatus, and the seeds are broken due to weak hardness, resulting in an increase in friability (FIG. 1A).

**1.2. Problems in conventional manufacturing method**

The use of extract dry powders has a problem in that the contents of herbal extract solids or marker components contained in final products get gradually smaller since various kinds of excipients are added in the manufacture of powders in a spray drier, the preparation of seeds, and the preparation of a preparation solution for fluidized bed spraying

When, for use as basic seeds in a fluidized bed granulator, plant herbal extract dry powders, a extract concentrates, and excipients are mixed, and made into an indeterminate form of granular pellets by using a high shear mixing and granulator or vertical granulator or made into granules by using a conventional extruder, the resultant extrudate, unlike an extrudate for western medicines, has very strong stickiness and thus is lengthily extruded, like noodles, without being naturally easily cut. When such an extrudate is ground to be made into spherical pellets in a spheronizer, the spherical pellets are made into a rectangular cylindrical shape with both edges worn. In cases where such pellets are used as seeds in a fluidized bed granulator, the final shape is impossible to form into a sphere regardless of coating with the same kind of extract.

When the resultant product formed in the form of rod-like granules or linear granules, disclosed in the application described in the background art, is ground in a spheronizer according to a conventional pellet manufacturing method, complete spherical pellets cannot be produced as shown in the examples of shape of FIG. 2. When such pellets are introduced into a fluidized bed granulator and an extract is sprayed and coated, final products have non-uniform shapes, deteriorating in marketability. Moreover, the rod-like granules and the like manufactured by a high shear mixing and granulator or vertical granulator have weak hardness, and thus seeds are broken in the subsequent fluidized bed coating process, and granules with small broken particles sticking thereto result in a lack of uniformity of the entire appearance and marketability.

In oriental medicine, as for single herbal medicines considering the presence of an aroma component according to the characteristics of raw materials and complex herbal medicines having a high content of an aromatic single herbal medicine, no consideration was given to the presence or absence of the aroma component in the final formulation or the transfer rate of the aroma component to the final formulation in the conventional art. Herbal decoction extracts are medicines with high safety since the experience of dosing is ensured for a long period of time, and according to the Qi-taste theory, which is considered as the basis of oriental medicine theories, there exists justification that aroma or smell components should be contained in final products, and the efficacy of the herbal decoction extracts has been proved in even modern aroma therapy using aromas.

The conventional pellet formulation manufacturing methods did not consider physical characteristics, such as the content of sugar contained in an extract and viscosity of the extract, and thus, the viscosity continuously increases and heat is generated during the manufacture of pellets by common stirring and extrusion. As a result, a kneaded product that has not been extruded is hardened to cause instant mechanical resistance, and thus a screw may be stopped and screen holes may be clogged, and at last, a granulator may become inoperable.

**1.3. Problems in utilization of final products manufactured by conventional method**

In conventional herbal medicines, the contents of marker components administered to a patient cannot be accurately identified, and the adjustment of the contents of important medicinal herbs or marker components, which is involved in personal characteristics of patients and disease symptom worsening or improvement, is impossible on the spot to issue a prescription.

A prescription of a complex herbal medicine using a combination of single herbal medicines, manufactured by the method of the present disclosure, can be promptly delivered on the spot. Furthermore, in cases where the complex herbal medicine manufactured by the method of the present disclosure is required to effectively reduce disease symptoms of a patient, the weight of a single herbal medicine containing an important marker component corresponding to the requirement can be easily increased or decreased, and finally, a personal customized prescription of herbal medicines can be promptly made. Therefore, the herbal formulation of the present application can be effectively applied in an apparatus for automatic mixing and manufacturing herbal medicinal prescriptions.

**Example 1: Classification of single herbal extracts and complex herbal extracts according to sugar content of extract**

**1.1. Preparation and classification of single herbal extracts**

In the Korean Herbal Pharmacopoeia, 68 kinds of single herbal medicines are listed. Out of these, 66 kinds of single herbal medicines excluding Natrii sulfas (sodium sulfate) and Gypsum Fibrosum (calcium sulfate) as mineral medicines, that is, Puerariae Radix, Chrysanthemi Flos, Glycyrrhizae Radix, Notopterygii Rhizoma, Zingiberis Rhizoma Siccus, Cinnamomi Ramulus, Agastachis Herba, Trichosanthis Semen, Lonicerae Flos, Platycodi Radix, Angelicae Gigantis Radix, Jujubae Fructus, Rhei Rhizoma, Persicae Semen, Angelicae Pubescentis Radix, Ephedrae Herba, Viticis Fructus, Liriopes Radix, Hordei Fructus Germiniatus, Moutan Cortex, Aucklandiae Radix, Menthae Herba, Pinelliae Rhizoma, Saposhnikovia Radix, Thujae Resina, Atractylodis Rhizoma Alba, Poria(Hoelen), Amomi Fuctus, Crataegii Fructus, Scirpi Rhizoma, Mori Radicis Cortex, Zingiberis Rhizoma Recens, Rehmanniae Radix, Asari Herba Cum Radix, Rehmanniae Radix Preparat, Cimicifugae Rhizoma, Bupleuri Radix, Massa Medicata Fermentata, Zedoariae Rhizoma, Forsythiae Fructus, Schizandrae Fructus, Cinnamomi Cortex Spissus, Ginseng Radix, Artemisiae Capillaris Herba, Perilla Folium, Paeoniae Radix, Peucedani Radix, Fritillariae Thunbergii Bulbus, Aurantii Fructus Pericarpium, Anemarrhenae Rhizoma, Aurantii Immaturus Fructus, Fraxini Cortex, Atractylodis Rhizoma, Ligustici Rhizoma, Gastrodiae Rhizoma, Citrii Unshiu Immaturi Pericarpium, Gardeniae Fructus, Alismatis Rhizoma, Armeniacae Semen, Cyperi Rhizoma, Schizonepetae Spica, Scutellariae Radix, Astragali Radix, Coptidis Rhizoma, Phellodendri Cortex, and Magnoliae Cortex were used to prepare extracts thereof. The results are shown in Table 1, and the names of known important maker components are also indicated therein.

Each herbal medicine 200g was introduced into an extractor, and 10-fold purified water 2000 g was introduced thereinto, followed by extraction at 95°C for 2 hours. The sugar content of the extract was measured (Atago Digital saccharometer Pal-3), and the content of solids contained in the extract was measured by an infrared moisture analyzer (FD-720, KETT Electronic, Japan). The measurement was repeatedly conducted three times.

[Table 1]

| Measurement of sugar content and extract solid content in 66 kinds of single herbal extracts | | | | | | |
|---|---|---|---|---|---|---|
| No. | Single herbal medicine | Based on 10-fold water extract | | | | Sugar content classification | Marker component name and content(% or more in extract solids) |
| | | Range | | Average | | | |
| | | Sugar content (Brix) | Extract solid content, % | Sugar content (Brix) | Extract solid content , % | | |
| 1 | Puerariae Radix | 2.8-3.1 | 1. 8-2.3 | 2.95 | 2.05 | Low | puerarin(2.0%), daidzin (0.3%) |
| 2 | Chrysanthemi Flos | 0.8-1.4 | 0.8-1.2 | 1.10 | 1.00 | Low | chlorogenic acid (0.15%), luteolin (0.05%) |
| 3 | Glycyrrhizae Radix | 5.8-6.4 | 6.3-8.5 | 6.10 | 7.5 | High | glycyrrhizic acid(2.0%) |
| 4 | Notopterygii Rhizoma | 6.0-6.4 | 6.3-6.8 | 6.20 | 6.55 | High | notopterol+isoim peratorin(0.4%) |
| 5 | Zingiberis Rhizoma Siccus | 1.6-2.0 | 2.1-2.8 | 1.80 | 2.45 | Low | 6-gingerol(0.35%) |
| 6 | Cinnamomi Ramulus | 0.1-1.0 | 0.2-0.3 | 0.55 | 0.25 | Low | cinnamic acid(0.035%) |
| 7 | Agastachis Herba | 0.2-0.4 | 1.0-2.0 | 0.30 | 1.50 | Low | patchouli alcohol (0.10%) |
| 8 | Trichosanthis Semen | 0.2-1.0 | 1.1-2.1 | 0.60 | 1.60 | Low | 3,29-dibenzoyl rarounitriol(0.0 6%) |
| 9 | Lonicerae Flos | 6.8-7.2 | 6.4-6.9 | 7.00 | 6.65 | High | chlorogenic acid, luteoloside |
| 10 | Platycodi Radix | 7.6-8.6 | 7.7-8.7 | 8.10 | 8.20 | High | platycodin D(0.10%) |
| 11 | Angelicae Gigantis Radix | 5.5-7.8 | 10-14 | 6.65 | 12.00 | High | nodakenin + decursin + decursinol angelate = total decursin 6.0% |
| 12 | Jujubae Fructus | 5.6-7.6 | 7.0-9.5 | 6.60 | 8.25 | High | total sugar(55.0%) |
| 13 | Rhei Rhizoma | 1.8-2.6 | 0.6-1.7 | 2.20 | 1.15 | Low | sennoside a(0.02%), rhein + emodin + chrysophanol + physcion + aloe emodin (1.5%) |
| 14 | Persicae Semen | 1.6-2.0 | 1.9-2.3 | 1.80 | 2.10 | Low | amygdalin (0.5%) |
| 15 | Angelicae Pubescentis Radix | 2.8-3.6 | 1.6-2.5 | 3.20 | 2.05 | Medium | kaurenoic acid + continentalic acid (0.4%) |
| 16 | Ephedrae Herba | 3.2-4 | 3.6-4.5 | 3.60 | 4.10 | Medium | ephedrin + pseudoephedrin(0 . 7%) |
| 17 | Viticis Fructus | 0.6-1.0 | 0.8-1.0 | 0.80 | 0.90 | Low | vitexicarpin(0.0 3%) |

**EP 3 782 609 B1**

(continued)

| Measurement of sugar content and extract solid content in 66 kinds of single herbal extracts | | | | | | |
|---|---|---|---|---|---|---|
| No. | Single herbal medicine | Based on 10-fold water extract | | | | Sugar content classification | Marker component name and content(% or more in extract solids) |
| | | Range | | Average | | | |
| | | Sugar content (Brix) | Extract solid content, % | Sugar content (Brix) | Extract solid content , % | | |
| 18 | Liriopes Radix | 4.2-5.6 | 3.8-5 | 4.90 | 4.40 | Medium | ruscogenin(0.12% ) |
| 19 | Hordei Fructus Germiniatus | 0.6-1.6 | 0.9-1.9 | 1.10 | 1.40 | Low | - |
| 20 | Moutan Cortex | 5.7-6.9 | 5.4-7.2 | 6.30 | 6.30 | High | paeonol(1.0%) |
| 21 | Aucklandiae Radix | 3.8-4.8 | 3.8-4.5 | 4.30 | 4.15 | Medium | costunolide+dehy drocostus lactone (1.8%) |
| 22 | Menthae Herba | 0.6-1.4 | 0.7-1.1 | 1.00 | 0.90 | Low | essential oil (0.4%) |
| 23 | Pinelliae Rhizoma | 0.6-0.8 | 0.9-1.2 | 0.70 | 1.05 | Low | adenine(0.005%) |
| 24 | Saposhnikovia Radix | 5.6-6.2 | 5.4-5.9 | 5.90 | 5.65 | High | prin-o-glycosylcimifugi n + 4'-o-beta-glucopyranosyl-5-o-methylyisa mminol (0.24%) |
| 25 | Thujae Resina | 7.4-8.6 | 7.4-8.3 | 8.00 | 7.85 | High | imperatorin(0.08 %) |
| 26 | Atractylodis Rhizoma Alba | 5.5-6.1 | 5.2-6.5 | 5.80 | 5.85 | High | atractylon(1.45% ) |
| 27 | Poria(Hoelen | 0.03-0.05 | 0.2-0.5 | 0.04 | 0.35 | Low | Pachymic acid(0.25%) |
| 28 | Amomi Fuctus | 1.0-1.8 | 1.2-1.9 | 1.40 | 1.55 | Low | bornyl acetate |
| 29 | Crataegii Fructus | 5.6-6.2 | 5.4-6.2 | 5.90 | 5.80 | High | chlorogenic acid (0.01%), hyperoside (0.01% ) |
| 30 | Scirpi Rhizoma | 0.6-1.4 | 0.9-1. 6 | 1.00 | 1.25 | Low | betulin, betulinic acid |
| 31 | Mori Radicis Cortex | 1.0-2.0 | 0.9-1.9 | 1.50 | 1.25 | Low | total sanggenon(0.5%) |
| 32 | Zingiberis Rhizoma Recens | 3.5-4.6 | 6-7 | 4.05 | 6.50 | Medium | 6-gingerol(0.35%) |
| 33 | Rehmanniae Radix | 6.5-7.2 | 6.7-7.5 | 6.95 | 7.35 | High | stachyose, iridoid compound(catalpo 1), catalpinoside(0. 20%), acteoside(0.02%) |
| 34 | Asari Herba Cum Radix | 0.6-1.0 | 1.0-1.4 | 0.80 | 1.20 | Low | l-asarinin, aristolochic acid |
| 35 | Rehmanniae Radix Preparat | 8-10 | 7.8-9.2 | 9.00 | 8.50 | High | 5-hydroxymethyl-2-furaldehyde(0.1% ), acteoside(0.02%) |

(continued)

| | | Based on 10-fold water extract | | | | Sugar content classification | Marker component name and content(% or more in extract solids) |
|---|---|---|---|---|---|---|---|
| No. | Single herbal medicine | Range | | Average | | | |
| | | Sugar content (Brix) | Extract solid content, % | Sugar content (Brix) | Extract solid content, % | | |
| 36 | Cimicifugae Rhizoma | 3-3.8 | 2.8-3.5 | 3.40 | 3.15 | Medium | caffeic acid + isoferulic acid + ferulic acid (0.55%) |
| 37 | Bupleuri Radix | 1.6-2.4 | 1.6-2.3 | 2.00 | 1.95 | Low | saikosaponin a(0.3%) |
| 38 | Massa Medicata Fermentata | 1.0-1.4 | 1.4-2.3 | 1.20 | 1.85 | Low | - |
| 39 | Zedoariae Rhizoma | 0.1-0.6 | 0.4-1.2 | 0.35 | 0.80 | Low | germacrone, curcumenol |
| 40 | Forsythiae Fructus | 0.4-1.6 | 1.3-2.6 | 1.00 | 1.95 | Low | arctigenin(0.4%), forsythoside a(0.25%) |
| 41 | Schizandrae Fructus | 4.6-5.4 | 5.0-5.9 | 5.0 | 5.45 | Medium | schizandrin + gomisin a + gomisin n(0.7%) |
| 42 | Cinnamomi Cortex Spissus | 1.0-1.4 | 0.8-1.4 | 1.20 | 1.10 | Low | cinnamic acid(0.035%) |
| 43 | Ginseng Radix | 5.2-6.8 | 5.7-7.6 | 6.00 | 6.65 | High | ginsenoside rg1(0.1%) +ginsen oside rb1 (0.2%)=0.3% |
| 44 | Artemisiae Capillaris Herba | 0.8-1.6 | 1.0-1.9 | 1.20 | 1.45 | Low | dimethyl esculetin (0.1%) |
| 45 | Perilla Folium | 0.2-0.8 | 0.6-1.1 | 0.5 | 0.85 | Low | Rosmarinic acid (0.03%) |
| 46 | Paeoniae Radix | 3.8-4.2 | 8.5-12 | 4.00 | 6.00 | Medium | albiflorin + paeoniflorin (2.3 %) |
| 47 | Peucedani Radix | 1.1-1.5 | 1.3-1.9 | 1.30 | 1.60 | Low | praeruptorin |
| 48 | Fritillariae Thunbergii Bulbus | 1.6-2.4 | 2.0-2.4 | 2.00 | 2.20 | Low | peimine + peiminine (0.08%) |
| 49 | Aurantii Fructus Pericarpium | 4.0-5.0 | 3.5-4.2 | 4.50 | 3.85 | Medium | naringin, neohesperidin |
| 50 | Anemarrhenae Rhizoma | 6.2-7.0 | 5.2-5.8 | 6.60 | 5.50 | High | mangiferin(0.7%), timosaponin(3.0 %) |
| 51 | Aurantii Immaturus Fructus | 1.4-2.4 | 1.7-2.4 | 1.90 | 2.05 | Low | poncirin(2.0%)+n aringin(0.7%)=2. 7% |
| 52 | Fraxini Cortex | 6.8-7.2 | 7.2-8.5 | 7.0 | 7.85 | High | hesperidin(3.5%) |

Measurement of sugar content and extract solid content in 66 kinds of single herbal extracts

(continued)

| Measurement of sugar content and extract solid content in 66 kinds of single herbal extracts | | | | | | |
|---|---|---|---|---|---|---|
| No. | Single herbal medicine | Based on 10-fold water extract | | | | Sugar content classification | Marker component name and content(% or more in extract solids) |
| | | Range | | Average | | | |
| | | Sugar content (Brix) | Extract solid content, % | Sugar content (Brix) | Extract solid content , % | | |
| 53 | Atractylodis Rhizoma | 4.6-5.2 | 4.8-5.4 | 4.90 | 5.10 | Medium | atractylodin(0.1 5%), β-eudesmol(3.5%), |
| 54 | Ligustici Rhizoma | 1.4-2.4 | 1.9-2.4 | 1.90 | 2.15 | Low | ferulic acid(0.10%) |
| 55 | Gastrodiae Rhizoma | 2.9-3.4 | 2.9-3.8 | 3.15 | 3.35 | Medium | gastrodin + gastrodigenin(0. 2%) |
| 56 | Citrii Unshiu Immaturi Pericarpium | 3.8-4.4 | 3.5-4.2 | 4.10 | 3.85 | Medium | hesperidin(4.0%) |
| 57 | Gardeniae Fructus | 3.0-4.0 | 3.4-3.8 | 3.50 | 3.60 | Medium | geniposide(3.0%) , gardenoside(1.8% ) |
| 58 | Alismatis Rhizoma | 2.6-3.2 | 3.2-4.5 | 2.90 | 3.85 | Low | alisol b + 23-acetyl alisol b(0.2%) |
| 59 | Armeniacae Semen | 1.6-2.2 | 1.3-2.4 | 1.90 | 1.85 | Low | amygdalin (3.0%) |
| 60 | Cyperi Rhizoma | 1. 8-2.4 | 1. 6-2.4 | 2.10 | 2.00 | Low | benzoylaconine(0 . 33%) |
| 61 | Schizonepetae Spica | 0.4-0.8 | 0.2-1.0 | 0.60 | 0.60 | Low | pulegone(0.02%) |
| 62 | Scutellariae Radix | 8.6-9.0 | 8.4-9.5 | 8.80 | 8.95 | High | baicalin + baicalein + wogonin(10.0%) |
| 63 | Astragali Radix | 3.8-4.6 | 4.2-4.8 | 4.20 | 4.50 | Medium | astragaloside |
| 64 | Coptidis Rhizoma | 3.4-4.8 | 3.1-4.5 | 3.60 | 3.80 | Medium | berberine (4. 0%) |
| 65 | Phellodendri Cortex | 2.6-3.0 | 2.2-2.5 | 2.80 | 2.35 | Low | berberine(0.6%) |
| 66 | Magnoliae Cortex | 0.5-0,9 | 0.8-1.2 | 0.70 | 1.00 | Low | magnolol + honokiol (1.0%) |

Based on the sugar content and solid content measurement results of the extracts of 66 kinds of single herbal medicines on Table 1, the single herbal medicines were classified into ① high-sugar-content single herbal medicines having a sugar content of 5.5 or more and less than 10.0 Brix in the extracts, ② medium-sugar-content single herbal medicines having a sugar content of 3.0 or more and less than 5.5 Brix in the extracts, and ③ low-sugar-content single herbal medicines having a sugar content of 0.01 or more and less than 3.0 Brix in the extracts.

From the analysis results, the high-sugar-content single herbal medicines encompass 17 kinds of single herbal medicines ranging from the Atractylodis Rhizoma Alba extract having a sugar content of 5.8 to the Rehmanniae Radix Preparat extract having a sugar content of 9.0 and account for 25.8%; and the medium-sugar-content single herbal medicines encompass 15 kinds of single herbal medicines ranging from the Angelicae Pubescentis Radix having a sugar content of 3.2 and the Gastrodiae Rhizoma extract having a sugar content of 3.15 to the Schizandrae Fructus extract having a sugar content of 5.0 and account for 22.7%. The low-sugar-content single herbal medicines encompass 34 kinds of single herbal medicines largely ranging from the Poria(Hoelen) extract having a sugar content of 0.04 and the Magnoliae Cortex extract having a sugar content of 0.7 to the Puerariae Radix extract having a sugar content of 2.95 and account for 51.5%. Then, as shown in Table 2, five kinds for each of high-, medium-, and low-sugar-content single herbal medicines were randomly selected from the 66 kinds of single herbal medicines. Out of the selected herbal medicines, aromatic materials are Atractylodis Rhizoma and Cinnamomi Cortex Spissus.

[Table 2]

| Classification of single herbal medicines according to sugar content of extract and selection of samples | | |
|---|---|---|
| Classific ation by sugar contents | Sugar content range, Brix | Five kinds of selected single herbal medicines (Brix) |
| High-sugar-content medicines | 5.5- 10.0 | Glycyrrhizae Radix(6.10), Jujubae Fructus (6.60), Ginseng Radix (6.00), Fraxini Cortex(7.00), Scutellariae Radix(8.80) |
| Medium-sugar-content medicines | 3.0-5.5 | Ephedrae Herba(3.60), Zingiberis Rhizoma Recens(4.05), Paeoniae Radix(4.00), Atractylodis Rhizoma(4.90), Coptidis Rhizoma(3.60) |
| Low-sugar-content medicines | 0.01- 3.0 | Pinelliae Rhizoma(0.70), Bupleuri Radix(2.00), Cinnamomi Cortex Spissus(1.20), Aurantii Immaturus Fructus(1.90), Magnoliae Cortex(0.70) |

## 1.2. Preparation and classification of complex herbal extracts

Like the single herbal medicines, a 10-fold purified water was added to each of complex herbal medicines composed of two or more kinds of single herbal medicines, followed by extraction at 95°C for 2 hours. The sugar content of the extract was measured (Atago Digital saccharometer Pal-3), and the content of solids contained in the extract was measured by an infrared moisture analyzer (FD-720, KETT Electronic, Japan). The measurement was repeatedly conducted three times. Table 3 shows the sugar content and solid content measurement results.

[Table 3]

| Measurement of sugar content and solid content of 26 kinds of complex herbal extracts | | | | | | |
|---|---|---|---|---|---|---|
| No. | Complex herbal decoction | Based on 10-fold water extract | | | | Sugar content classificat ion |
| | | Range | | Average | | |
| | | Sugar content (Brix) | Solid content, % | Sugar content (Brix) | Solid content, % | |
| 1 | *Gamisoyosan* | 2.3-2.8 | 2.7-3.5 | 2.7 | 3.3 | Low |
| 2 | *Galgeun-tang* | 3.4-4.1 | 3.2-4.9 | 3.9 | 4.2 | Medium |
| 3 | *Galgeunhaegi-tang* | 1.5-2.0 | 2.1-2.6 | 1.7 | 2.4 | Low |
| 4 | *Gumiganghwal-tang* | 6.0-7.6 | 6.9-8.5 | 6.7 | 7.2 | High |
| 5 | *Dangguiyukhwang -tang* | 4.2-5.5 | 4.0-5.8 | 4.7 | 5.0 | Medium |
| 6 | *Dashiho-tang* | 2.1-2.7 | 1.8-3.1 | 2.5 | 2.8 | Low |
| 7 | *Daecheongryong-tang* | 2.3-3.2 | 2.0-4.1 | 2.6 | 2.9 | Low |
| 8 | *Daehwajungeum* | 3.2-4.1 | 2.9-4.3 | 3.8 | 3.6 | Medium |
| 9 | *Daehwangmokdanp i-tang* | 1.7-2.4 | 1.2-2.8 | 2.0 | 2.2 | Low |
| 10 | *Doinseunggi-tang* | 1.2-1.7 | 0.8-1.3 | 1.5 | 0.9 | Low |
| 11 | *Mahwang-tang* | 3.5-4.6 | 3.3-4.1 | 3.8 | 3.6 | Medium |
| 12 | *Banhabaekchulch eonma-tang* | 2.4-3.0 | 2.2-3.5 | 2.6 | 2.9 | Low |

(continued)

| | | Based on 10-fold water extract | | | | |
|---|---|---|---|---|---|---|
| | | Range | | Average | | Sugar content classificat ion |
| No. | Complex herbal decoction | Sugar content (Brix) | Solid content, % | Sugar content (Brix) | Solid content, % | |
| 13 | *Banhasasim-tang* | 3.4-4.4 | 3.0-4.8 | 3.8 | 3.7 | Medium |
| 14 | *Banhahubak-tang* | 2.0-2.8 | 2.5-3.7 | 2.5 | 3.2 | Low |
| 15 | *Baekchul-tang* | 4.5-5.8 | 4.2-6.0 | 5.0 | 5.3 | Medium |
| 16 | *Bojungikgi-tang* | 3.3-4.3 | 3.0-4.6 | 3.7 | 3.5 | Medium |
| 17 | *Bohe-tang* | 4.7-5.2 | 4.5-6.1 | 4.9 | 5.3 | Medium |
| 18 | *Bokryeongbosim-tang* | 2.3-2.9 | 2,0-3.5 | 2.5 | 3.1 | Low |
| 19 | *Bulhwangeumj eon ggisan* | 4.5-6.1 | 4.3-6.7 | 5.3 | 5.8 | Medium |
| 20 | *Samsoum* | 4.6-5.5 | 4.1-6.8 | 5.1 | 4.8 | Medium |
| 21 | *Samchulgeonbi-tang* | 4.2-5.8 | 3.8-5.8 | 5.0 | 4.8 | Medium |
| 22 | *Saengmaeksan* | 3.3-4.2 | 3.2-5.5 | 3.7 | 4.3 | Medium |
| 23 | *Sosiho-tang* | 3.8-4.5 | 3.5-5.2 | 4.1 | 4.7 | Medium |
| 24 | *Yeongyangjihwan g-tang* | 5.8-7.0 | 6.2-7.8 | 6.2 | 6.8 | High |
| 25 | *Palmul-tang* | 4.4-5.5 | 4.1-6.8 | 4.8 | 5.7 | Medium |
| 26 | *Pyeongwisan* | 4.0-4.8 | 3.7-5.3 | 4.4 | 4.1 | Medium |

Measurement of sugar content and solid content of 26 kinds of complex herbal extracts

As shown in the results in Table 3, the 10-fold water extracts based on the weights of medicinal herbs of the complex herbal decoctions were measured to have similar sugar content ranges to medium- and low-sugar-content single herbal medicines. Therefore, based on the sugar content and solid content measurement results of the extracts of 26 kinds of complex herbal decoctions in Table 3, the complex herbal decoctions, like the single herbal medicines, were classified into ① high-sugar-content complex herbal decoctions having a sugar content of 5.5 or more and less than 10.0 Brix in the extracts, ② medium-sugar-content herbal decoctions having a sugar content of 3.0 or more and less than 5.5 Brix in the extracts, and ③ low-sugar-content herbal decoctions having a sugar content of 0.01 or more and less than 3.0 Brix in the extracts.

That is, the extracts of the complex herbal decoctions showed a tendency in which, as the content percentage of a raw material corresponding to the high-sugar-content signal herbal medicines increases, the sugar content of the extract also increases. Out of the 26 kinds of complex herbal medicines, 2 kinds (7.7%), 15 kinds (57.7%), and 9 kinds (34.6%) corresponded to the high-, medium-, and low-sugar-content extracts, respectively. Consequently, when multiple single herbal medicines were mixed, the mixture was changed into a medium-sugar-content extract, accounting for 57% or more. Therefore, 90% of the complex herbal decoctions corresponded to the extracts of the medium- or low-sugar-content complex herbal decoctions.

Then, out of the 26 kinds of complex herbal medicines composed of one or more single herbal medicines, one kind of each of complex herbal decoctions in high-, medium-, and low-sugar-content ranges for preparing seeds was selected, and is shown in Table 4.

[Table 4]

| Classification of complex herbal decoctions according to sugar content of extract and selection of samples for seed preparation | | |
|---|---|---|
| Sugar content-specific classific ation | Sugar content range, Brix | Selected complex herbal decoction (Brix: constituent single herbal medicine) |
| High-sugar-content complex herbal decoction | 5.5- 10.0 | *Gumiganghwal-tang* (6.7: Notopterygii Rhizoma, Saposhnikovia Radix, Ligustici Rhizoma, Thujae Resina, Atractylodis Rhizoma, Scutellariae Radix, Rehmanniae Radix, Asari Herba Cum Radix, Glycyrrhizae Radix) |
| Medium-sugar-content complex herbal decoction | 3.0 -5.5 | *Mahwang-tang*(3.8: Ephedrae Herba, Armeniacae Semen, Cinnamomi Ramulus, Glycyrrhizae Radix) |
| Low-sugar-content complex herbal decoction | 0.01- 3.0 | *Doinseunggi-tang* (1.5: Rhei Rhizoma, Persicae Semen, Cinnamomi Ramulus, Natrii sulfas, Glycyrrhizae Radix) |

**Example 2: Preparation of seeds by extruder and spheronizer, and spherical pelletizing of seeds by fluidized bed granulator**

**2.1. Preparation of square-cylindrical pellets**

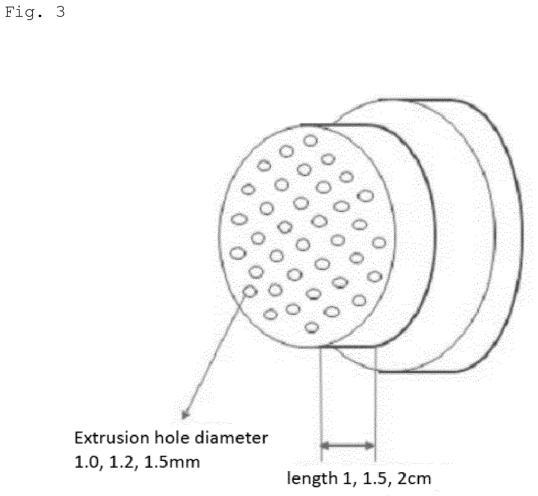

An extruder equipped with a dome-type screen and a spheronizer (Enger E-50 extruder and S-250 Spheronizer) were used for the manufacture of spherical pellets by the conventional method, and an extruder used in the present disclosure includes an extruder die mold part having a plurality of circular extrusion holes, instead of the screen, as shown in FIG. 3. The extrusion holes, through which an extrudate passes, have a shape with a length of 1-2 cm and a diameter of 1-1.5 mm, and a cutting part with a plurality of rotary-type knife blades for cutting the extrudate into a length of 1-1.5 mm were disposed on the front surface of the die part.

In the conventional extrusion molding manner, a kneaded product obtained by adding an herbal extract concentrate or an herbal extract dry powder and an excipient is transferred into a cylinder of an extruder, and while a screw is rotated, the kneaded product is remixed and compressed, and thus has rapidly increased viscosity and generates heat by friction inside the cylinder, causing the hardening of the contents, with the result that existing screen holes may be clogged, or the kneaded product is lengthily extruded and thus is not easily naturally cut, unlike extrudates of western medicines.

Moreover, in western medicines, a cylindrical extrudate is easily cut to a length of 2-4 times the diameter, and when the extrudate cut to a length of 2-4 times the diameter is introduced into the spheronizer, the extrudate is relatively easily ground due to weak stickiness and hardness thereof, with the result that a spherical pellet shape can be made within 5-10 minutes. On the other hand, a rectangular cylindrical extrudate formed of an herbal extract concentrate or an herbal extract dry powder has high hardness due to high stickiness thereof, and thus when a spheronizer is operated, the extrudate cannot be easily cut to a shorter length due to internal friction and collision, and even though the extrudate is artificially cut into a length of 2-4 times the diameter, like extrudates of western medicines, and introduced into the spheronizer, the extrudate is made into a rectangular cylindrical shape. Therefore, when again coated with an herbal extract concentrate in a fluidized bed apparatus, such a product is made into pellets with a very bulging appearance.

**2.2. Preparation of seeds and comparison of finished product after completion of fluidized bed coating of prepared seeds**

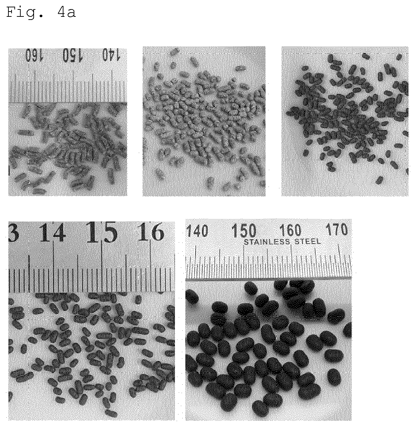

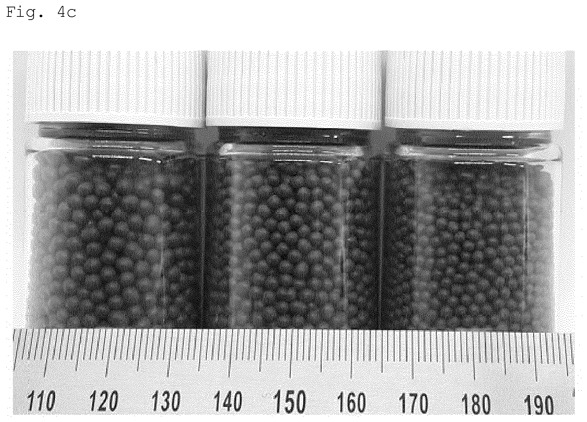

The pellets obtained by cutting a cylindrical extrudate into a length of 2-4 times the diameter (FIG. 4a) and the square-cylindrical pellets obtained by cutting the cylindrical extrudate into a length of the diameter, employed in the present application (FIG. 4b), were each introduced into a spheronizer, and ground in the same conditions, that is, at 1500 rpm for 10 min, and then sieved. Then, the pellets selected as accounting for the largest percentage in the sieve size were introduced as seeds in the same amount into a bottom-spray type fluidized bed apparatus, and then the resultant products were formed in the same concentrate and spray time. As shown in FIG. 4, as the length of the pellet increases, the pellet gains a rectangular shape with a longer length even after grinding.

**2.2.1. Preparation of seeds ground according to extruding conditions and shape comparison test of final formulations after fluidized bed coating of prepared seeds**

① After a dome type screen with a hole size of 1.5 mm was mounted on a conventional extruder, a mixture of a *Mahwang-tang* concentrate and an excipient was made into an extrudate at 40 rpm. Thereafter, samples obtained by cutting the extrudate into a length of 2-3 times the diameter, that is, 3.0-4.5 mm, were ground at 1500 rpm for 10 min in a spheronizer, and the ground samples were sieved. Then, 2.0-2.8 mm-sized seeds were introduced into a fluidized bed granulator, and sprayed with an Ephedrae Herba concentrate, thereby manufacturing a final formulation.

② An extrusion part disc with a hole length of 1.5 cm and a hole diameter 1.5 mm, fabricated in the present application, was used for an extruder. The cut length of an extrudate was 1.5 mm, that is, samples obtained by cutting the extrudate to be equal in length and diameter were ground at 1500 rpm for 10 min corresponding to the same conditions as in ① in a spheronizer, and the ground samples were sieved. Then, 1.0-1.4 mm-sized seeds were introduced into a fluidized bed granulator, and sprayed with an Ephedrae Herba concentrate, thereby manufacturing a final formulation.

③ For the *Mahwang-tang* extract concentrate for preparing seeds, an extract concentrate having a sugar content of 62.5 Brix and a solid content of 60.2% was used, and in the fluidized bed coating process, an extract concentrate having a sugar content of 35.2 Brix and a solid content of 33.1% was used. For preparation of seeds, a kneaded product obtained by adding and mixing an excipient such that the solid content of the extract concentrate and the excipient content were 1:1 was used.

Table 5 shows the grinding results of the extrudate manufactured by the conventional method and the extrudate manufactured by the method of the present disclosure, and grinding was repeated seven times for 200 g of an extrudate for each time. In the conventional method and the method of the present disclosure, 2.8 to 2.0 mm- and 1.4 to 1.0 mm-sized seeds were used as seeds for a fluidized bed apparatus, respectively.

[Table 5]

| Grinding results by spheronizer in conventional extrudate and inventive extrudate | | | | | |
|---|---|---|---|---|---|
| Classification | | | *Mahwang-tang* concentrates | | |
| | | | ① Conventional extruder: Dome-type screen | | (2) Inventive extruder: Mold-type extruder die |
| | | | Extrudate cut length 3.0-4.5mm | | Extrudate cut length 1.5 mm |
| Extruder | Die part | Diameter, mm | - | | 1.5 |
| | Screen | Diameter, mm | 1.5 | | - |
| | Screw speed, rpm | | 40 | | 40 |
| Spheronizer | Grinding speed x time | | 1500rpm x 10min | | 1500rpm x 10min |
| | Loading weight, g | | 200 | | 200 |
| Sieving | | | Weight, g | Percent age,% | Weight, g | Percen tage,% |
| 2.8 mm or more | | | 7.0±1.1 | 3.5 | - | - |
| 2.8-2.36 | | | 95.2±9.4 | 47.6 | - | - |
| 2.36-2.0 | | | 70.8114.2 | 35.4 | - | - |
| 2.0-1.7 | | | 13.4±1.5 | 6.7 | - | - |
| 1.7-1.4 | | | 5.2±0.8 | 2.6 | - | - |
| 1.4-1.18 | | | - | - | 134.0±3.6 | 67.0 |
| 1.18-1.0 | | | - | - | 50.4±1.8 | 25.2 |
| 1.0-850um | | | - | - | 2.8±0.2 | 1.4 |
| 850-710 | | | - | - | 1.7±0.1 | 0.9 |
| 710-600 | | | - | - | - | - |
| Pan(600 um or less) | | | 5.0±0.7 | 2.5 | 6.5±0.2 | 3.3 |

(continued)

| | Extrudate cut length 3.0-4.5mm | | Extrudate cut length 1.5 mm | |
|---|---|---|---|---|
| Loss | 3.4±0.4 | 1.7 | 4.6±0.4 | 2.3 |
| Total | 200 g | 100% | 200 g | 100% |
| Hardness, kgf | 19.4±0.8 | - | 17.3±0.5 | - |

As shown in the results of Table 5, the production efficiency values of pellets with a size that can be used as seeds were 83.2% and 92.2%, respectively, and showed an increase tendency when the length was equal to the diameter in the extrudate. The reason why the deviation was large in the size of 2.0-2.8 mm for the dome type screen was that pellets standing at right angles passed through the sieve during sieving. All the finished seeds had a hardness of 17-19 kgf, which was considered to be significantly hard.

④ After 1000 g of the prepared seeds were introduced into a fluidized bed granulator, a prepared solution obtained by mixing an extract concentrate prepared for a fluidized bed granulator with an excipient in 8% relative to solids of the extract concentrate was sprayed in a bottom-spray manner (spraying from bottom to top). That is, 1000 g of seeds for each case were introduced, and then the seeds were coated with a mixed-solution obtained by homogenizing the same Mahwang-tang concentrate 3500 g (extract solid content of 33.1%) and an excipient 92.7 g (8% of 1158.5 g of extract solids of Ephedrae Herba concentrate).

*Mahwang-tang* belongs to the medium-sugar-content medicines according to the classification criteria of the present application, and the operating conditions of the fluidized bed granulator followed those presented in Table 6 below.

[Table 6]

| Operating conditions of bottom-spray type fluidized bed granulator having a finished product production capacity of 5 kg/batch | | | | |
|---|---|---|---|---|
| Classification | | Operation and initial stage (within 60 min after operation) | Stabilization maintaining step | Final coating step |
| Spray of preparation solution, ml/hr | High-sugar-content | 100 → 400 | 400 → 700 | - |
| | Medium-sugar-content | 300 → 800 | 800 → 1500 | - |
| | Low-sugar-content | 600 → 1000 | 1000 → 2500 | - |
| Product temperature, °C | | 60 → 70 | 70 → 85 | 40 - 45 |
| Spray pressure, kg/cm$^2$ | | 1.5 → 2.0 | 2.0 → 4.0 | 2.0 → 2.5 |
| Preparation solujtion for final coating, ml/hr | | - | - | 500 → 1000 |
| Partition column gap, cm | | 1.0 → 2.0 | 2.0 → 4.0 | 4.0 |
| *Partition column=Draft tube=Wurster tube | | | | |

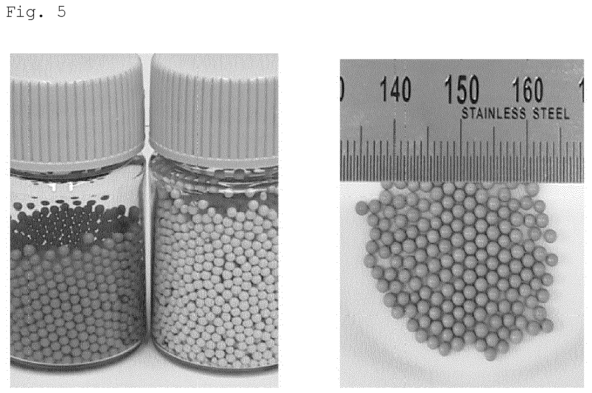

⑤ FIGS. 4A and 4B show changes from seeds to finished spherical pellets during the above procedure and spherical pellets finished using seeds manufactured according to the extrusion hole diameters. FIG. 5 illustrates images showing the color coating of finished spherical pellets. The weight of $TiO_2$ used was 1% per weight of spherical pellets to be coated, and the pellets were white coated and then coated with a color extracted from red radish.

In the present disclosure, an extract concentrate and an excipient were mixed and kneaded, and the kneaded product was passed through extruders including extruder die parts in which extrusion holes with diameters of 1, 1.2, and 1.5 mm were applied to discs having extrusion holes with lengths of 1, 1.5, and 2 cm. The square cylindrical extrudate thus obtained was introduced into a spheronizer, and ground at 1500 rpm for 10 min, thereby manufacturing spherical pellets usable as seeds. The extrudate hardness by screw compression was measured in the range of 17-19 kgf, and thus can be used as seeds for a fluidized bed granulator, without breakage or friability, thereby improving the appearance shape of final products.

**Example 3: Summary of overall process for manufacture of spherical pellets of single herbal medicine and complex herbal medicine**

**3.1. Overall process**

Since single and complex herbal extract concentrates have a high sugar content ranging of 20-70 Brix, kneaded products obtained by adding an excipient to the extract concentrates have high stickiness due to sugar components. The kneaded products are compressed by screw rotation inside an extruder, and thus have largely increased density, thereby significantly increasing plastic mass formability. Therefore, a solid hardened material is formed by frictional heat generated due to the reduction of the flow passing through a die for extrusion, with the result that the extruder becomes inoperable.

Even in the methods for spray drying plant herbal extracts and methods for manufacturing pellets by using extract concentrates or spray dry materials, various binders, anti-caking additives, and film coating agents are used, and various compositional ratios thereof are suggested according to the contents and kinds thereof, the contents of extracts contained in final products, and the like.

In examples of the present disclosure, ultimately desired spherical pellets were completed by:

1) dividing the overall process into a seed preparation process and a fluidized bed process for coating prepared seeds;
2) establishing conditions for maximally increasing the content of extract solids contained in the seeds according to characteristics of herbal extracts in the seed preparation process; and
3) maximizing the increasing content of extract solids while conducting coating in the fluidized bed process using the primarily prepared seeds.

The overall process was summarized in Table 7 below.

[Table 7]

| Summary of overall process for manufacture of spherical pellets of single herbal medicine and complex herbal medicine | | | | | | | |
|---|---|---|---|---|---|---|---|
| Classification | | Concentrates for seeds | | | Concentrates for fluidized bed | | |
| Prepar ation of seeds | Sugar content, Brix | High | Medium | Low | High | Medium | Low |
| | | 45-65 | 40-60 | 30-60 | 15-35 | 25-45 | 20-35 |
| | Kneaded product moisture content , % | 20 - 30 | | | - | | |
| | Extract solid content, % (dry product) | 35-55 | 45-65 | 50-70 | - | | |
| Prepar ation of fluidi zed bed | Excipient addition, % | - | | | 10-15 | 5-10 | 3-8 |
| | Fluidized bed increse,% | - | | | 20-25 | | |
| | Final pellet extract solid content, % (dry product) | - | | | 55-80 | 60-90 | 70-95 |

For the use of excipients and additives, sucrose fatty acid ester as an emulsifier was used, a combination of stearic acid-magnesium, stearic acid-calcium, PEG 6000, beta-cyclodextrin powder, colloidal silicon dioxide, silicon dioxide powder, zein powder, starch sodium octenyl succinate, lactose, precipitated calcium carbonate, microcrystalline cellulose, kaolin, talc, CMC-Ca, casein, and dextrin (DE 10 or less) was used as a releasing agent and an anti-caking agent, and HPMC and pullulan were used as a binder.

For example, ① for a kneaded product using an extract concentrate for seeds in high-sugar-content single or complex herbal medicines, at least one kind of excipient including microcrystalline, beta-cyclodextrin powder, starch sodium octenyl succinate, and the like was used at a weight of 74-83 g, at least one of excipients including precipitated calcium carbonate, silicon dioxide powder, stearic acid-magnesium, and the like was used at a weight of 15-25 g, and sucrose fatty acid ester as an emulsifier was used at a weight of 1-2 g, relative to 100 g of the total excipients.

② For a kneaded product using an extract concentrate for seeds in medium-sugar-content single or complex herbal medicines, at least one kind of excipient including microcrystalline, beta-cyclodextrin powder, starch sodium octenyl succinate, and the like was used at a weight of 78-88 g, at least one kind of excipient including precipitated calcium carbonate, silicon dioxide powder, stearic acid-magnesium, and the like was used at a weight of 10-20 g, and sucrose fatty acid ester as an emulsifier was used at a weight of 1-2 g, relative to 100 g of the total excipients.

③ For a kneaded product using an extract concentrate for seeds in low-sugar-content single or complex herbal medicines, at least one kind of excipient including microcrystalline, beta-cyclodextrin powder, starch sodium octenyl succinate, and the like was used at a weight of 83-94 g, at least one kind of excipient including precipitated calcium carbonate, silicon dioxide powder, stearic acid-magnesium, and the like was used at a weight of 5-15 g, and sucrose fatty acid ester as an emulsifier was used at a weight of 1-2 g, relative to 100 g of the total excipients.

**3.2. Preparation of concentrate for seed preparation and concentrate for fluidized bed coating**

**3.2.1. Preparation of concentrates for seed preparation and for fluidized bed coating after preparation of high-sugar-content single herbal extracts**

Jujubae Fructus, Fraxini Cortex, Ginseng Radix, Glycyrrhizae Radix, and Scutellariae Radix selected among the high-sugar-content single herbal medicines were subjected to massive extraction, and then the concentrates thereof were prepared. The concentrate of each extract was divided into concentrates for two-type purposes. That is, a concentrate for seed preparation and a concentrate for fluidized bed coating were separately prepared.

Each kind of single herbal medicine was immersed in purified water for 30 min after the purified water was added at a weight of 250 kg per 25 kg of the single herbal medicine, and then subjected to extraction at 95-100°C for 3 h, and thereafter, the 10-fold water extract was transferred to a reduced pressure concentrator, and then concentrated under reduced pressure while the temperature was maintained at 60°C. Of the total weight of the 10-fold water extract, 1/3 of the extract and 2/3 of the extract were concentrated to have different sugar contents for seeds and for a fluidized bed, respectively. The sugar content was measured by Atago digital saccharometer (Pal-3) and the content of solids was measured by an infrared moisture analyzer (FD-720, KETT Electronic, Japan), and calculated. Table 8 shows the results of extraction and concentration.

[Table 8]

| Results of preparation of extracts and use-specific concentrates of high-sugar-content herbal medicines | | | | | | | | | |
|---|---|---|---|---|---|---|---|---|---|
| | 10-fold water extract | | | Concentrate for seeds | | | Concentrate for fluidized bed | | |
| Classification | Extraction amount, kg | Sugar content, Brix | Solid content, % | Sugar content, Brix | Solid content, % | Production amount, kg | Sugar content, Brix | Solid content, % | Production amount, kg |
| Jujubae Fructus | 208 | 7.2 | 8.7 | 45.5 | 54.5 | 11.1 | 15.5 | 18.7 | 64.2 |
| Fraxini Cortex | 200 | 6.8 | 7.8 | 47.2 | 54.1 | 9.4 | 25.2 | 28.9 | 36.5 |
| Ginseng Radix | 188 | 6.2 | 6.5 | 59.5 | 62.4 | 6.3 | 35.4 | 37.1 | 22.5 |
| Glycyrrhizae Radix | 175 | 6.5 | 7.5 | 55.2 | 63.7 | 6.5 | 30.3 | 34.6 | 26.0 |
| Scutellariae Radix | 212 | 8.1 | 8.5 | 60.5 | 63.5 | 9.4 | 25.2 | 26.8 | 45.4 |

**3.2.2. Preparation of concentrates for seed preparation and for fluidized bed coating after preparation of medium-sugar-content single herbal extracts**

Atractylodis Rhizoma, Coptidis Rhizoma, Zingiberis Rhizoma Recens, Paeoniae Radix, and Ephedrae Herba selected among the medium-sugar-content single herbal medicines were subjected to massive extraction, and then the concentrates thereof were prepared. The concentrate of each extract was divided into concentrates for two-type purposes. That is, a concentrate for seed preparation and a concentrate for fluidized bed coating were separately prepared.

Each kind of single herbal medicine was immersed in purified water for 30 min after the purified water was added at a weight of 250 kg per 25 kg of the single herbal medicine, and then subjected to extraction at 95-100°C for 3 h, and thereafter, the 10-fold water extract was transferred to a reduced pressure concentrator, and then concentrated under reduced pressure while the temperature was maintained at 60°C. Of the total weight of the 10-fold water extract, 1/3 of the extract and 2/3 of the extract were concentrated to have different sugar contents for seeds and for a fluidized bed, respectively. The concentration for fluidized bed coating and the concentrate for seed preparation having different sugar contents were separately prepared. The sugar content was measured by Atago digital saccharometer (Pal-3) and the content of solids was measured by an infrared moisture analyzer (FD-720, KETT Electronic, Japan), and calculated. Table 9 shows the results of extraction and concentration. In addition, the concentration of the Atractylodis Rhizoma extract and the recovery of an essential oily layer will be described in detail in Example 3.4 below.

[Table 9]

| Results of preparation of extracts and use-specific concentrates of medium-sugar-content herbal medicines | | | | | | | | | |
|---|---|---|---|---|---|---|---|---|---|
| | 10-fold water extract | | | Concentrate for seeds | | | Concentrate for fluidized bed | | |
| Classifi cation | Extra ction amoun t, kg | Sugar conte nt, Brix | Soli d cont ent, % | Sugar conte nt, Brix | Solid conten t, % | Produc tion amount, kg | Sugar conte nt, Brix | Solid conten t, % | Produc tion amount, kg |
| Atractyl odis Rhizoma | 188 | 4.6 | 5.0 | 50.1 | 53.8 | 5.6 | 34.3 | 37.8 | 17.5 |
| Coptidis Rhizoma | 200 | 3.2 | 3.8 | 46.8 | 55.7 | 4.4 | 25.4 | 31.4 | 17.5 |
| Zingiber is Rhizoma Recens | 205 | 3.9 | 6.1 | 40.2 | 62.6 | 6.3 | 31.4 | 48.3 | 17.5 |
| Paeoniae Radix | 213 | 4.2 | 5.8 | 50.3 | 68.1 | 5.9 | 33.8 | 46.6 | 17.2 |
| Ephedrae Herba | 215 | 3.5 | 4.3 | 52.5 | 64.5 | 4.7 | 41.3 | 50.7 | 12.3 |

### 3.2.3. Preparation of concentrates for seed preparation and for fluidized bed coating after preparation of low-sugar-content single herbal extracts

Pinelliae Rhizoma, Cinnamomi Cortex Spissus, Aurantii Immaturus Fructus, Bupleuri Radix, and Magnoliae Cortex selected among the low-sugar-content single herbal medicines were subjected to massive extraction, and then the concentrates thereof were prepared. The concentrate of each extract was divided into concentrates for two-type purposes. That is, a concentrate for seed preparation and a concentrate for fluidized bed coating were separately prepared.

Each kind of single herbal medicine was immersed in purified water for 30 min after the purified water was added at a weight of 1000 kg per 100 kg of the single herbal medicine, and then subjected to extraction at 95-100°C for 3 h, and thereafter, the 10-fold water extract was transferred to a reduced pressure concentrator, and then concentrated under reduced pressure while the temperature was maintained at 60°C. As for Magnoliae Cortex, a 10-fold purified water was added to 150 kg of the herbal medicine. Of the total weight of the 10-fold water extract, 1/3 of the extract and 2/3 of the extract were concentrated to have different sugar contents for seeds and for a fluidized bed, respectively. The concentration for fluidized bed coating and the concentrate for seed preparation having different sugar contents were separately prepared. The sugar content was measured by Atago digital saccharometer (Pal-3) and the content of solids was measured by an infrared moisture analyzer (FD-720, KETT Electronic, Japan), and calculated. Table 10 shows the results of extraction and concentration. In addition, the concentration of the Cinnamomi Cortex Spissus extract and the recovery of an essential oily layer will be described in detail in Example 3.4 below.

Exceptionally for Pinelliae Rhizoma, regarding a concentrate for preparation used for a fluidized bed, the content of soluble starch contained in the 10-fold water extract of Pinelliae Rhizoma is very high due to the characteristics of the raw material, and thus the extract needs to be concentrated to a range of 10-15 Brix. When the extract is concentrated to 15 Brix or higher, the concentrate cannot be transferred to a peristaltic pump, and thus the viscosity of the concentrate should be lowered by addition of purified water.

[Table 10]

| Results of preparation of extracts and use-specific concentrates of low-sugar-content herbal medicines | | | | | | | | | |
|---|---|---|---|---|---|---|---|---|---|
| Classification | 10-fold water extract | | | Concentrate for seeds | | | Concentrate for fluidized bed | | |
| | Extraction amount, kg | Sugar content, Brix | Solid content, % | Sugar content, Brix | Solid content, % | Production amount, kg | Sugar content, Brix | Solid content, % | Production amount, kg |
| Pinelliae rhizoma | 870 | 0.7 | 1.2 | 35.3 | 60.5 | 6.2 | 11.5 | 19.7 | 34.8 |
| Cinnamomi cortex spissus | 850 | 1.3 | 1.5 | 48.5 | 56.5 | 8.5 | 25.3 | 27.7 | 28.7 |
| Aurantii immaturus fructus | 800 | 1.8 | 1.9 | 55.8 | 58.9 | 8.1 | 30.1 | 30.5 | 33.7 |
| Bupleuri radix | 800 | 2.4 | 2.6 | 60.3 | 65.3 | 9.9 | 35.4 | 39.1 | 36.5 |
| Magnoliae cortex | 1275 | 0.6 | 0.9 | 46.7 | 70.1 | 5.1 | 21.2 | 32.8 | 24.2 |

**3.3. Preparation of concentrates for seed preparation and for fluidized bed coating after preparation of complex herbal decoction extracts**

The content ranges of sugar and solids in extracts of complex herbal decoctions each composed of two or more single herbal medicines were almost similar to physical properties of the single herbal medicines, and thus one kind of complex herbal medicines representing each sugar content range was selected. *Gumiganghwal-tang, Mahwang-tang, and Doinseunggi-tang* were selected as representatives for respective sugar content standards, and extraction and concentrate preparation were carried out in the same manner as in single herbal medicines.

*Gumigangh wal-tang* and *Mahwang-tang* as complex herbal decoctions were immersed in purified water for 30 min after the purified water was added at a weight of 500 kg per 50 kg of the complex herbal medicine, and *Doinseunggi-tang* as a complex herbal decoction was immersed in purified water for 30 min after the purified water was added at a weight of 1000 kg per 100 kg of the complex herbal medicine, and then subjected to extraction at 95-100°C for 3 h. Thereafter, each of the 10-fold water extracts was transferred to a reduced pressure concentrator, and then concentrated under reduced pressure while the temperature was maintained at 60°C. Of the total weight of the 10-fold water extract, 1/3 of the extract and 2/3 of the extract were concentrated to have different sugar contents for seeds and for a fluidized bed, respectively. The concentration for fluidized bed coating and the concentrate for seed preparation having different sugar contents were separately prepared. The sugar content was measured by Atago digital saccharometer (Pal-3) and the content of solids was measured by an infrared moisture analyzer (FD-720, KETT Electronic, Japan), and calculated. Table 11 shows the results of extraction and concentration. In addition, the concentration of the *Gumigangh wal-tang* extract and the recovery of an essential oily layer will be described in detail in Example 3.4 below.

[Table 11]

| Results of preparation of extracts and use-specific concentrates of complex herbal decoctions | | | | | | | | | |
|---|---|---|---|---|---|---|---|---|---|
| Classification | 10-fold water extract | | | Concentrate for seeds | | | Concentrate for fluidized bed | | |
| | Extraction amount, kg | Sugar content, Brix | Solid content, % | Sugar content, Brix | Solid content, % | Production amount, kg | Sugar content, Brix | Solid content, % | Production amount, kg |
| High[1] | 388 | 6.5 | 7.2 | 53.2 | 58.9 | 15.8 | 25.3 | 27.8 | 67.9 |
| Medium[2] | 402 | 3.7 | 3.5 | 62.5 | 60.2 | 7.3 | 35.2 | 33.1 | 29.2 |

(continued)

| Results of preparation of extracts and use-specific concentrates of complex herbal decoctions | | | | | | | | | |
|---|---|---|---|---|---|---|---|---|---|
| Classi ficati on | 10-fold water extract | | | Concentrate for seeds | | | Concentrate for fluidized bed | | |
| | Extra ction amoun t, kg | Sugar conte nt, Brix | Solid conte nt, % | Sugar conte nt, Brix | Solid conte nt, % | Produ ction amoun t, kg | Sugar conte nt, Brix | Solid conte nt, % | Produ ction amoun t, kg |
| Low[3] | 850 | 1.2 | 1.5 | 52.4 | 64.5 | 6.8 | 20.4 | 25.9 | 33.5 |
| *1) High: High-sugar-content, *Gumiganghwal-tang,*<br>2) Medium: Medium-sugar-content, *Mahwang-tang,*<br>3) Low: Low-sugar-content, *Doinseunggi-tang* | | | | | | | | | |

### 3.4. Extraction from aromatic single herbal medicine and aromatic single herbal medicine-containing complex herbal decoction, preparation of concentrate, recovery of essential oil, and encapsulation

Out of the single herbal medicines, Nardostachyos Rhizoma, Dalbergiae Odoriferae Lignum, Osterici Radix, Agastachis Herba, Cinnamomi Ramulus, Aucklandiae Radix, Saposhnikovia Radix, Alpiniae Japonicae Semen, Menthae Herba, Santali Albi Lignum, Moschi Moschus, Thymi Herba, Styrax Liquides, Lysimachiae Foeni-graeci Herba, Olibanum, Cinnamomi Cortex Spissus, Myristicae Semen powder, Pterocarpi Lignum, Syzygii Flos, Atractylodis Rhizoma, Aquilariae Resinatum Lignum, Helenii Radix, Pini Semen, Sesami Oleum, and the like belong to aromatic single herbal medicines that strongly emit a unique smell or aroma. Therefore, in oriental medicines, in cases of single herbal medicines considering the presence of an aroma component according to the characteristics of raw materials and complex herbal medicines having a high content of an aromatic single herbal medicine, consideration needs to be given to the transfer rate of the aroma component to the final medicine or the presence or absence of the aroma component therein, and there exists justification that the aroma component should be contained in the final medicine. As used herein, the term aroma component refers to an essential oil, and most of essential oil components contained in plant medicinal herbs include monoterpenes and sesquiterpenes. In some cases, these components are volatilized in a significant amount during extraction and concentrate preparation, and thus are hardly contained in the obtained extract concentrates.

Therefore, during the preparation of each concentrate for use as preparation solutions for seed preparation and fluidized bed coating, the extraction and concentration of Atractylodis Rhizoma, Cinnamomi Cortex Spissus, and *Gumiganghwal-tang,* which is a complex herbal medicine and contains Notopterygii Rhizoma, Saposhnikovia Radix, and Atractylodis Rhizoma, were carried out in an extractor and concentrator equipped with devices for encapsulating, condensing, and separating a volatile essential oil. The conditions for extraction and concentration were the same as in Examples 2, 3, and 4, but the selected samples were extracted and concentrated in a concentrator equipped with a device for recovering an essential oil layer.

A raw material or a mixed herbal medicine (25 kg of Atractylodis Rhizoma, 100 kg of Cinnamomi Cortex Spissus, and 50 kg of *Gumiganghwal-tang*) was immersed in a 10-fold weight of purified water for 30 min before extraction, and then subjected to extraction at 95-100°C for 3 h. Thereafter, the extract was transferred to a reduced pressure concentrator, and then concentrated under reduced pressure while the temperature was maintained at 55-60°C.

For the recovery of an essential oil layer, a tube through which steam passes was provided at the top portion of the concentrator to which the 10-fold water extract was transferred, and the tube was connected to a condenser in which vaporized steam can be converted into a liquid state. The liquid passing through the condenser was allowed to primarily stand in a tank for condensate water, and then separated into an essential oil component layer and a water portion in an easily detachable liquid separator. A liquid separator marked with units capable of measuring volume was used.

Table 12 shows the essential oil recovery results in the preparation process of the concentrates used for seeds and for a fluidized bed. The sugar contents of the respective use-specific concentrates were the same as the above results.

[Table 12]

| Volumes of essential oil produced by concentration of Atractylodis Rhizoma, Cinnamomi Cortex Spissus, and *Gumiganghwal-tang* extracts | | | | | | | | |
|---|---|---|---|---|---|---|---|---|
| Classific ation | Herbal medicine + purified water, kg | 10-fold water extract produced, kg | | | Concentrate produced, kg | | Essential oil recovered, ml | |
| | | Total | For seeds | For fluid ized bed | For seeds | For fluid ized bed | For seeds | For fluid ized bed |
| Atractylo dis rhizoma | 25 + 250 | 188 | 60 | 128 | 5.6 | 17.5 | 165 | 310 |
| Cinnamomi cortex spissus | 100 + 1000 | 850 | 300 | 550 | 8.5 | 28.7 | 205 | 375 |
| *Gumigangh wal-tang* | 50 + 500 | 388 | 130 | 258 | 15.8 | 67.9 | 53 | 102 |

The essential oil layers were used by calculating the weight percentages of the respective concentrates used for seed preparation and for a fluidized bed process, summing respective portions calculated according to the corresponding ratios to calculate the total amount of the essential oil, and encapsulating the total amount of the essential oil and adding the essential oil to a spray solution prepared for a fluidized bed.

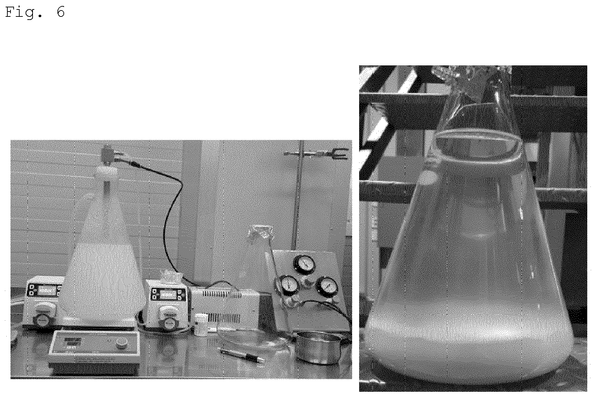

After the completion of extraction and concentration, the essential oil layer was separated, and an oil component was encapsulated using alginic acid or beta-cyclodextrin (Table 13). Thereafter, as shown in FIG. 6, the precipitated slurry layer was added to a preparation solution for a fluidized bed, and used together for coating.

[Table 13]

| Amount of essential oil used and applied encapsulation method | | | | |
|---|---|---|---|---|
| Classification | | Atractylodis Rhizoma | Cinnamomi Cortex Spissus | Gumiganghwal-tang |
| Concentrate for seeds | Production amount, g | 5600 | 8500 | 15800 |
| | Amount of seeds used, g | 2500 (44.6% used) | 4000 (47.1% used) | 3000 (19.0% used) |
| Concentrate for fluidized bed | Production amount, g | 17500 | 28700 | 67900 |
| | Amount of coating, g | 2800 (16.0% used) | 6000 (20.9% used) | 7500 (11.0% used) |
| Amount of recovered essential oil used | Equivalent for seeds, ml | 73.7(165 x 0.446) | 96.6(205 x 0.471) | 10.1(53 x 0.19) |
| | Equivalent for fluidized bed, ml | 49.6(310 x 0.16) | 78.4 (375 x 0.209) | 11.2(102 x 0.11) |
| | Total amount of essential oil used — ml | 123.3 | 175 | 21.3 |
| | Total amount of essential oil used — (g) | (105) | 147) | (18.5) |
| Encapsulation method | | Alginic acid inclusion | Alginic acid inclusion | CD inclusion |
| * see Tables 21, 24, and 27 for concentrates used for seed preparation<br>* see Tables 33, 34, and 35 for concentrates used for fluidized bed coating | | | | |

**3.4.1. Encapsulation of Atractylodis Rhizoma and Cinnamomi Cortex Spissus essential oils by formation of alginic acid coating film**

Based on encapsulation for single dosing by alginic acid:
20 g of Atractylodis Rhizoma essential oil, 2 g of soybean oil, and 2 g of sucrose fatty acid ester were added to 1000 ml of a 2% alginic acid solution, followed by mixing using a homogenizer at 17,000 rpm for 15 min, to thereby prepare a homogenous solution, and then 5000 ml of a 4% CaCl$_2$ solution was prepared. As shown in FIG. 5, 4000 ml of a 4% CaCl$_2$ solution was placed in a 5000 ml-conical flask, and the homogeneous solution of alginic acid and essential oil was sprayed using a 1 mm-diameter nozzle, a spray pressure of 1.0 Bar, and a peristaltic pump rate of 20 ml/min, thereby manufacturing a reaction product in which the Atractylodis Rhizoma essential oil was encapsulated. The reaction product was precipitated, and then washed three times with purified water. The other Atractylodis Rhizoma essential oil was also used to manufacture a reaction product by the same method, and then the recovered reaction product was centrifuged to maximally reduce the total weight, leading to a total production of 247 g.
In addition, 147 g of the Cinnamomi Cortex Spissus essential oil was also encapsulated by the same method. After centrifugation and supernatant removal, 325 g of an inclusion product of Cinnamomi Cortex Spissus essential oil was recovered.

**3.4.2. Encapsulation of *Gumiganghwal-tang* essential oil by beta-cyclodextrin**

1) Preparation of 500 g of 10% β-CD solution: To make a solution having ethanol and purified water at a ratio of 2:1 (w/w), 500 g of ethanol and 250 g of purified water were mixed, and warmed to 55°C. After 450 g of the warmed ethanol solution was taken, 50 g of β-CD powder was added and dissolved therein while stirring.
2) The recovered *Gumiganghwal-tang* essential oil 21.3 ml (18.5 g in weight unit) was dissolved in 200 ml of ethanol.
3) While 500 g of the warmed 10% β-CD solution was stirred at a rate of 100 rpm on a hot plate, the *Gumiganghwal-tang* essential oil-ethanol solution in 2) was slowly added at a constant rate, followed by stirring for 4 h.
4) On the completion of the stirring reaction, the reaction solution was allowed to stand for 12 h in a 4°C refrigerator to complete the reaction. After separation, 130 g of the precipitated reaction product slurry was added to a preparation solution for fluidized bed spraying, prepared to coat *Gumiganghwal-tang* seeds before use.

Table 14 shows the characterization results of capsules in which an essential oil was encapsulated. A coating material for encapsulating an essential oil was sufficiently used with reference to known literature, and the essential oil used was evaluated as being 100% encapsulated since the smell emitted from the reaction product was hardly detected.
The products separated after the reaction were taken equally at 5 g, and used in the measurement of the moisture content, and the remainders were added in the preparation of the extract concentrate for coating used in the fluidized bed process. The essential oil content of the dried capsules was evaluated 52.2% for Atractylodis Rhizoma, 56.6% for Cinnamomi Cortex Spissus, and 20.5% for *Gumiganghwal-tang.*

[Table 14]

| Manufacturing results of capsules in which essential oil was encapsulated | | | | | | |
|---|---|---|---|---|---|---|
| | Amount of essential oil used, g | Amount of enclosure produced, g | | Moisture content, % | Amount of capsules used for fluidized be | * Amount of essential oil encapsulated , g (excluding moisture measurement) | Content of essential oil in capsule, % (Based on capsule dry product) |
| | | Total production, g | Amount of enclosure used in moisture measurement, g | | | | |
| Atractylodis Rhizoma | 105 | 247 | -5 (5/247=2%) | 18.5 | 242 (197.2) | 105 x 98%=102.9g | 102.9/197.2 =52.2% |
| Cinnamomi Cortex Spissus | 147 | 325 | -5 (5/325=1.5% ) | 20.0 | 320 (256.0 ) | 147 x 98.5%=144.8g | 144.8/256=5 6.6% |

(continued)

| Manufacturing results of capsules in which essential oil was encapsulated | | | | | | | |
|---|---|---|---|---|---|---|---|
| | Amount of essen tial oil used, g | Amount of enclosure produced, g | | Moist ure conte nt, % | Amount of capsules used for fluidized be | * Amount of essential oil encapsulated , g (excluding moisture measurement) | Content of essential oil in capsule, % (Based on capsule dry product) |
| | | Total produ ction, g | Amount of enclosure used in moisture measurement, g | | | | |
| *Gumigan ghwal- tang* | 18.5 | 130 | -5 (5/130=3.85 %) | 30.5 | 125( (86.9 ) | 18.5 x 96.15%=17.8g | 17.8/86.9=2 0.5% |
| * The amount of essential oil encapsulated means an amount of pure essential oil contained in enclosures, excluding the exclusions of enclosures used for moisture measurement from the total enclosures, in terms of the amount of essential oil used. | | | | | | | |

### 3.4.3. Measurement of aroma intensity

An electronic nose was used to measure the intensity of aroma components. The measurement was carried out by an electronic nose sensor system using the PEN 3 Portable Electronic Nose (Airsense Analytics GmbH, Germany) equipped with 10 different metal oxides semi-conducting (MOS) sensors with high sensitivity and rapid response in the measurement of volatile organic compounds. The measurement was repeated three times, and the measurement results were expressed as the average measurement value (sensor response signal) by DC mV, and the intensity as a measurement value was compared under the same conditions.

The measured samples were 10-fold water extracts, two types of concentrates (extract concentrates for seed preparation and for fluidized bed coating), and two types of spherical pellets (addition with and without essential oil- encapsulating capsule liquid). Five types of samples were taken at a weight corresponding to 1 g on the basis of the content of extracted solids, and 10 g of spherical pellets as dried samples were powdered in a mortar, and 1 g was taken based on the extract solids. Each sample except for the 10-fold water extract was corrected to the same weight by making up for a portion lacking in weight on the basis of the 10-fold water extract through the addition of distilled water. Therefore, the weights of the 10-fold water extracts of Atractylodis Rhizoma (Table 9), Cinnamomi Cortex Spissus (Table 10), and *Gumiganghwal-tang* (Table 11), corresponding to 1 g of extract solids, were 20, 66.7, and 13.9 g, respectively. The Atractylodis Rhizoma and *Gumiganghwal-tang* samples were placed in 50 ml-glass vials and Cinnamomi Cortex Spissus was placed in 100 ml-glass vials, and then sealed with septum screw caps, followed by mixing. The vials were allowed to stand in a constant-temperature water bath at 60°C for 30 min. Then, in the electronic nose, the gas flow rate was 30 ml/min, the gas sampling time was 40 s, the measurement time was 20 s, and the zero washing time was 180 s. For washing, the top portions of 50 ml-beakers containing distilled water were sealed with silver foil, and then allowed to stand in a constant-temperature water in the same conditions, followed by washing with washing gas.

[Table 15]

| Comparison of aroma intensity by electronic nose | | | | | | |
|---|---|---|---|---|---|---|
| Classification | 10-fold water extract | | Concentrates | | Final spherical pellets | |
| | | | For seeds | For fluidized bed | Capsule (-) | Capsule (+) |
| Atractylod is rhizoma | mV | 7125 | 263 | 440 | 270 | 4250 |
| | Transfer rate,% | 100 | 3.7 | 6.2 | 3.8 | 59.6 |
| Cinnamomi cortex spissus | mV | 8550 | 760 | 1840 | 1425 | 6870 |
| | Transfer rate, % | 100 | 8.9 | 21.5 | 16.7 | 80.4 |
| *Gumiganghw al-tang* | mV | 8200 | 1025 | 1530 | 1025 | 6180 |
| | Transfer rate,% | 100 | 12.5 | 18.7 | 12.5 | 75.4 |

(continued)

| Comparison of aroma intensity by electronic nose | | | | | | |
|---|---|---|---|---|---|---|
| Classification | 10-fold water extract | | Concentrates | | Final spherical pellets | |
| | | | For seeds | For fluidized bed | Capsule (-) | Capsule (+) |
| Transfer range, % | | 100 | 4 - 13 | 6-22 | 4 - 17 | 60 - 80 |

From the results on Table 15, a comparison between the single herbal medicines Atractylodis Rhizoma and Cinnamomi Cortex Spissus showed a significant difference in the mV level of sensor response signal remaining in the two types of extract concentrates, and the important aroma components therefor were reported to be cinnamic acid and beta-eudesmol, respectively. Therefore, the difference was thought to result from the fact that the melting point of beta-eudesmol, 72°C, is lower than the melting point of cinnamic acid, 133°C. Actually, the aromaticity of the Atractylodis Rhizoma concentrate was hardly detectable by the human nose.

As for the aroma level of a 10-fold purified water extract being evaluated as 100%, the percentage of the aroma component contained in final spherical pellets (Tables 33, 34, and 35) was 60-80% when the essential oil volatilized in the concentration process was recovered and returned, and the aroma level was 4-17% without the addition of the encapsulated essential oil slurry, indicating a significant difference.

**Example 4: Preparation of seeds for single and complex herbal medicines**

The preparation of seeds for single herbal medicines and complex herbal medicines was configured of: manufacturing square-cylindrical pellets by an extruder; and then grinding the pellets in a spheronizer to manufacture spherical pellets. As for a primary product by an extruder, in order to solve problems in the manufacturing method discussed above, the kinds, mutual ratio, and levels of excipients added in an extract concentrate prepared for seed preparation, and the moisture content of a kneaded product mixed with the excipients were adjusted. Table 16 shows preparation conditions of seeds.

The excipients used were classified into a mineral additive, a releasing agent, and an emulsifier. At least one of precipitated calcium carbonate (precipitated $CaCO_3$), magnesium oxide, calcium silicate, magnesium silicate, silicon dioxide, titanium dioxide, bentonite, kaolin, and talc was selected and used as a mineral additive; at least one of microcrystalline cellulose (MCC), lactose, stearic acid-magnesium, stearic acid-calcium, PEG 6000, beta-cyclodextrin powder, zein powder, and starch sodium octenyl succinate (SSOC) was used as a releasing agent; and a sucrose fatty acid ester powder was used as an emulsifier.

[Table 16]

| Preparation conditions of seeds | | | | | |
|---|---|---|---|---|---|
| | | | Kind of seeds (single herbal medicines and complex herbal medicies ) | | |
| | | | High | Medium | Low |
| Seed extract solid content, % (based on dry prodcut) | | | 35-55 | 45-65 | 50-70 |
| Mater ial mixin g condi tions | Total excipient content (based on dry produt) | Addition level, % | 45-65 | 35-55 | 30-50 |
| | Percentages by excipients, % | Mineral, % | 15-25 | 10-20 | 5-15 |
| | | Releasing agent, % | 74-83 | 78-88 | 83-94 |
| | | Emulsifier, % | 1-2 | 1-2 | 1-2 |
| | | Total, % | 100 | 100 | 100 |
| | Mix kneaded product | Moisture content | 20-30 | 20-30 | 20-30 |

(continued)

| Preparation conditions of seeds | | | | | |
|---|---|---|---|---|---|
| | | | Kind of seeds (single herbal medicines and complex herbal medicies ) | | |
| | | | High | Medium | Low |
| Extru sion condi tion | Extruder Die mold | Diameter, mm | 1.2-1.5 | 1.5 | 1.5 |
| | | Die Length, cm | 1.5-2.0 | 2.0 | 2.0 |
| | Screw rate, rpm | | 30-40 | 50 | 60 |
| | Cutting length, mm | | 1.2-1.5 | 1.5 | 1.5 |

### 4.1. Preparation of seeds containing high-sugar-content extract concentrate

The high-sugar-content materials selected above were Jujubae Fructus, Fraxini Cortex, Ginseng Radix, Glycyrrhizae Radix, and Scutellariae Radix. The preparation of kneaded products added with excipients for seed preparation will be described in detail.

### 4.1.1. Manufacture of square-cylindrical pellets by extruder

As a mineral additive, at least one selected from precipitated calcium carbonate (precipitated $CaCO_3$), magnesium oxide (MgO), calcium silicate ($CaSiO_3$), magnesium silicate ($3MgSiO_3·5H_2O$), silicon dioxide ($SiO_2$), titanium dioxide ($TiO_2$), bentonite, kaolin, and talc was used. As for the standard of use, the mineral additive is contained in 25-15% per 100 g of the total excipients.

At least one kind selected from microcrystalline cellulose (MCC), lactose, stearic acid-magnesium (stearate-Mg), stearic acid-calcium (stearate-Ca), PEG 6000, beta-cyclodextrin (CD) powder, zein powder, and starch sodium octenyl succinate (SSOC) was used as a releasing agent, and a sucrose fatty acid ester powder was used as an emulsifier. The release agent was contained in 74-83% per 100 g of the total excipients, and the emulsifier accounted for the remainder 1-2%. Table 17 details the use of excipients.

[Table 17]

| Contents of excipients added, characteristics of excipients, and percentages of use by excipient types in preparation of seeds containing high-sugar-content concentrates | | | | | | |
|---|---|---|---|---|---|---|
| | | Jujubae Fructus | Fraxini Cortex | Ginseng Radix | Glycyrrh izae Radix | Scutellari ae Radix |
| Content of excipients added, % | | 65 | 60 | 55 | 50 | 45 |
| Excipient mixing percentag e, % | Mineral | 25 | 20 | 17 | 20 | 15 |
| | Release agent | 74 | 79 | 81 | 79 | 83 |
| | Emulsifier | 1 | 1 | 2 | 1 | 2 |
| | Total | 100 | 100 | 100 | 100 | 100 |
| Especial compositi onal ratio by chacteris tics | Mineral | $CaCO_3$:$TiO_2$= 1:1 | $3MgSiO_3$ : $SiO_2$=1: 1 | MgO:$CaSiO_3$ =1:1 | $TiO_2$: tal c=1:1 | bentonite: kaoliln=1: 1 |
| | Release agent | MCC:SSOC: PE G6000=0.5: 0.4: 0.1 | MCC:SSOC : Stearat e-Mg=0.5: 0.3: 0.2 | SCCO:Stea rate-Ca: CD=0.5 : 0.3: 0.2 | MCC: SCCO : Zein=0. 5: 0.3: 0.2 | SCCO: Lacto se: CD=0.5:0.3: 0.2 |
| | Emulsifier | sucrose fatty acid ester =1 | | | | |

Thereafter, the excipients required for treatment were first well mixed, and then mixed with an extract concentrate, and the prepared mix kneaded product was checked for the moisture content. For example, the moisture content of the mix kneaded product of 2000 g of a Jujubae Fructus extract and 2050 g of excipients was analyzed to be 22.5%. Table 18 shows the detailed results of preparations for extrusion with respect to five kinds of high-sugar-content single herbal medicines. The product molded in a square-cylindrical shape was dried over air at room temperature overnight, and then subjected to a grinding step by a spheronizer for manufacturing spherical pellets.

[Table 18]

| Extrusion of high-sugar-content materials | | | | | | | |
|---|---|---|---|---|---|---|---|
| | | | High-sugar-content materials | | | | |
| | | | Jujubae Fructus | Fraxini Cortex | Ginseng Radix | Glycyrrhizae Radix | Scutellariae Radix |
| Mateiral mixing conditions | | Extract Concentrate, g | 2000 | 2000 | 2000 | 2000 | 2000 |
| | | Excipient, g | 2050 | 1620 | 1520 | 1270 | 1040 |
| Moisture measurement | | Kneaded product moisture content, % | 22.5 | 25.4 | 21.4 | 22.2 | 24.0 |
| Seed conditions (Based on extract solids) | | extract solid content, % | 34.7 | 40.0 | 45.1 | 50.1 | 55.0 |
| | | Excipient content, % | 65.3 | 60.0 | 54.9 | 49.9 | 45.0 |
| Ext rus ion con dit ion s | Extruder Die mold | Diameter, mm | 1.2 | 1.2 | 1.2 | 1.5 | 1.5 |
| | | Die Length, cm | 1.5 | 1.5 | 1.5 | 2.0 | 2.0 |
| | Screw rate, rpm | | 30 | 30 | 30 | 40 | 40 |
| | Cutting length, mm | | 1.2 | 1.2 | 1.2 | 1.5 | 1.5 |

### 4.1.2. Manufacture of spherical pellets by spheronizer

The square-cylindrical product molded by an extruder was dried over air at room temperature overnight, thereby obtaining 3847.5 g of Jujubae Fructus, 3439 g of Fraxini Cortex, 3344 g of Ginseng Radix, 3106.5 g of Glycyrrhizae Radix, and 2888 g of Scutellariae Radix. Each of the single herbal medicines was ground seven times while the weight for grinding once was 200 g. Spherical pellets with a size selected after sieving were measured for hardness by using a hardness tester (USA, Copley Scientific, Hardness Tester Model TBF1000), and the breaking force was measured by a load cell at a rate of 0.2 mm/sect and expressed as kgf. Table 19 shows the spheronization conditions and results, and the sieving results were expressed as an average value.

[Table 19]

| Spheronization results of square-cylindrical pellets of high-sugar-content materials | | | | | | | | | | |
|---|---|---|---|---|---|---|---|---|---|---|
| Spheronizer | High-sugar-content materials | | | | | | | | | |
| | Jujubae Fructus | | Fraxini Cortex | | Ginseng Radix | | Glycyrrhiz ae Radix | | Scutellari ae Radix | |
| Grinding rate, rpm, and time | 1500rpm x 10min | | | | | | | | | |
| dose, g/once | 200 | | | | | | | | | |
| Sieving, mm | g | % | g | % | g | % | g | % | g | % |
| 1.40 or more | - | - | - | - | - | - | - | - | - | - |
| 1.40-1.18 | - | - | - | - | - | - | 112.7 | 56. 4 | 103.5 | 51. 8 |
| 1.18-1.00 | 104. 0 | 52.0 | 97.8 | 48.9 | 110.2 | 55. 1 | 75.1 | 37. 6 | 87.0 | 43. 5 |

(continued)

| Spheronization results of square-cylindrical pellets of high-sugar-content materials | | | | | | | | | | |
|---|---|---|---|---|---|---|---|---|---|---|
| Spheronizer | High-sugar-content materials | | | | | | | | | |
| | Jujubae Fructus | | Fraxini Cortex | | Ginseng Radix | | Glycyrrhiz ae Radix | | Scutellari ae Radix | |
| 1.00-0.85 | 82.3 | 41.1 | 88.4 | 44.2 | 80.5 | 40. 3 | 4.3 | 2.2 | 1.7 | 0.9 |
| 0.85-0.71 | 3.4 | 1.7 | 5.1 | 2.6 | 1.3 | 0.7 | 2.8 | 1.4 | 1.3 | 0.7 |
| 0.71-0.60 | 1.7 | 0.9 | 2.4 | 1.2 | 0.6 | 0.3 | - | - | 1.3 | 0.7 |
| Pan (0.6 or less) | 3.9 | 2.0 | 2.5 | 1.3 | 1.7 | 0.9 | 1.5 | 0.8 | 2.2 | 1.1 |
| Total amount of recovery | 195. 3 | 97.7 | 196.2 | 98.1 | 194.3 | 97. 2 | 196.4 | 98. 2 | 197. 0 | 98. 5 |
| Prod ucti on/o nce | 1.18 - 0.85 | 186. 3 | 93.1 | 186.2 | 93.1 | 190.7 | 95. 4 | - | - | - | - |
| | 1.40 1.00 | - | - | - | - | - | - | 187.8 | 94. 0 | 190. 5 | 95. 3 |
| | Hard ness, kgf | 20.3 | - | 19.7 | - | 18.2 | - | 18.9 | - | 16.4 | - |

As shown in Table 19, the spherical pellets used as seeds in a fluidized bed apparatus after grinding had a size of 1.18-0.85 mm for Jujubae Fructus, Fraxini Cortex, and Ginseng Radix and a size of 1.4-1.0 mm for Glycyrrhizae Radix and Scutellariae Radix. Jujubae Fructus 1302 g, Fraxini Cortex 1302 g, Ginseng Radix 1330 g, Glycyrrhizae Radix 1309 g, and Scutellariae Radix 1330g were made into the products suitable for use in a fluidized bed granulator, and the production efficiency compared with the input amount was shown to be in the range of 93-95%. The weight of spherical seeds introduced into the fluidized bed granulator was in the range of 1000-1300 g.

Regarding hardness, western medicines are generally considered to have the lowest level of hardness when broken at a pressure of 3.7-7.0 kgf, and general tablets are considered to generally have a suitable level when having a hardness of 10 kgf and considered to be very firm when having a hardness of 15-17 kgf. Therefore, the measured hardness range above was 16-20 kgf, indicating that the pellets were very firm. Such a tendency is thought to be affected by the binding strength of the sugar components contained in extract solids.

The western medicines are finished products, and the above hardness level range corresponds to suitable levels to sufficiently prevent damage to the finished products due to shaking or friction during distribution, but in cases of the present application, the prepared seeds are semifinished products, and during a subsequent fluidized bed process, a higher level of hardness thereof is more suitable since consecutive impacts and friction are induced by the spray pressure of a preparation solution and particles move infinitely up and down. As a result of actual application, in the coating process of a preparation solution by a fluidized bed granulator, which is performed below, debris and the like due to friction and abrasion were not found, and the surfaces of the finished products were also clean. However, the hardness of the existing sugar spheres was in the range of 0.85-1.58 kgf, and sugar spheres having an average hardness of 1.25 kgf, when used as seeds for a fluidized bed, were highly likely to break or smash.

**4.2. Preparation of seeds containing medium-sugar-content extract concentrates**

The medium-sugar-content materials selected above were Atractylodis Rhizoma, Coptidis Rhizoma, Zingiberis Rhizoma Recens, Paeoniae Radix, and Ephedrae Herba. The preparation of kneaded products added with excipients for seed preparation will be described in detail.

**4.2.1. Manufacture of square-cylindrical pellets by extruder**

As a mineral additive, at least one selected from precipitated calcium carbonate (precipitated $CaCO_3$), magnesium oxide (MgO), calcium silicate ($CaSiO_3$), magnesium silicate ($3MgSiO_3 \cdot 5H_2O$), silicon dioxide ($SiO_2$), titanium dioxide ($TiO_2$), bentonite, kaolin, and talc was used. As for the standard of use, the mineral additive is contained in 10-20% per 100 g of the total excipients.

At least one kind selected from microcrystalline cellulose (MCC), lactose, stearic acid-magnesium (stearate-Mg), stearic acid-calcium (stearate-Ca), PEG 6000, beta-cyclodextrin (CD) powder, zein powder, and starch sodium octenyl succinate (SSOC) was used as a releasing agent, and a sucrose fatty acid ester powder was used as an emulsifier. The release agent was contained in 78-88% per 100 g of the total excipients, and the emulsifier accounted for the remainder 1-2%. Table 20 details the use of excipients.

[Table 20]

| Contents of excipients added, characteristics of excipients, and percentages of use by excipient types in preparation of seeds containing medium-sugar-content concentrates | | | | | | |
|---|---|---|---|---|---|---|
| | | Atractylodi s Rhizoma | Coptidis Rhizoma | Zingiberi s Rhizoma Recens | Paeoniae Radix | Ephedrae Herba |
| Content of excipients added, % | | 55 | 50 | 45 | 40 | 35 |
| Excipien t mixing percenta ges, % | Mineral | 20 | 20 | 15 | 15 | 10 |
| | Release agent | 78 | 79 | 84 | 84 | 88 |
| | Emulsifier | 2 | 1 | 1 | 1 | 2 |
| | Total | 100 | 100 | 100 | 100 | 100 |
| Especial composit ional ratio by chacteri zation | Mineral | $Caco_3$: $Tio_2$ = 1:1 | $3MgSio_3$:$Si\,o_2$ = 1:1 | $Mgo$:$CaSio_3$ =1:1 | $Tio_2$. tal c = 1: 1 | Bentonite : Kaolin = 1 : 1 |
| | Release agent | MCC:SSOC: PE G6000=0.5: 0.4: 0.1 | MCC:SSOC: Stearate-Mg=0.5: 0.3: 0.2 | SCCO:Stea rate-Ca: CD=0.5 : 0.3: 0.2 | MCC: SCCO : Zein=0. 5: 0.3: 0.2 | SCCO:Lact ose:CD=0. 5: 0.3: 0.2 |
| | Emulsifier | Sucrose fatty acid ester = 1 | | | | |

Thereafter, the excipients required for treatment were first well mixed, and then mixed with an extract concentrate, and the prepared mix kneaded product was checked for the moisture content. For example, the moisture content of the mix kneaded product of 2500 g of an Atractylodis Rhizoma extract and 1640 g of excipients was analyzed to be 27.9%. Table 21 shows the detailed results of preparations for extrusion with respect to five kinds of medium-sugar-content single herbal medicines. The product molded in a square-cylindrical shape was dried over air at room temperature overnight, and then subjected to a grinding step by a spheronizer for manufacturing spherical pellets.

[Table 21]

| Extrusion of medium-sugar-content materials | | | | | | |
|---|---|---|---|---|---|---|
| | | Medium-sugar-content materials | | | | |
| - | | Atracty lodis Rhizoma | Copti dis Rhizo ma | Zingi beris Rhizo ma Recen s | Paeon iae Radix | Ephed rae Herba |
| Mateiral mixing conditions | Concentrate,g | 2500 | 3000 | 2500 | 2500 | 3000 |
| | Excipients, g | 1640 | 1670 | 1260 | 1135 | 1040 |
| Moisture measurement | Kneaded product moisture content, % | 27.9 | 28.5 | 25.5 | 21.9 | 26.4 |
| Seed conditions (Based on extract solids) | Extract solid content, % | 45.1 | 50.0 | 55.0 | 60.0 | 65.0 |
| | Excipient content, % | 54.9 | 50.0 | 45.0 | 40.0 | 35.0 |

(continued)

| Extrusion of medium-sugar-content materials | | | | | | | |
|---|---|---|---|---|---|---|---|
| - | | Medium-sugar-content materials | | | | | |
| | | | Atracty lodis Rhizoma | Copti dis Rhizo ma | Zingi beris Rhizo ma Recen s | Paeon iae Radix | Ephed rae Herba |
| Extrus ion condit ions | Extruder Die mold | Diameter, mm | 1.5 | 1.5 | 1.5 | 1.5 | 1.5 |
| | | Die Length, cm | 2.0 | 2.0 | 2.0 | 2.0 | 2.0 |
| | Screw rate, rpm | | 50 | 50 | 50 | 50 | 50 |
| | Cutting length, mm | | 1.5 | 1.5 | 1.5 | 1.5 | 1.5 |

## 4.2.2. Manufacture of spherical pellets by spheronizer

The square-cylindrical product molded by an extruder was dried over air at room temperature overnight, thereby obtaining 3920 g of Atractylodis Rhizoma, 4435 g of Coptidis Rhizoma, 3550 g of Zingiberis Rhizoma Recens, 3465 g of Paeoniae Radix, and 3835 g of Ephedrae Herba. Each of the single herbal medicines was ground seven times while the weight for grinding once was 200 g. Spherical pellets used in the fluidized bed granulator after sieving were measured for hardness by using a hardness tester (USA, Copley Scientific, Hardness Tester Model TBF1000), and the breaking force was measured by a load cell at a rate of 0.2 mm/sect and expressed as kgf. Table 22 shows the spheronization conditions and results, and the sieving results were expressed as an average value.

[Table 22]

| Spheronization results of square-cylindrical pellets of medium-sugar-content materials | | | | | | | | | | |
|---|---|---|---|---|---|---|---|---|---|---|
| Spheronizer | Medium-sugar-content materials | | | | | | | | | |
| | Atractylod is Rhizoma | | Coptidis Rhizoma | | Zingiberis Rhizoma Recens | | Paeoniae Radix | | Ephedrae Herba | |
| Grinding rate, rpm, and time | 1500rpm X 10min | | | | | | | | | |
| Dose, g/once | 200 | | | | | | | | | |
| Sieving, mm | g | % | g | % | g | % | g | % | g | % |
| 1.40 or more | - | - | - | - | - | - | - | - | - | - |
| 1.40-1.18 | 102. 0 | 51.0 | 110.4 | 55.2 | 97.4 | 48. 7 | 108.0 | 54. 0 | 114. 0 | 57. 0 |
| 1.18-1.00 | 83.7 | 41.9 | 78.8 | 39.4 | 85.0 | 42. 5 | 77.9 | 39. 0 | 78.0 | 39. 0 |
| 1.00-0.85 | 5.2 | 2.6 | 3.8 | 1.9 | 4.4 | 2.2 | 6.3 | 3.2 | 1.9 | 1.0 |
| 0.85-0.71 | 2.8 | 1.4 | 1.8 | 0.9 | 2.6 | 1.3 | 1.7 | 0.9 | 2.1 | 1.1 |
| 0.71-0.60 | - | - | - | - | 2.3 | 1.2 | - | - | - | - |
| Pan (0.6 or less) | 2.4 | 1.2 | 2.6 | 1.3 | 3.2 | 1.6 | 2.1 | 1.1 | 1.4 | 0.7 |
| Total amount | 196. 1 | 98.1 | 197.4 | 98.7 | 194.9 | 97. 5 | 196.0 | 98. 0 | 197. 4 | 98. 7 |

(continued)

| Spheronization results of square-cylindrical pellets of medium-sugar-content materials | | | | | | | | | | | |
|---|---|---|---|---|---|---|---|---|---|---|---|
| Spheronizer | | Medium-sugar-content materials | | | | | | | | | |
| | | Atractylod is Rhizoma | | Coptidis Rhizoma | | Zingiberis Rhizoma Recens | | Paeoniae Radix | | Ephedrae Herba | |
| Prod ucti on/o nce | 1.40 1.00 | 185. 7 | 92.9 | 189.2 | 94.6 | 182.4 | 91. 2 | 185.9 | 93. 0 | 192. 0 | 96. 0 |
| | Hard ness, kgf | 17.7 | - | 16.1 | - | 18.8 | - | 18.3 | - | 17.6 | - |

As shown in Table 22, the spherical pellets of medium-sugar-content herbal medicines, used as seeds in a fluidized bed granulator after grinding, were all 1.40-1.00 mm in size. The amount of products with a size suitable for the standard was 1300 g for Atractylodis Rhizoma, 1325 g for Coptidis Rhizoma, 1275 g for Zingiberis Rhizoma Recens, 1300 g for Paeoniae Radix, and 1345 g for Ephedrae Herba, and the production efficiency of standardized products compared with the input amount was shown to be in the range of 91-96%. In addition, the measured hardness range of the spherical pellets having a selected size was measured to be a similar range to the seeds of the high-sugar-content herbal medicines.

### 4.3. Preparation of seeds containing low-sugar-content extract concentrates

The low-sugar-content materials selected above were Pinelliae Rhizoma, Cinnamomi Cortex Spissus, Aurantii Immaturus Fructus, Bupleuri Radix, and Magnoliae Cortex. The preparation of kneaded products added with excipients for seed preparation will be described in detail.

### 4.3.1. Manufacture of square-cylindrical pellets by extruder

As a mineral additive, at least one selected from precipitated calcium carbonate (precipitated $CaCO_3$), magnesium oxide (MgO), calcium silicate ($CaSiO_3$), magnesium silicate ($3MgSiO_3 \cdot 5H_2O$), silicon dioxide ($SiO_2$), titanium dioxide ($TiO_2$), bentonite, kaolin, and talc was used. As for the standard of use, the mineral additive is contained in 5-15% per 100 g of the total excipients.

At least one kind selected from microcrystalline cellulose (MCC), lactose, stearic acid-magnesium (stearate-Mg) , stearic acid-calcium (stearate-Ca), PEG 6000, beta-cyclodextrin (CD) powder, zein powder, and starch sodium octenyl succinate (SSOC) was used as a releasing agent, and a sucrose fatty acid ester powder was used as an emulsifier. The release agent was contained in 83-94% per 100 g of the total excipients, and the emulsifier accounted for the remainder 1-2%. Table 23 details the use of excipients.

[Table 23]

| Contents of excipients added, characteristics of excipients, and percentages of use by excipient types in preparation of seeds containing low-sugar-content concentrates | | | | | | |
|---|---|---|---|---|---|---|
| | | Pinelliae Rhizoma | Cinnamomi Cortex Spissus | Aurantii Immaturus Fructus | Bupleuri Radix | Magnoliae Cortex |
| Content of excipients added, % | | 50 | 45 | 40 | 35 | 30 |
| Excipient mixing percentage s, % | Mineral | 5 | 10 | 10 | 15 | 15 |
| | Release agent | 94 | 89 | 88 | 83 | 84 |
| | Emulsifier | 1 | 1 | 2 | 2 | 1 |
| | Total | 100 | 100 | 100 | 100 | 100 |

(continued)

| Contents of excipients added, characteristics of excipients, and percentages of use by excipient types in preparation of seeds containing low-sugar-content concentrates | | | | | | |
|---|---|---|---|---|---|---|
| | | Pinelliae Rhizoma | Cinnamomi Cortex Spissus | Aurantii Immaturus Fructus | Bupleuri Radix | Magnoliae Cortex |
| Especial compositio nal ratio by characteri stics | Mineral | $CaCO_3$: $TiO_2$ =1:1 | $3MgSiO_3$: $SiO_2$=1: 1 | MgOn:CaSi$O_3$=1:1 | $TiO_2$: tal c=1:1 | bentonite : kaolin=1 :1 |
| | Release agent | MCC:SSOC: PEG6000=0 . 5: 0.4: 0.1 | MCC:SSOC: Stearate- Mg=0.5: 0.3: 0.2 | SCCO:Stea rate-Ca: CD=0.5 : 0.3: 0.2 | MCC:SCCO : Zein=0. 5: 0.3: 0.2 | SCCO:Lact ose:CD=0. 5: 0.3: 0.2 |
| | Emulsifier | Sucrose fatty acid ester=1 | | | | |

The excipients required for treatment were first well mixed, and then mixed with an extract concentrate, and the prepared mix kneaded product was checked for the moisture content. For example, the moisture content of the mix kneaded product of 4000 g of a Pinelliae Rhizoma extract and 2420 g of excipients was analyzed to be 24.6%. Table 24 shows the detailed results of preparations for extrusion with respect to five kinds of low-sugar-content single herbal medicines. The product molded in a square-cylindrical shape was dried over air at room temperature overnight, and then subjected to a grinding step by a spheronizer for manufacturing spherical pellets.

[Table 24]

| Extrusion of low-sugar-content materials | | | | | | | |
|---|---|---|---|---|---|---|---|
| - | | Low-sugar-content materials | | | | | |
| | | Pinel liae Rhizo ma | Cinnam omi Cortex Spissu s | Aurant ii Immatu rus Fructu s | Buple uri Radix | Magno liae Corte x | |
| Mateiral mixing conditions | concentrate, g | 4000 | 4000 | 4000 | 5000 | 4000 | |
| | excipients, g | 2420 | 1860 | 1570 | 1760 | 1205 | |
| Moisture measurement | Kneaded product moisture content, % | 24.6 | 29.7 | 29.5 | 25.7 | 23.0 | |
| Seed conditions (Based on extract solids) | extract solid content, % | 50.0 | 54.9 | 60.0 | 65.0 | 69.9 | |
| | excipients content, % | 50.0 | 45.1 | 40.0 | 35.0 | 30.1 | |
| Extru sion condi tions | Extruder Die | Diameter, mm | 1.5 | 1.5 | 1.5 | 1.5 | 1.5 |
| | | Die Length, cm | 2.0 | 2.0 | 2.0 | 2.0 | 2.0 |
| | screw rate, rpm | 60 | 60 | 60 | 60 | 60 | |
| | Cutting length, mm | 1.5 | 1.5 | 1.5 | 1.5 | 1.5 | |

### 4.3.2. Manufacture of spherical pellets by spheronizer

The square-cylindrical product molded by an extruder was dried over air at room temperature overnight, thereby obtaining 6100 g of Pinelliae Rhizoma, 5560 g of Cinnamomi Cortex Spissus, 5285 g of Aurantii Immaturus Fructus, 6415 g of Bupleuri Radix, and 5055 g of Magnoliae Cortex. Each of the single herbal medicines was ground seven times while the weight for grinding once was 200 g. Spherical pellets used in the fluidized bed apparatus after sieving were measured for hardness by using a hardness tester (USA, Copley Scientific, Hardness Tester Model TBF1000), and the breaking force was measured by a load cell at a rate of 0.2 mm/sect and expressed as kgf. Table 25 shows the spheronization conditions and results, and the sieving results were expressed as an average value

[Table 25]

| Spheronization results of square-cylindrical pellets of low-sugar-content materials | | | | | | | | | | | |
|---|---|---|---|---|---|---|---|---|---|---|---|
| Spheronizer | | Low-sugar-content materials | | | | | | | | | |
| | | Pinelliae Rhizoma | | Cinnamomi Cortex Spissus | | Aurantii Immaturus Fructus | | Bupleuri Radix | | Magnoliae Cortex | |
| Grinding rate, rpm, and time | | 1500rpm x 10min | | | | | | | | | |
| Dose, g | | 200 | | | | | | | | | |
| Sieving, mm | | g | % | g | % | g | % | g | % | g | % |
| 1.40 or more | | | | | | | | | | | |
| 1.40-1.18 | | 105.8 | 52.9 | 95.3 | 47.7 | 102.3 | 51. 2 | 114.0 | 57. 0 | 107.6 | 53. 8 |
| 1.18-1.00 | | 83.0 | 41.5 | 84.4 | 42.2 | 82.6 | 41. 3 | 75.4 | 37. 7 | 81.4 | 40. 7 |
| 1.00-0.85 | | 4.3 | 2.2 | 6.5 | 3.3 | 5.8 | 2.9 | 3.2 | 1.6 | 3.7 | 1.9 |
| 0.85-0.71 | | 2.1 | 1.1 | 2.8 | 1.4 | 1.5 | 0.8 | 2.4 | 1.2 | 1.3 | 0.7 |
| 0.71-0.60 | | - | - | 1.8 | 0.9 | 1.3 | 0.7 | - | - | - | - |
| Pan (0.6 or less) | | 1.8 | 0.9 | 2.7 | 1.4 | 2.5 | 1.3 | 2.3 | 1.2 | 1.5 | 0.8 |
| Total amount | | 197.0 | 98.5 | 193.5 | 96.8 | 196.0 | 98. 0 | 197.3 | 98. 7 | 195.5 | 97. 8 |
| Producti on/o nce | 1.40 - 1.00 | 188.8 | 94.4 | 179.7 | 89.9 | 184.9 | 92. 5 | 189.4 | 94. 7 | 189.0 | 94. 5 |
| | Hard ness, kgf | 16.4 | - | 15.1 | - | 16.2 | - | 15.3 | - | 15.8 | - |

As shown in Table 25, the spherical pellets of low-sugar-content herbal medicines, used as seeds in a fluidized bed granulator after grinding, were all 1.40-1.00 mm in size. The amount of products with a size suitable for the standard was 1320 g of Pinelliae Rhizoma, 1255 g of Cinnamomi Cortex Spissus, 1295 g of Aurantii Immaturus Fructus, 1325 g of Bupleuri Radix, and 1320 g of Magnoliae Cortex, and the production efficiency of standardized products compared with the input amount was shown to be in the range of 89-95%, showing 94.3% for Pinelliae Rhizoma, 89.6% for Cinnamomi Cortex Spissus, 92.5% for Aurantii Immaturus Fructus, 94.6% for Bupleuri Radix, and 94.3% for Magnoliae Cortex. In addition, the measured hardness range of the spherical pellets having a selected size was measured to be a somewhat lower range than the seeds of the high-and medium-sugar-content herbal medicines.

**4.4. Preparation of seeds containing extract concentrates of complex herbal decoctions**

The preparation of seeds containing complex herbal decoction concentrates was also carried out under the same conditions for preparing seeds of single herbal medicines, as show in the general seed preparation conditions of Table 16. The complex herbal decoctions representing respective sugar content types were *Gumiganghwal-tang, Mahwang-tang, Doinseunggi-tang.*

**4.4.1. Preparation of square-cylindrical pellets by extruder**

As a mineral additive, at least one selected from precipitated calcium carbonate (precipitated CaCO$_3$), mag-

nesium oxide (MgO), calcium silicate (CaSiO$_3$), magnesium silicate (3MgSiO$_3$-5H$_2$O), silicon dioxide (SiO$_2$), titanium dioxide (TiO$_2$), bentonite, kaolin, and talc was used. The kind and use range of a mineral additive were the same as in the single herbal medicines, that is, the mineral additive was contained in 15-25% for the high-sugar-content complex herbal decoction, 10-20% for the medium-sugar-content complex herbal decoction, and 5-15% for the low-sugar-content complex herbal decoction, relative to 100 g of the total excipients.

At least one kind selected from microcrystalline cellulose (MCC), lactose, stearic acid-magnesium (stearate-Mg), stearic acid-calcium (stearate-Ca), PEG 6000, beta-cyclodextrin (CD) powder, zein powder, and starch sodium octenyl succinate (SSOC) was used as a releasing agent, and a sucrose fatty acid ester powder was used as an emulsifier. Table 26 details the use of excipients.

[Table 26]

| Contents of excipients added, characteristics of excipients, and percentages of use by excipient types in preparation of seeds containing complex herbal decoction concentrates | | | | |
|---|---|---|---|---|
| | | High-sugar-content complex herbal medicine | Medium-sugar-contentcomplex herbal medicine | Low-sugar-contentcomplex herbal medicine |
| | | *Gumiganghwal-tang* | *Mahwang-tang* | *Doinseunggi-tang* |
| Content of excipients added, % | | 45 | 35 | 30 |
| Excipient mixing percentages, % | Mineral | 20 | 15 | 10 |
| | Release agent | 78 | 83 | 88 |
| | Emulsifier | 2 | 2 | 2 |
| | Total | 100 | 100 | 100 |
| Especial compositional ratio by chacteristics | Mineral | CaCo$_3$:Tio$_2$=1:1 | 3MgSio$_3$:Sio$_2$=1:1 | Mgo:CaSio$_3$=1:1 |
| | Release agent | MCC:SSOC: PEG6000 =0.5: 0.4: 0.1 | MCC:SSOC:Stearat e-Mg=0.5: 0.3: 0.2 | SCCO:Stearate-Ca: CD=0.5: 0.3: 0.2 |
| | Emulsifier | Sucrose fatty acid ester=1 | | |

Thereafter, the excipients required for treatment were first well mixed, and then mixed with an extract concentrate, and the prepared mix kneaded product was checked for the moisture content. For example, the moisture content of the mix kneaded product of 3000 g of a *Gumiganghwal-tang* extract and 1450 g of excipients was analyzed to be 27.7%. Table 27 shows the detailed results of preparations for extrusion with respect to the other two kinds of complex herbal decoctions. The product molded in a square-cylindrical shape was dried over air at room temperature overnight, and then subjected to a grinding step by a spheronizer for manufacturing spherical pellets.

[Table 27]

| Extrusion conditions for manufacturing seeds for complex herbal decoctions | | | | |
|---|---|---|---|---|
| Classification | | High-sugar-content complex herbal medicine | Medium-sugar-content complex herbal medicine | Low-sugar-content complex herbal medicine |
| | | *Gumiganghwal -tang* | *Mahwang-tang* | *Doinseungg i-tang* |
| Mateiral mixing conditions | concentrate,g | 3000 | 4000 | 4000 |
| | excipients, g | 1450 | 1300 | 1105 |
| Moisture measurement | Kneaded product moisture content, % | 27.7 | 30.0 | 27.8 |

(continued)

| Extrusion conditions for manufacturing seeds for complex herbal decoctions | | | High-sugar-content complex herbal medicine | Medium-sugar-content complex herbal medicine | Low-sugar-content complex herbal medicine |
|---|---|---|---|---|---|
| Classification | | | *Gumiganghwal -tang* | *Mahwang-tang* | *Doinseungg i-tang* |
| Seed conditions (Based on extract solids) | | extract solid content, % | 54.9 | 64.9 | 70.0 |
| | | Excipient content, % | 45.1 | 35.1 | 30.0 |
| Extr usio n cond itio ns | Extruder Die | Diameter, mm | 1.2 | 1.5 | 1.5 |
| | | Die Length, cm | 1.5 | 2.0 | 2.0 |
| | screw rate, rpm | | 40 | 50 | 60 |
| | Cutting Length, cm | | 1.2 | 1.5 | 1.5 |

### 4.4.2. Manufacture of spherical pellets by spheronizer

The square-cylindrical product molded by an extruder was dried over air at room temperature overnight, thereby obtaining 4190 g of *Gumiganghwal-tang,* 5045 g of *Mahwang-tang,* and 4770 g of *Doinseunggi-tang.* Each of the single herbal medicines was ground seven times while the weight for grinding once was 200 g. Spherical pellets used in the fluidized bed apparatus after sieving were measured for hardness by using a hardness tester (USA, Copley Scientific, Hardness Tester Model TBF1000), and the breaking force was measured by a load cell at a rate of 0.2 mm/sect and expressed as kgf. Table 28 shows the spheronization conditions and results, and the sieving results were expressed as an average value.

[Table 28]

| Spheronization results of square-cylindrical pellets of complex herbal medicines | | | | | | | |
|---|---|---|---|---|---|---|---|
| Spheronizer | | Complex herbal medicines (by high, medium, and low sugar contents) | | | | | |
| | | *Gumiganghwal-tang* | | *Mahwang-tang* | | *Doinseunggi-tang* | |
| Grinding rate, rpm, and time | | 1500rpm X 10min | | | | | |
| Dose, g | | 200 | | 200 | | 200 | |
| Sieving, mm | | g | % | g | % | g | % |
| 1.40 or more | | - | - | - | - | - | - |
| 1.40-1.18 | | - | - | 105.3 | 52.7 | 112.3 | 56.2 |
| 1.18-1.00 | | 126.4 | 63.2 | 85.0 | 42.5 | 78.5 | 39.3 |
| 1.00-0.85 | | 65.3 | 32.7 | 2.2 | 1.1 | 3.4 | 1.7 |
| 0.85-0.71 | | 2.6 | 1.3 | 1.3 | 0.7 | 1.7 | 0.9 |
| 0.71-0.60 | | - | - | - | - | - | - |
| Pan(0.6 or less) | | 3.4 | 1.7 | 2.8 | 1.4 | 2.6 | 1.3 |
| Total amount | | 197.7 | 98.9 | 196.6 | 98.3 | 198.5 | 99.3 |
| Product ion/onc e | 1.18-0.85 | 191.7 | 95.9 | - | - | - | - |
| | 1.40-1.00 | - | - | 190.3 | 95.2 | 190.8 | 95.4 |
| | Hardness, kgf | 18.3 | - | 17.7 | - | 15.4 | - |

As shown in Table 28, the spherical pellets used as seeds in a fluidized bed granulator after grinding had a size of 1.18-0.85 mm for *Gumi ganghwal-tangand* a size of 1.40-1.00 mm for *Mahwang-tang* and *Doinseunggi-tang.* The amount of products with a size suitable for the standard was 1340 g of *Gumiganghwal-tang* seeds, and 1330 g and 1335 g of *Mahwang-tang* and *Doinseunggi-tang* seeds, respectively. The production efficiency of standardized products compared with the input amount was shown to be a level of 95%, which were similar for the above seeds. In addition, the measured hardness range of the spherical pellets having a selected size was measured to show a somewhat lower tendency in *Doinseunggi-tang,* which is a low-sugar-content herbal extract.

**Example 5: Concentrate coating in fluidized bed granulator using seeds**

In the manufacture of final formulations, a fluidized bed process may smoothen the surface of a final product and shape the final product as a clean sphere, but requires a long time for basic processes and has many problems, for example, as in a case of a high sugar content, the time for preparing a spray solution added with additives and the spray time are long, and thus the previously coated components are concentrated on surfaces, causing sticking between particles. Moreover, the formulating through extrusion and grinding is somewhat easy compared with a fluidized bed process, but results in a non-favorable shape and surface. Therefore, in the present application, as many extract solids as possible are contained in the process of extrusion and grinding, and as small amounts as possible or an adequate amount of extract solids is used in the fluid bed process.

Hence, the content range of extract solids contained in the final formulation was set according to the characteristics of extracts, and a test was conducted for the set range, and then the set range was adjusted again.

The coating of an extract concentrate in a fluidized bed granulator was carried out according to the following sequence. That is, considering the characteristics of the extract concentrate for a fluidized bed granulator, prepared in the above examples, optimal excipients were selected and the additional amounts thereof were determined, and separately prepared essential oil capsules were added together to prepare a preparation solution for spraying. In the fluidized bed coating process, when a preparation solution is sprayed, sugar components are concentrated through the volatilization of moisture, causing a sharp increase in stickiness, resulting in the agglomeration of pellet particles (aggregate formation). Therefore, the preparation solution to be sprayed is required to have appropriate viscosity and sticking strength, retain uniform spreadability on the particle surface, and prevent agglomeration by mitigating the sticking strength of sugar components. Therefore, in the present disclosure, in order to increase the extract solid content, the extract concentration degree in the concentrates for seed preparation was increased to allow the extract solids contained in the prepared seeds to be maintained at a high content, whereas the solid content and the sugar content of the extract concentrates used for a fluidized bed granulator were differently adjusted to have lower levels according to the characteristics of medicinal herbs. In addition, the ranges of excipients added to the extract concentrates for coating used in the fluidized bed process were set to 10-15, 5-10, and 3-8%, respectively (Table 29), and the kinds and additional amounts of excipients used were controlled (Table 31) to achieve prompt drying, and the solutions were concentrated so as not to have excessive sticking strength due to sugar components. In addition, the content of extract solids in the fluidized bed process was increased to a range of 20-25% (Table 29) to promptly manufacture spherical pellets containing ultimately desired solid extracts.

Table 29 summarizes the manufacturing conditions of final spherical pellets by a fluidized bed process. However, as for the low-sugar-content material, Pinelliae Rhizoma, the sugar content of the concentration for a fluidized bed was set to 10-15.

[Table 29]

| Manufacturing conditions of final spherical pellets by fluidized bed process | | | |
|---|---|---|---|
| Classification | Single and complex herbal medicines | | |
| | High | Medium | Low |
| Seed extract solid content, % | 35-55 | 45-65 | 50-70 |
| Concentrate for fluidized bed, sugar content (brix) | 15-35 | 25-45 | 20-35 |
| Level of excipients added, % | 10-15 | 5-10 | 3-8 |
| Extract solid increase in fluidized bed process, % | 20-25 | 20-25 | 20-25 |
| Final pellet extract solid content, % | 55-80 | 60-90 | 70-95 |

The operating conditions of the fluidized bed granulator were summarized in Table 30. In Table 30, the final coating step refers to a coating step of ethyl cellulose (EC) or shellac dissolved in ethanol.

[Table 30]

| Operating conditions of bottom-spray type fluidized bed granulator having a finished product production capacity of 5 kg/batch | | | | |
|---|---|---|---|---|
| classification | | Operation and initial stage (within 60 min after operation ) | Stabilization maintaining step | Final coating step |
| Spray of preparation solution, ml/hr | High-sugar-content | 100 ⇝ 400 | 400 ⇝ 700 | - |
| | Medium-sugar-content | 300 ⇝ 800 | 800 ⇝ 1500 | - |
| | Low-sugar-content | 600 ⇝ 1000 | 1000 ⇝ 2500 | - |
| Product temperature, °C | | 60 ⇝ 70 | 70 ⇝ 85 | 40 - 45 |
| spray pressure, kg/cm$^2$ | | 1.5 ⇝ 2.0 | 2.0 ⇝ 4.0 | 2.0 ⇝ 2.5 |
| preparation solujtion for final coating, ml/hr | | - | - | 500 ⇝ 1000 |
| Partition column gap, cm | | 1.0 ⇝ 2.0 | 2.0 ⇝ 4.0 | 4.0 |
| *Partition column=Draft tube=Wurster tube | | | | |

Even though various additives were generally used in the preparation of a preparation solution for spray in a fluidized bed apparatus, the additives selected through preliminary tests were delimited as additives in the present disclosure. The selected additives were a mixture of precipitated calcium carbonate, stearic acid-magnesium, and PEG6000, a mixture of HPMC, pullulan, and glutinous starch, and starch sodium octenyl succinate, and sucrose fatty acid ester was used as an emulsifier. Table 3 shows the details of mixing. As for the levels of the excipients added, the excipients were added in 10-15% for the high sugar content, 5-10% for the medium sugar content, and 3-8% for the low sugar content, on the basis of extract solids of a concentrate for a fluidized bed.

[Table 31]

| Addition level of excipients in preparation solution for fluidized bed and mixing percentages by excipient types | | High | Mediu m | Low |
|---|---|---|---|---|
| Classification | | High | Mediu m | Low |
| Total addition level of excipients, $(based on concentrate extract solids) | | 10-15 | 5-10 | 3-8 |
| Percen tages by excipi ent types, % | Precipitated CaCO$_3$: Stearate-Mg: PEG6000 =50:30:20 | 40-50 | 15-25 | 5-15 |
| | HPMC: pullulan: glutinous starch = 60: 20:20 | 5-15 | 25-35 | 50-70 |
| | Starch sodium octenyl Succinate | 35-45 | 35-45 | 35-45 |
| | Emulsifier(Sucrose fatty acid ester) | 2-8 | 2-8 | 2-8 |
| | Total, % | 100 | 100 | 100 |

EC or shellac was used for the final external coating of finished spherical pellets. As for the level of use, the coating agent was used in 2% relative to the sum of the weight of seeds introduced into the fluidized bed apparatus and the weight of the dried matter of the preparation solution sprayed.

The contents of marker components contained in each of the finally completed spherical pellets were analyzed using ultra-performance liquid chromatography (UPLC; Waters ACQUITY™ ultra performance LC system, USA) equipped with Waters ACQUITY™ photodiode array detector (PDA), and a detection wavelength of 208-345nm was used according to the marker components. Waters ACQUITY™ BEH C18 (Octadecylsilca gel) column (1.7 μm, 2.1 × 100) was used, and Empower software was used.

### 5.1. High-sugar-content single herbal medicines

The seeds containing extracts of Jujubae Fructus, Fraxini Cortex, Ginseng Radix, Glycyrrhizae Radix, and Scutellariae Radix, as high-sugar-content single herbal medicines, prepared above, were introduced at 1000 g for each case into a fluidized bed apparatus. A homogenized preparation solution was prepared by adding an extract concentrate for a fluidized bed required and additives required according to the level of extract solids contained in the finally completed spherical pellets. The levels and percentages of excipients added in the preparation of a preparation solution for fluidized bed spraying were in the range of 10-15% for a high-sugar-content herbal medicine as shown in Tables 31 and 32. The excipients were used in 15% for Jujubae Fructus, 12% for Fraxini Cortex, and 10% for Ginseng Radix, Glycyrrhizae Radix, and Scutellariae Radix.

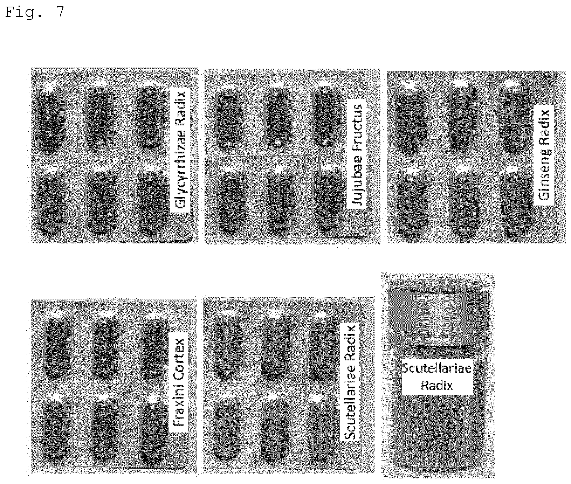

The contents of extract solids and the contents of marker components, which were contained in the final spherical pellets completed according to the operating conditions of the fluidized bed apparatus in Table 30, are shown by herbal medicines in Table 32. FIG. 7 shows that the transparent hard capsules were filled with the completed shapes for each case.

Therefore, the final step for completing the spherical pellets was a step wherein the extract concentrate for fluidized bed spraying prepared above for use in the fluidized bed apparatus was coated on the surfaces of the seeds. Table 32 below shows the amounts of the extract concentrates for fluidized bed coating used. As an example, the fluidized bed coating process of the seeds containing Jujubae Fructus extract solids, which were prepared as seeds of a high-sugar-content single herbal medicine, will be described in detail.

(1) The Jujubae Fructus seeds were introduced at 1000 g into a bottom-spray type fluidized bed granulator.

20 The extract concentrate having a solid content of 18.7% prepared above was prepared 3500 g for fluidized bed coating. In the extract concentrate, 654.5 g of solids were contained.

③ The additives having a weight of 15% of 654.5 g of the solids were prepared. The kinds of additives and the mutual percentages thereof are shown in Table 31. That is, 98.2 g of additives containing: 1) 45% of an additive of precipitated $CaCO_3$, stearic acid-magnesium, and PEG600 mixed at a ratio of 50:30:20; 2) 10% of an additive of HPMC, pullulan, and glutinous starch mixed at a ratio of 60:20:20; 3) 40% of starch sodium octenyl succinate; and 4) 5% of sucrose fatty acid ester, that is, containing four kinds of additives in 45, 10, 40, and 5% were mixed with the concentrate to prepare a homogeneous solution.

④ The concentrate was coated on the surfaces of the seeds while the operating conditions of the fluidized bed apparatus, that is, the spray pressure, the spray amount, the gap between the bottom plate and the partition column, and the temperature of a fluidization room (product temperature) were controlled and gently increased as shown in Table 30.

(5) Upon the completion of coating with the prepared concentrate, the temperature of the fluidization room was lowered to 40°C, and 35 g of ethyl cellulose was measured corresponding to a weight of 2% of the calculated total weight of solids, that is, 1000 g of the seeds, 654.5 g of the concentrate extract solids, and 98.2 g of the additives. A solution of 1% ethyl cellulose obtained by dissolving ethyl cellulose in ethanol as a solvent was sprayed to coat the seeds, thereby completing spherical pellets.

60 The completed spherical pellets were analyzed for marker components. The total sugar content was measured for Jujubae Fructus, which has no specifically indicated marker component, and corresponding components were analyzed by UPLC for the other single herbal medicines. The single herbal medicines used were analyzed wherein the total sugar content in Jujubae Fructus was 65%, the content of hesperidin in Fraxini Cortex was 4.0%, the content of ginsenosides (Rg1+Rb1) in Ginseng Radix was 0.3%, the content of glycyrrhizic acid in Glycyrrhizae Radix was 2.5%, and the content of marker components (baicalin + baicalein + ugonin) in Scutellariae Radix was 10%. The contents of marker components contained in the completed spherical pellets are shown in Table 32.

The other single herbal medicines and complex herbal medicines were also analyzed by the methods corresponding to the above. The manufacturing results of spherical pellets and images thereof are sequentially shown below. The content of extract solids contained in the actual spherical pellets obtained after the completion of the fluidized bed process or the total percentage of essential oil contained in the extract solids were calculated on the basis of actual weights obtained after the completion of the fluidized bed process.

[Table 32]

| Manufacturing results of spherical pellets of five kinds of high-sugar-content single herbal medicines | | | |
|---|---|---|---|
| Jujubae fructus | | | |
| Seed process | Seeds, g | 1000 | Extract solids (34.7%) 347g |
| Fluidized bed processing | Extract solids of concentrate for fluidized bed, g | 654.5 | 3500 g used x extract solids 18.7% |
| | Excipients added to spray solution, g | 98.2 | Extract solids654.5 g x 15% |
| | Final coating agent (EC),g | 35 | 1000+654.5+98.2=1752.7g, 2% used |
| | Spherical pellet production amount,g | 1775 | 1000+654.5+98.2+35=1787.2g |
| Marker component analysis | Extract solids content,% | 56.4 | (347+654.5)/1775 x 100=56.4 |
| | Marker component content,mg/g | 378.7 | Total sugar |
| Fraxini cortex | | | |
| Seed process | Seeds, g | 1000 | Extract solids(40.0%)=400 g |
| Fluidized bed processing | Extract solids of concentrate for fluidized bed,g | 682 | Extract solids28.9% x 2360 g used |
| | Excipients added to spray solution,g | 82 | Extract solids (682) x 12% |
| | Final coating agent (EC),g | 35.3 | 1000+682+82=1764, 2% used |
| | Spherical pellet production amount,g | 1785 | 1000+682+82+35.3=1799.3g |
| Marker component analysis | Extract solid content,% | 60.6 | (400+682)/1785 x 100=60.0% |
| | Marker component content,mg/g | 23.113 | Hesperidin |
| Ginseng radix | | | |
| Seed process | Seeds, g | 1000 | Extract solids (45.1%)=451g |
| Fluidized bed processing | Extract solids of concentrate for fluidized bed,g | 850 | Extract solids37.1% x 2290 g used |
| | Excipients added to spray solution,g | 85 | Extract solids (850) x 10% |
| | final coating agent (Shellac),g | 38.7 | 1000+850+85=1935, 2% used |
| | Spherical pellet production amount,g | 1960 | 1000+850+85+38.7=1973.7g |
| Marker component analysis | Extract solid content,% | 66.4 | (451+850)/1960 x 100=66.4% |
| | Marker component content,mg/g | 2.837 | ginsenoside Rb1+Rg1 |
| Glycyrrhizae radix | | | |
| Seed process | Seeds, g | 1000 | Extract solids (50.1%)=501g |
| Fluidized bed processing | Extract solids of concentrate for fluidized bed,g | 1038 | Extract solids34.6% x 3000 g used |
| | Excipients added to spray solution,g | 103.8 | Extract solids (1038) x 10% |
| | Final coating agent (EC),g | 42.8 | 1000+1038+103.8=2141.8, 2% used |
| | Spherical pellet production amount,g | 2180 | 1000+1038+103.8+42.8=2184.6g |
| Marker component analysis | Extract solid content,% | 70.6 | (501+1038)/2180 x 100=70.6% |
| | Marker component content,mg/g | 18.709 | Glycyrrhizic acid |

(continued)

| Seed process | Seeds, g | 1000 | Extract solids (50.1%)=501g |
|---|---|---|---|
| Scutellariae radix | | | |
| Seed process | Seeds, g | 1000 | Extract solids (55.0%)=550 g |
| Fluidized bed processing | Extract solids of concentrate for fluidized bed,g | 1474 | Extract solids26.8% x 5500 g used |
| | Excipients added to spray solution,g | 147.4 | Extract solids1474 x 10%=147.4 |
| | final coating agent (Shellac),g | 52.4 | 1000+1474+147.4=2621.4, 2% used |
| | Spherical pellet production amount,g | 2670 | 1000+1474+147.4+52.4=2673.8 |
| Marker component analysis | Extract solid content,% | 75.8 | (550+1474)/2670 x 100=75.8% |
| | Marker component content,mg/g | 65.001 | Baicalin+baicalein+wogonin |
| *EC=Ethylcellulose | | | |

## 5.2. Medium-sugar-content single herbal medicines

The seeds containing extracts of Atractylodis Rhizoma, Coptidis Rhizoma, Zingiberis Rhizoma Recens, Paeoniae Radix, and Ephedrae Herba, as medium-sugar-content single herbal medicines, prepared above, were introduced at 1000-1300 g for each case into a fluidized bed granulator. A homogenized preparation solution was prepared by adding a extract concentrate for a fluidized bed required and additives required according to the level of extract solids contained in the finally completed spherical pellets. The levels and percentages of excipients added in the preparation of a preparation solution for fluidized bed spraying were in the range of 5-10% for a medium-sugar-content herbal medicine as shown in Tables 31 and 33. The excipients were used in 10% for Atractylodis Rhizoma, 6% for Coptidis Rhizoma, 8% for Zingiberis Rhizoma Recens, 7% for Paeoniae Radix, and 5% for Ephedrae Herba. The single herbal medicines used were analyzed wherein the content of atractylodin in Atractylodis Rhizoma was 0.15%, the content of berberine in Coptidis Rhizoma was 4.2%, the content of 6-gingerol in Zingiberis Rhizoma Recens was 0.4%, the content of albiflorin and paeoniflorin in Paeoniae Radix was 2.5%, and the content of ephedrine in Ephedrae Herba was 1.51%. The contents of marker components contained in the completed spherical pellets are shown in Table 33.

Atractylodis Rhizoma was used by adding 242 g of the essential oil-encapsulated slurry prepared above to the preparation solution, and the other herbal medicines were measured for the moisture content. The essential oil used was set to be 100% encapsulated and the content of essential oil contained in the slurry added to the preparation solution for a fluidized bed was calculated and specifically indicated in the manufacturing details of Atractylodis Rhizoma pellets in Table 33.

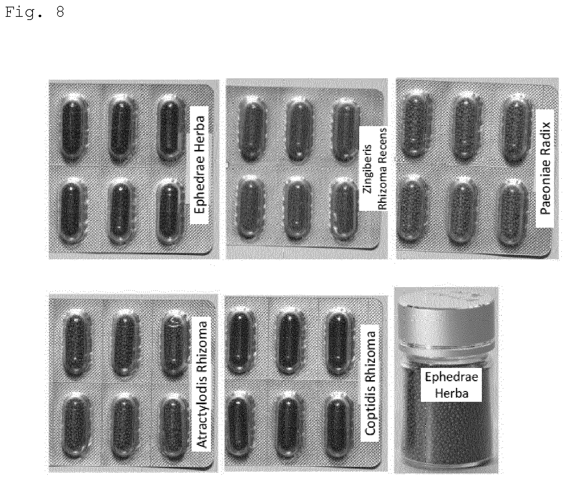

The contents of extract solids and the contents of marker components, which were contained in the final spherical pellets completed according to the operating conditions of the fluidized bed granulator in Table 30, are shown by single herbal medicines in Table 33. FIG. 8 shows that the transparent hard capsules were filled with the completed shapes for each case.

[Table 33]

| Manufacturing results of spherical pellets of five kinds of medium-sugar-content single herbal medicines | | | |
|---|---|---|---|
| Atractylodis rhizoma | | | |
| Seed process | Seeds, g | 1300 | Extract solids(45.1%)=586.3g |
| Fluidized bed processing | Extract solids of concentrate for fluidized bed,g | 1058.4 | Extract solids 37.8% x 2800g used |
| | *essential oil capsule dry product, g | 197.2 | *242 x dry product 81.5$=197.2g (*essential oil content: 197.2 x 52.2=102.9g) |
| | Excipients added to spray solution, g | 105.8 | Extract solids 1058.4g x 10% |
| | Final coating agent, g | 49.3 | 1300+1058.4+105.8=2464.2g, 2% used |
| | Spherical pellet production amount, g | 2695 | 1300+1058.4+105.8+197.2+49.3= 2710.7g |
| Marker component analysis | Extract solid content, % | 64.8 | (586.3+1058.4+102.9)/2695 x 100=64.8 |
| | Marker component content, mg/g | 1,590 | Atractylodin |
| Coptidis rhizoma | | | |
| Seed process | Seeds, g | 1000 | Extract solids(50.0%)=500 g |
| Fluidized bed processing | Extract solids of concentrate for fluidized bed,g | 942 | Extract solids 31.4% x 3000 g used |
| | Excipients added to spray solution,g | 56.5 | Extract solids 942 x 6%=56.5 |
| | Final coating agent,g | 40 | 1000+942+56.5=1998.5g, 2% used |
| | Spherical pellet production amount,g | 2035 | 1000+942+56.5+40=2038.5 |
| Marker component analysis | Extract solid content,% | 70.9 | (500+942)/2.03 x 100=70.9% |
| | Marker component content,mg/g | 67.09 | Berberine |
| Zingiberis rhizoma recens | | | |
| Seed process | Seeds, g | 1000 | Extract solids(55.0)=550 g |
| Fluidized bed processing | Extract solids of concentrate for fluidized bed,g | 1207.5 | Extract solids48.3% x 2500 g used |
| | Excipients added to spray solution,g | 96.6 | Extract solids207.5 x 8%=96.6g |
| | Final coating agent,g | 46.1 | 1000+1207.5+96.6=2304.1g, 2% used |
| | Spherical pellet production amount,g | 2345 | 1000+1207.5+96.6+46.1=2350.2g |
| Marker component analysis | Extract solid content,% | 74.9 | (550+1207.5)/2345 x 100=74.9 |
| | Marker component content,mg/g | 4.228 | 6-gingerol |

(continued)

| Paeoniae radix | | | |
|---|---|---|---|
| Seed process | Seeds, g | 1000 | Extract solids(60.0%)=600 g |
| Fluidized bed processing | Extract solids of concentrate for fluidized bed,g | 1887.3 | Extract solids 46.6% x 4050 g used |
| | Excipients added to spray solution,g | 132 | Extract solids 1887 x 7%=132g |
| | Final coating agent,g | 60.4 | 1000+1887.3+132=3019g, 2% used |
| | Spherical pellet production amount,g | 3070 | 1000+1887+132+60=3079g |
| Marker component analysis | Extract solid content,% | 81.0 | (600+1887)/3070 x 100=81.0% |
| | Marker component content,mg/g | 30.298 | Albiflorin + Paeoniflorin |
| Ephedrae herba | | | |
| Seed process | Seeds, g | 1000 | Extract solids (65%)=650 g |
| Fluidized bed processing | Extract solids of concentrate for fluidized bed,g | 2788.5 | Extract solids 50.7% x 5500 g used |
| | Excipients added to spray solution,g | 139.4 | Extract solids2788.5 x 5%=139.4g |
| | Final coating agent,g | 78.6 | 1000+2788.5+139.4=3927.9g, 2% used |
| | Spherical pellet production amount,g | 4000 | 1000+2788.5+139.4+78.6=4006.5 g |
| Marker component analysis | Extract solid content,% | 86.0 | (650+2788.5)/4000 x 100=86.0 |
| | Marker component content,mg/g | 28.116 | Ephedrin + Pseudoephedrin |

### 5.3. Low-sugar-content single herbal medicines

The seeds containing extracts of Pinelliae Rhizoma, Cinnamomi Cortex Spissus, Aurantii Immaturus Fructus, Bupleuri Radix, and Magnoliae Cortex, as low-sugar-content single herbal medicines, prepared above, were introduced at 1000-1300 g for each case into a fluidized bed granulator. A homogenized preparation solution was prepared by adding a extract concentrate for a fluidized bed required and additives required according to the level of extract solids contained in the finally completed spherical pellets. The levels and percentages of excipients added in the preparation of a preparation solution for fluidized bed spraying were in the range of 3-8% for a low-sugar-content herbal medicine as shown in Tables 31 and 34. The excipients were used in 4% for Pinelliae Rhizoma, 5% for Cinnamomi Cortex Spissus, 6% for Aurantii Immaturus Fructus, 8% for Bupleuri Radix, and 3% for Magnoliae Cortex. The final coating agent shellac was used in a content of 2% equally for all the cases. The single herbal medicines used were analyzed wherein the content of adenine in Pinelliae Rhizoma was 0.0006%, the content of cinnamic acid in Cinnamomi Cortex Spissus was 0.04%, the content of poncirin and naringin in Aurantii Immaturus Fructus was 3.2%, the content of saikosaponin in Bupleuri Radix was 0.4%, and the content of magnolol and honokiol in Magnoliae Cortex was 1.5%. The contents of marker components contained in the completed spherical pellets are shown in Table 34.

Cinnamomi Cortex Spissus was used by adding 320 g of the essential oil-encapsulated slurry prepared above to the preparation solution, and the other herbal medicines were measured for the moisture content. The essential oil used was set to be 100% encapsulated and the content of essential oil contained in the slurry added to the preparation solution for a fluidized bed was calculated and specifically indicated in the manufacturing details of Cinnamomi Cortex Spissus pellets in Table 34.

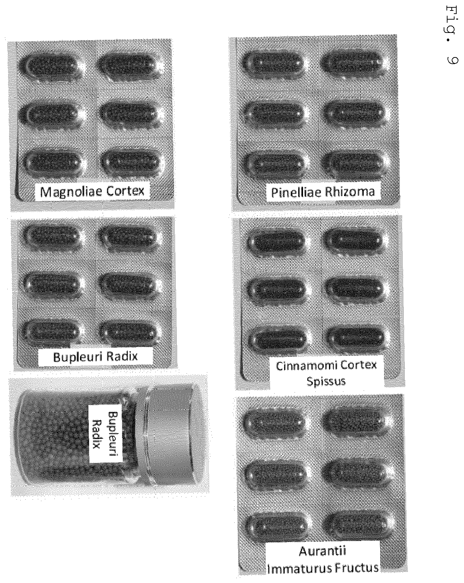

The contents of extract solids and the contents of marker components, which were contained in the final spherical pellets completed according to the operating conditions of the fluidized bed granulator in Table 30, are shown by single herbal medicines in Table 34. FIG. 9 shows that the transparent hard capsules were filled with the completed shapes for each case.

[Table 34]

| Manufacturing results of spherical pellets of five kinds of low-sugar-content single herbal medicines | | | |
|---|---|---|---|
| Pinelliae rhizoma | | | |
| Seed process | Seeds, g | 1000 | Extract solids(50.0%)=500 g |
| Fluidized bed processing | Extract solids of concentrate for fluidized bed,g | 900.3 | Extract solids 19.7% x 4570 g used |
| | Excipients added to spray solution,g | 36 | Extract solids 900.0 g x 4%=36g |
| | Final coating agent,g | 38.7 | 1000+900.3+36=1936.3g, 2% used |
| | Spherical pellet production amount,g | 1970 | 1000+900.3+36+38.7=1975 g |
| Marker component analysis | Extract solid content,% | 71.1 | (500+900.3)/1970 x 100=71.1% |
| | Marker component content,mg/g | 0.259 | Adenine |
| Cinnamomi cortex spissus | | | |
| Seed process | Seeds, g | 1250 | Extract solids(54.9%)=686.3g |
| Fluidized bed processing | Extract solids of concentrate for fluidized bed,g | 1662 | Extract solids 27.7% x 6000g used |
| | *essential oil capsule dry product, g | 256 | *320g x dry product 80%=256g (essential oil content: 256g x56.6%=144.9g) |
| | Excipients added to spray solution,g | 83.1 | Extract solids 1662 x 5%=83.1g |
| | Final coating agent,g | 60 | 1250+1662+83.1=2995.1g, 2% used |
| | Spherical pellet production amount,g | 3285 | 1250+1662+83.1+256+60= 3311.1g |
| Marker component analysis | Extract solid content,% | 75.9 | (686.3+1662+144.9)/ 3285 x 100=75.9% |
| | Marker component content,mg/g | 1.833 | Cinnamic acid |
| Aurantii immaturus fructus | | | |
| Seed process | Seeds, g | 1000 | Extract solids(60.0%)=600 g |
| Fluidized bed processing | Extract solids of concentrate for fluidized bed,g | 1830 | Extract solids 30.5% x 6000 g used |
| | Excipients added to spray solution,g | 109.8 | Extract solids1830 g x 6%=109.8g |
| | Final coating agent,g | 58.8 | 1000+1830+109.8=2939.8g, 2% used |
| | Spherical pellet production amount,g | 2995 | 1000+1830+109.8+58.8=2998.6g |
| Marker component analysis | Extract solid content,% | 81.1 | (600+1830)/2995 x100=81.1% |
| | Marker component content,mg/g | 118.68 | Poncirin + Naringin |

(continued)

| Bupleuri radix | | | |
|---|---|---|---|
| Seed process | Seeds, g | 1000 | Extract solids(65.0)=650 g |
| Fluidized bed processing | Extract solids of concentrate for fluidized bed,g | 2737 | Extract solids 39.1% x 7000 g used |
| | Excipients added to spray solution,g | 219 | Extract solids 2737g x 8%=219g |
| | Final coating agent,g | 79.1 | 1000+2737+219=3956, 2% used |
| | Spherical pellet production amount,g | 4030 | 1000+2737+219+79.1=4035.1g |
| Marker component analysis | Extract solid content,% | 84.0 | (650+2737)/4030 x 100=84.0% |
| | Marker component content,mg/g | 10.332 | Saikosaponin a |
| Magnoliae cortex | | | |
| Seed process | Seeds, g | 1000 | Extract solids(69.9%)=699 |
| Fluidized bed processing | Extract solids of concentrate for fluidized bed,g | 4756 | Extract solids 32.8% x 14,500 g used |
| | Excipients added to spray solution,g | 142.7 | Extract solids 4756g x 3%=142.7 |
| | Final coating agent,g | 118.0 | 1000+4756+142.7=5898.7g, 2% used |
| | Spherical pellet production amount,g | 6010 | 1000+4756+142.7+118=6016.7g |
| Marker component analysis | Extract solid content,% | 90.8 | (699+4756)/6010 x 100=90.8 |
| | Marker component content,mg/g | 109.81 | Magnolol+Honokiol |

### 5.4. Complex herbal decoction

The final spherical pellets of complex herbal decoctions were also manufactured under the same conditions as in the single herbal medicines. The above-prepared seeds containing extracts of *Gumiganghwal-tang* as a high-sugar-content complex herbal medicine, *Mahwang-tang* as a medium-sugar-content complex herbal medicine, and

*Doinseunggi-tang* as a low-sugar-content complex herbal medicine were coated with respective extract solid concentrates in a fluidized bed apparatus to increase the content of extract solids.

The seeds were introduced at 1000-1300 g for each case into the fluidized bed granulator. A homogenized preparation solution was prepared by adding the extract concentrate for a fluidized bed required and additives required according to the level of extract solids contained in the finally completed spherical pellets. The levels and percentages of excipients added in the manufacture of a preparation solution for fluidized bed spraying are as shown in Tables 29 and 31 and the actual use amounts are as shown in Table 35. The excipients are in the range of 10-15% for a high-sugar-content complex herbal medicine, and were used in 12% in *Gumiganghwal-tang* concentrate prepared for a fluidized bed; the excipients are in the range of 5-10% for a medium-sugar-content complex herbal medicine, and were used in 8% in the prepared *Mahwang-tang* concentrate; and the excipients are in the range of 3-8% for a low-sugar-content complex herbal medicine, and were used in 5% in the prepared *Doinseunggi-tang* concentrate.

*Gumiganghwal-tang* was used by adding 125 g of the essential oil-encapsulated slurry prepared above to the preparation solution, and the other herbal medicines were measured for the moisture content. The essential oil used was set to be 100% encapsulated and the content of essential oil contained in the slurry added to the preparation solution for a fluidized bed was calculated and specifically indicated in the manufacturing details of pellets in Table 35.

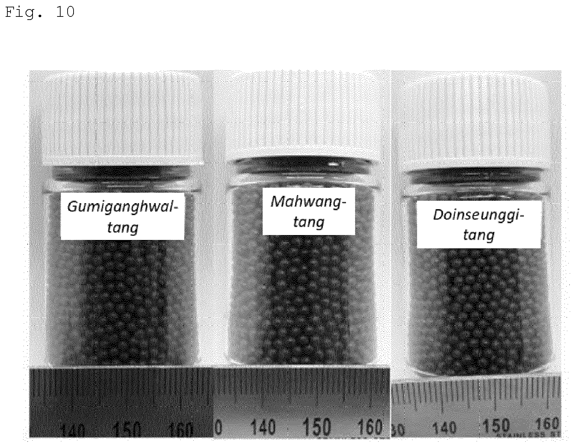

The contents of extract solids and the contents of marker components, which were contained in the final spherical pellets completed according to the operating conditions of the fluidized bed apparatus in Table 30, are shown by single herbal medicines in Table 35. FIG. 10 shows that the transparent hard capsules were filled with the completed shapes for each case.

[Table 35]

| Manufacturing results of spherical pellets of three kinds of complex herbal decoctions | | | |
|---|---|---|---|
| *Gumiganghwal-tang* | | | |
| Seed process | Seeds, g | 1300 | Extract solids(54.9%)=713.7g |
| Fluidiz ed bed process ing | Extract solids of concentrate for fluidized bed,g | 2085 | Extract solids 27.8% x 7500 g used |
| | *essential oil capsule dry product, g | 86.9 | *125g x dry product 69.5%=86.9g (essential oil content: 86.9g x 20.5%=17.8g) |
| | Excipients added to spray solution,g | 250.2 | Extract solids 2085 g x 12%=250.2 |
| | Final coating agent,g | 72.7 | 1300+2085+250.2=3635.2g, 2% used |
| | Spherical pellet production amount,g | 3765 | 1300+2085+250.2+86.9+72.7=3794.8g |
| Extract solid content, % | | 74.8 | (713.7+2085+17.8)/3765 x 100=74.8 |
| *Mahwang-tang* | | | |
| Seed process | Seeds, g | 1000 | Extract solids(64.9%)=649 |
| Fluidiz ed bed process ing | Extract solids of concentrate for fluidized bed,g | 3144.5 | Extract solids 33.1% x 9,500 g used |
| | Excipients added to spray solution,g | 251.6 | Extract solids 3144.5 g x 8%=251.6 |
| | Final coating agent,g | 87.9 | 1000+3144.5+251.6=4396.1g, 2% used |
| | Spherical pellet production amount,g | 4480 | 1000+3144.5+251.6+87.9=4484g |
| Marker compone nt analysis | Extract solid content,% | 84.7 | (649+3144.5)/4480 x 100=84.7% |
| | Marker component content,mg/g | 14.254 | Total Ephedrin |
| *Doinseunggi-tang* | | | |
| Seed process | Seeds, g | 1000 | Extract solids(70.0%)=700 |
| Fluidiz ed bed process ing | Extract solids of concentrate for fluidized bed,g | 5180 | Extract solids 25.9% x 20,000 g used |
| | Excipients added to spray solution,g | 259 | Extract solids 5180 g x 5%=259 |
| | Final coating agent,g | 128.8 | 1000+5180+259=6439g, 2% used |
| | Spherical pellet production amount,g | 6550 | 1000+5180+259+128.8=6567.8g |
| Extract solid content,% | | 89.8 | (700+5180)/6550 x 100=89.8 |

**Example 6: Prescription and modified prescription using completed single herbal medicines**

The prescriptions of *Pyeongwisan, Banhasasim-tang, Sosiho-tang, Sihogyeji-tang, Hubak-tang,* and *Ijung-tang* were easily configured using 15 kinds of single herbal medicines manufactured above. The contents of various marker components contained in mixed herbal medicines using the single herbal medicines of the present application can be monitored. Tables 36 to 41 exemplified the results of mixed herbal medicines configured using the single herbal medicines manufactured above. In Tables 36 to 41, the "dry extract" refers to extract solids, recited in the standard prescription in Oriental Medical Health Insurance of the Korean Pharmacopoeia, and means an excipient-free dry extract. Therefore, for convenience of comparison, a single dose was also indicated on the basis of 50% the addition amount of conventional common excipients.

[Table 36]

| Prescription using single herbal medicines (*Pyeongwisan*) | | | | | | | | |
|---|---|---|---|---|---|---|---|---|
| | Atractylo dis Rhizoma | Fraxini Cortex | Magnolia e Cortex | Glycyrrhi zae Radix | Zingi beris Rhizo ma Recen s | Jujub ae Fruct us | Single dose | * |
| Dried extract, g | 1.800 | 1.050 | 0.188 | 0.380 | 0.053 | 0.728 | 4.199 | 8.398 |
| Extract content of medicine, % | 64.7 | 60.6 | 90.8 | 70.6 | 74.9 | 56.4 | | |
| Equivalent weight of medicine, g | 2.782 | 1.733 | 0.207 | 0.538 | 0.071 | 1.291 | 6.622 | |
| Marker component name | Atractylo din | Hesperid in | Magnolol | Glycyrrhi zic acid | Ginge rol | Total sugar | | |
| Marker component content, mg/g medicine weight | 4.663 | 40.055 | 22.731 | 10.065 | 0.300 | 488.9 0 | | |
| * Based on an average of 50% contained in excipients added, single dose | | | | | | | | |

[Table 37]

| Prescription using single herbal medicines (*Banhasasim-tang*) | | | | | | | | |
|---|---|---|---|---|---|---|---|---|
| | Pinel liae Rhizo ma | Scute llari ae Radix | Ginse ng Radix | Glycy rrhiz ae Radix | Zingi beris Rhizoma Recens | Copti dis Rhizo ma | Jujub ae Fruct us | Singl e dose | * |
| Dried extract, g | 1.148 | 1.575 | 0.844 | 0.951 | 0.525 | 0.210 | 0.259 | 5.512 | 11.02 4 |
| Extract content of medicine, % | 71.1 | 75.8 | 66.4 | 70.6 | 74.9 | 70.9 | 56.4 | | |
| Medicine weight, g | 1.615 | 2.078 | 1.271 | 1.347 | 0.701 | 0.296 | 0.459 | 7.767 | |
| Marker component name | Adeni ne | Baica lin etc. | Ginse noside | Glycy rrhiz ic acid | Ginge rol | Berbe rine | Total sugar | | |
| Marker component content, mg/g medicine weight | 0.418 | 135.0 7 | 3.606 | 25.20 1 | 2.964 | 19.85 9 | 173.8 2 | | |

[Table 38]

| Prescription using single herbal medicines (Sosiho-tang) | | | | | | | | | |
|---|---|---|---|---|---|---|---|---|---|
| | Bupleuri Radix | Scutellariae Radix | Ginseng Radix | Pinelliae Rhizoma | Glycyrrhizae Radix | Zingiberis Rhizoma Recens | Jujubae Fructus | single dose | * |
| dried extract, g | 1.159 | 2.100 | 0.563 | 0.574 | 0.317 | 0.053 | 0.728 | 5.494 | 10.988 |
| extract content of medicine, % | 84.0 | 75.8 | 66.4 | 71.1 | 70.6 | 74.9 | 56.4 | | |
| Medicine weight, g | 1.380 | 2.770 | 0.848 | 0.807 | 0.449 | 0.071 | 1.291 | 7.616 | |
| marker component name | saiko saponin | Baicalin, etc. | ginsenoside | adenine | glycyrrhizic acid | Gingerol | Total sugar | | |
| marker component content, mg/g medicine weight | 14.258 | 180.053 | 2.406 | 0.209 | 8.400 | 0.300 | 488.90 | | |

[Table 39]

| Prescription using single herbal medicines (Sihogyeji-tang) | | | | | | | | | | |
|---|---|---|---|---|---|---|---|---|---|---|
| | Bupleuri Radix | Cinnamomi Cortex Spissus | Scutellariae Radix | Ginseng Radix | Paeoniae Radix | Pinelliae Rhizoma | Glycyrrhizae Radix | Zingiberis Rhizoma Recens | Jujubae Fructus | single dose | * |
| dried extract, g | 0.773 | 0.075 | 1.050 | 0.563 | 0.638 | 0.574 | 0.319 | 0.053 | 0.728 | 4.771 | 9.542 |
| extract content of medicine, % | 84.0 | 76.1 | 75.8 | 66.4 | 81.0 | 71.1 | 70.6 | 74.9 | 56.4 | | |
| Weight of medicine, g | 0.920 | 0.099 | 1.385 | 0.848 | 0.788 | 0.807 | 0.452 | 0.071 | 1.291 | 6.661 | |
| marker component name | saikosaponin | cinnamic acid | Baicalin, etc. | ginsenoside | Albiflorin, etc. | adenine | glycyrrhizic acid | gingerol | Total sugar | | |
| marker component content, mg/g medicine weight | 9.505 | 0.002 | 90.026 | 2.406 | 23.875 | 0.209 | 8.456 | 0.300 | 488.90 | | |

**EP 3 782 609 B1**

[Table 40]

| Prescription using single herbal medicines (*Hubak-tang*) | | | | | | | | | | |
|---|---|---|---|---|---|---|---|---|---|---|
| | Magno liae Corte x | Atrac tylod is Rhizo ma | Fraxi ni Corte x | Glycy rrhiz ae Radix | Pinel liae Rhizo ma | Auran tii Immat urus Fruct us | Zingi beris Rhizo ma Recen s | Jujub ae Fruct us | singl e dose | * |
| dried extract, g | 0.225 | 1.800 | 0.750 | 0.635 | 0.287 | 0.650 | 0.053 | 0.728 | 5.128 | 10.25 6 |
| extract content of medicine, % | 90.8 | 64.9 | 60.6 | 70.6 | 71.1 | 81.1 | 74.9 | 56.4 | | |
| Medicine weight, g | 0.248 | 2.773 | 1.238 | 0.899 | 0.404 | 0.801 | 0.071 | 1.291 | 7.725 | |
| marker component name | Magno lol, etc. | atrac tylod in | hespe ridin | glycy rrhiz ic acid | adeni ne | Ponci rin, etc. | ginge rol | Total sugar | | |
| marker component content, mg/g medicine weight | 27.23 3 | 4.409 | 28.61 4 | 16.81 9 | 0.105 | 95.06 3 | 0.300 | 488.9 0 | | |

[Table 41]

| Prescription using single herbal medicines (*Ijung-tang*) | | | | | | |
|---|---|---|---|---|---|---|
| | Ginseng Radix | Atractylo dis Rhizoma | Zingiberi s Rhizoma Recens | Glycyrrhi zae Radix | Single dose | * |
| Dried extract, g | 1.125 | 1.725 | 1.050 | 0.634 | 4.534 | 9.06 8 |
| Extract content of medicine, % | 66.4 | 64.9 | 74.9 | 70.6 | | |
| Medicine weight , g | 1.694 | 2.658 | 1.402 | 0.898 | 6.652 | |
| Marker component name | Ginsenosi de | Atractylo din | Gingerol | Glycyrrhi zic acid | | |
| Marker component content, mg/g medicine weight | 4.806 | 4.226 | 5.928 | 16.801 | | |

Referring to the evaluation results in Tables 36 to 41, as for existing single herbal extract powders, on the basis of 50% the addition amount of excipients, the intake weight of the inventive single herbal medicines for a single dose was showed be less 26.82% in *Pyeongwisan,* 41.93% less in *Banhasasim-tang,* 44.28% less in *Sosiho-tang,* 43.25% less in *Sihogyeji-tang,* 32.76% less *in Hubak-tang,* and 36.23% less in *Ijung-tang.*

In a modified prescription, the weights of single herbal medicines, which are important in a single herbal mix medicine, were increased by 2 times. For example, the weights of Atractylodis Rhizoma, Fraxini Cortex, and Magnoliae Cortex were increased for *Pyeongwisan;* the weights of Pinelliae Rhizoma and Scutellariae Radix were increased for *Banhasasim-tang;* the weights of Bupleuri Radix and Scutellariae Radix were increased for *Sosiho-tang;* the weights of Bupleuri Radix, Pinelliae Rhizoma, and Ginseng Radix were increased for *Sihogyeji-tang;* and the weights of Magnoliae Corte and Aurantii Immaturus Fructus were increased for *Hubak-tang.*

The "modified prescription" or *"gamibang"* refers to a different preparation for which "a corresponding marker component is enriched" or the content of at least one marker component recognized as having a weight of a supply source is increased.

In the single herbal medicines manufactured in the present application, the contents of marker components contained are indicated, and thus a doctor can quickly respond to the degrees of disease symptoms of a patient. In spite of an advantage in utilization as a single herbal formulation, even a generally favorably used medicine has been reported to have various side effects. That is, it has been reported that Furano diterpenoid contained in Scutellariae Radix, which is a single herbal medicine included in *Hwangnyeonhaedok-tang* and *Bangpungtongseongsan* causes liver damage due to glutathione depletion; exposure to cinnamon bark and Cinnamomi Cortex Spissus in high concentrations for a long period causes liver toxicity; saikosaponin a, which is a marker component contained in Bupleuri Radix, inhibits lipid metabolism; a component of Glycyrrhizae Radix causes hypokalemia; aconitin of Pulvis Aconiti Tuberis Purificatum causes ventricular arrhythmia; and ephedrine, which is a marker component in Ephedrae Herba, causes arrhythmia and myocardial infarction (J. Pharm. Soc. Korea 1998,42(4): 422-430, J. Korean Med. 2017, 38(3): 170-184, J. Korean Neurol. Assoc. 2009, 27(4): 424-427) .

Therefore, the single herbal medicine of the present application has a great advantage in that the content of extract solids administered to humans is adjusted easily and accurately, and in the event of side effects, the accurate understanding of causes thereof and the derivation of remedies are easily attained.

**Example 7: Prescription of adding single herbal medicine to complex herbal medicine and use thereof**

**7.1. Examples regarding four-stage prescription for adjusting total content of ephedrine of complex herbal medicine *mahwhang-tang***

Ephedrae Herba is the most frequently used medicinal herb by a prescription of a complex herbal medicine or a single herbal medicine relevant to obesity treatment in Korea. Ephedrine, which is a marker component of Ephedrae Herba, works as a sympatheticomimetic agent acting on adrenoreceptors and exerts effects of suppressing appetite and promoting heat metabolism, leading to weight loss, and thus Ephedrae Herba is frequently used in prescriptions for diet. However, Ephedrae Herba needs special attention for side effects, such as insomnia, anxiety, headache, and palpitations, and cardiovascular side effects, such as myocardial infarction and arrhythmia. *Mahwang-tang* is a toxic herbal medicine containing an alkaloid component, wherein the content of the marker component ephedrine needs to be precisely controlled to be appropriate for the situation of a patient.

The daily dose of ephedrine as an obesity medicine is reported in the range of 20-70 mg/day, but a lot of attention is required for more than 60 mg. In the present disclosure, a basic *mahwhang-tang* medicine containing a low content of ephedrine is manufactured as a complex herbal medicine and a single herbal medicine containing only Ephedrae Herba is manufactured (Table 33), so that four types of *gamibang* prescriptions having different ephedrine contents by stages of obesity treatment can be easily configured.

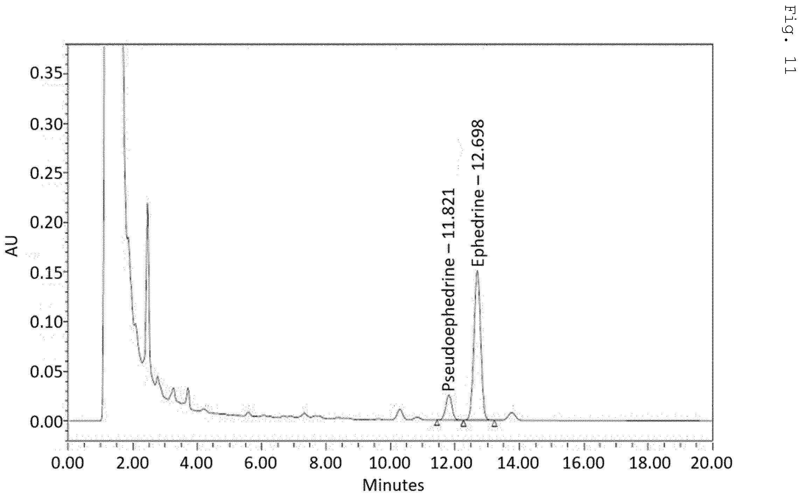

A total of ephedrine contained in the spherical pellets manufactured in Table 35 above was analyzed. HPLC (Alliance e2695, Waters, USA), Phenomenex Kinetex C18 (4.6x150 mm, 5 um) column, and Empower 3 (Waters, USA) software were used. FIG. 11 shows a chromatogram of the analysis of ephedrine of the complex herbal medicine *mahwhang-tang* (Table 35).

The total ephedrine content of the dried Ephedrae Herba used was measured to be 15.05 mg/g, and the total content of ephedrine included in the two kinds of medicines was 28.116 mg/g for the single herbal medicine (Table 33) and 14.254 mg/g for the *mahwhang* complex herbal medicine.

[Table 42]

| Intake of Ephedrae Herba-containing medicines according to total amount of ephedrine provided | | | | | |
|---|---|---|---|---|---|
| Classification | By stages | | | | |
| | Stage 1 | Stage 2 | Stage 3 | Stage 4 | |
| Doses by stages, total ephedrin mg/day | 20 | 30 | 40 | 50 | |
| *Mahwang-tang,* complex herbal medicine | Dose of complex herbal medicine, g/day | 1.403 | 1.403 | 1.403 | 1.403 |
| | Total serving amount of ephedrin, mg/day | 20 | 20 | 20 | 20 |
| Ephedrae Herba, single herbal medicine, mg/g | addition amount of single herbal medicine, g/day | - | 0.356 | 0.711 | 1.067 |
| | Total serving amount of ephedrin, mg/day | - | 10 | 20 | 30 |

(continued)

| Intake of Ephedrae Herba-containing medicines according to total amount of ephedrine provided | | | | |
|---|---|---|---|---|
| Classification | By stages | | | |
| | Stage 1 | Stage 2 | Stage 3 | Stage 4 |
| Total doses of medicine by stages, g/day | | 1.403 | 1.759 | 2.114 | 2.470 |
| Total serving amounts of ephedrin by stages, mg/day | | 20 | 30 | 40 | 50 |

As shown in the results of Table 42, the total intake of the medicines can be minimized by stages. In order for only the basically manufactured *mahwhang-tang* as a complex herbal medicine to satisfy the total amount of ephedrine on stage 4, the complex herbal medicine was required at 3.508 g/day, and at 2.470 g/day when Ephedrae Herba as a single herbal medicine was added. Therefore, in terms of the Ephedrae Herba single herbal medicine, the amount of the medicine administered can be reduced by 42.02%, leading to the reduction in discomfort for a patient who should ingest the single herbal medicine in large amounts. Besides, Saida Ibragic et.al (Utilisation of a sample and fast HPLC-UV method for separation and quantification of Ephedrine alkaloids in herb of different Ephedra Species. Research & Reviews: Journal of Pharmacology and Toxicological Studies 2017, 5(2) : 7-10) reported that the total ephedrine content of Ephedrae Herba was 0.4-71.9mg/g, which is in the range of dry weight, indicating a wide content range. Therefore, in cases where the total ephedrine content of a raw material used is low, the intake increases proportionally when only the *Mahwhang-tang* complex herbal medicine is used.

Therefore, as for Ephedrae Herba, Pulvis Aconiti Tuberis Purificatum, Aconiti Tuber, Aconiti Ciliare Tuber, Euphorbiae Kansui Radix, Euphorbiae Pekinensis Radix, Daphnis Genkwa Flos, Euphorbiae Fischerianae Radix, Arisaematis Rhizoma, which correspond to the toxic single herbal medicines requiring extreme attention to the amount of administration, a prescription of corresponding single and complex herbal medicines as described above can safely adjust the amount of a toxic marker component.

**7.2. Combinative use of Gumiganghwal-tang complex herbal medicine and single herbal medicine**

*Gumiganghwal -tang* is one of the most important prescriptions in oriental clinical practice, accounting for 6.1% of the total number of prescription days and 7.0% of the total pharmaceutical cost among 56 kinds of herbal medicines in Oriental Medical Health Insurance. Various research reports confirmed that *Gumiganghwal-tang* had anti-inflammatory effects, antipyretic effects, and pain-killing effects.

The constituent raw materials of *Gumiganghwal-tang* were Notopterygii Rhizoma 5.625 g, Saposhnikovia Radix 5.625 g, Ligustici Rhizoma 4.5 g, Thujae Resina 4.5 g, Atractylodis Rhizoma 4.5 g, Scutellariae Radix 4.5 g, Rehmanniae Radix 4.5 g, Asari Herba Cum Radix 1.875 g, Glycyrrhizae Radix 1.875 g, and the weight for a single dose of medicinal herbs was 37.5 g. The use will be described in detail with reference to the above example. A patient who comes to a hospital receiving single and complex herbal medicines can receive prescription drugs promptly on the spot according to the progress of disease symptoms. That is, as the important effects exerted by marker components of single herbal medicines constituting *Gumiganghwal-tang,* for example, it is known that Atractylodis Rhizoma exerts an effect on leg pain and joint pain, Scutellariae Radix exerts an edema relieving effect, and Glycyrrhizae Radix exerts on effect in the treatment of sore throat pain. Therefore, a doctor can provide the patient with a modified prescription by easily selecting which corresponding single herbal medicines manufactured above.

As another method in the above-described use methods, a formulation system for individually customized herbal medicines by Korean Patent Registration No. 10-1681235 can be utilized. That is, a formulation system composed of software with an information program on information, efficacy, and the like of the above-described medicines and hardware configured to actually select, weighing, and packaging medicines is used.

As a result, an oriental medical doctor can immediately select and mix a plurality of separate single herbal medicines on the basis of a prescription to be provided to a patient, and thus can immediately provide the same for the patient on the spot. Additionally, the oriental medical doctor can quickly respond to the increase or decrease of drug marker components, involved in the progress of symptoms after the patient visits a hospital. In addition, the manufactured single herbal medicine can be easily added to the prescription *gamibang,* in which the increase of important medicinal herbs is required even in complex herbal medicines that are usually administered. Ultimately, the contents of marker components actually provided to the patient can be accurately monitored.

Accordingly, the present disclosure can easily established considering various factors including the age, weight, overall health conditions, gender, and regimen difference of a patient, personal drug metabolic rates, and severity of specific disorders or conditions. Furthermore, the present disclosure can provide a combination capable of administering one or more single herbal medicines or a prescription at the same time, or administering the single herbal medicines before or after the prescription or vice versa.

**Claims**

1. A method for manufacturing a pellet formulation containing a single or complex herbal extract, the method comprising:

   (a) preparing a concentrate for seeds and a concentrate for fluidized bed coating from a single or complex herbal extract, wherein the concentrate for fluidized bed coating having a lower sugar content than the concentrate for seeds;
   (b) molding a seed by mixing the concentrate for seed and an excipient; and
   (c) coating surfaces of the seeds by mixing the concentrate for fluidized bed coating and an excipient,

   and wherein the excipient is at least one kind selected from the group consisting of precipitated calcium carbonate (precipitated $CaCO_3$), magnesium oxide (MgO), calcium silicate ($CaSiO_3$), magnesium silicate ($3MgSiO_3-5H_2O$), silicon dioxide ($SiO_2$), titanium dioxide ($TiO_2$), bentonite, kaolin, talc, CMC-Ca, casein, dextrin, hydroxypropyl methylcellulose (HPMC), pullulan, microcrystalline cellulose (MCC), lactose, stearic acid-magnesium (stearate-Mg), stearic acid-calcium (stearate-Ca), PEG 6000, beta-cyclodextrin powder, zein powder, starch sodium octenyl succinate (SSOC), and sucrose fatty acid esters.

2. The method of claim 1, wherein when a 10-fold water extract of the single or complex herbal medicine has a sugar content of 5.5 or more and less than 10.0 Brix in step (a), the concentrate for seeds in step (a) is prepared by concentrating the single or complex herbal extract to a sugar content of 45-65 Brix.

3. The method of claim 1, wherein when a 10-fold water extract of the single or complex herbal medicine has a sugar content of 3.0 or more and less than 5.5 Brix in step (a), the concentrate for seeds in step (a) is prepared by concentrating the single or complex herbal extract to a sugar content of 40-60 Brix.

4. The method of claim 1, wherein when a 10-fold water extract of the single or complex herbal medicine has a sugar content of 0.01 or more and less than 3.0 Brix in step (a), the concentrate for seeds in step (a) is prepared by concentrating the single or complex herbal extract to a sugar content of 30-60 Brix.

5. The method of claim 1, wherein when a 10-fold water extract of the single or complex herbal medicine has a sugar content of 5.5 or more and less than 10.0 Brix in step (a), the concentrate for fluidized bed coating in step (a) is prepared by concentrating the single or complex herbal extract to a sugar content of 15-35 Brix.

6. The method of claim 1, wherein when a 10-fold water extract of the single or complex herbal medicine has a sugar content of 3.0 or more and less than 5.5 Brix in step (a), the concentrate for fluidized bed coating in step (a) is prepared by concentrating the single or complex herbal extract to a sugar content of 25-45 Brix.

7. The method of claim 1, wherein when a 10-fold water extract of the single or complex herbal medicine has a sugar content of 0.01 or more and less than 3.0 Brix in step (a), the concentrate for fluidized bed coating in step (a) is prepared by concentrating the single or complex herbal extract to a sugar content of 20-35 Brix.

8. The method of claim 1, wherein step (b) comprises: passing a mixture of the concentrate for seeds and the excipient through an extruder to cut an extrudate; and introducing the cut extrudate into a spheronizer to grind the cut extrudate.

9. The method of claim 1, wherein when a 10-fold water extract of the single or complex herbal medicine has a sugar content of 5.5 or more and less than 10.0 Brix in step (a), step (b) is performed by mixing a concentrate for seeds, which contains 35-55 wt% of single or complex herbal extract solids relative to the total dry weight of the seeds, with the balance of an excipient.

10. The method of claim 1, wherein when a 10-fold water extract of the single or complex herbal medicine has a sugar content of 3.0 or more and less than 5.5 Brix in step (a), step (b) is performed by mixing a concentrate for seeds, which contains 45-65 wt% of single or complex herbal extract solids relative to the total dry weight of the seeds, with the balance of an excipient.

11. The method of claim 1, wherein when a 10-fold water extract of the single or complex herbal medicine has a sugar content of 0.01 or more and less than 3.0 Brix in step (a), step (b) is performed by a concentrate for seeds, which contains 50-70 wt% of single or complex herbal extract solids relative to the total dry weight of the seeds, with the balance of an excipient.

12. The method of claim 1, wherein when the single or complex herbal extract has aromaticity, the method further comprises: obtaining an essential oil volatilized during extraction and concentration; and encapsulating the essential oil.

13. The method of claim 1, wherein when the single or complex herbal extract has aromaticity, a product obtained by encapsulating an essential oil of the extract having aromaticity is further added to and mixed with the concentrate for fluidized bed coating, in step (c).

14. The method of claim 1, further additional comprising coating the surfaces of the seeds after step (c).

15. The method of claim 1, wherein when a 10-fold water extract of the single or complex herbal medicine has a sugar content of 5.5 or more and less than 10.0 Brix in step (a), the pellet formulation contains, relative to the total dry weight thereof, 55-80 wt% of single or complex herbal extract solids.

16. The method of claim 1, wherein when a 10-fold water extract of the single or complex herbal medicine has a sugar content of 3.0 or more and less than 5.5 Brix in step (a), the pellet formulation contains, relative to the total dry weight thereof, 60-90 wt% of single or complex herbal extract solids.

17. The method of claim 1, wherein when a 10-fold water extract of the single or complex herbal medicine has a sugar content of 0.01 or more and less than 3.0 Brix in step (a), the pellet formulation contains, relative to the total dry weight thereof, 70-95 wt% of single or complex herbal extract solids.

**Patentansprüche**

1. Verfahren zum Herstellen einer Pelletformulierung, die einen einzelnen oder komplexen Kräuterextrakt enthält, wobei das Verfahren Folgendes umfasst:

   (a) Anfertigen eines Konzentrats für Samen und eines Konzentrats für Wirbelschicht-Coating aus einem einzelnen oder komplexen Kräuterextrakt, wobei das Konzentrat für Wirbelschicht-Coating einen niedrigeren Zuckergehalt als das Konzentrat für Samen aufweist;
   (b) Formen eines Samens durch Mischen des Konzentrats für Samen und eines Hilfsstoffs; und
   (c) Beschichten von Oberflächen der Samen durch Mischen des Konzentrats für Wirbelschicht-Coating und eines Hilfsstoffs,

   und wobei der Hilfsstoff mindestens eine Art ist, die aus der Gruppe ausgewählt ist, die aus Calciumcarbonat (ausgefälltes $CaCO_3$), Magnesiumoxid (MgO), Calciumsilikat ($CaSiO_3$), Magnesiumsilikat ($3MgSiO_3$ $5H_2O$), Siliziumdioxid ($SiO_2$), Titandioxid ($TiO_2$), Bentonit, Kaolin, Talk, CMC-Ca, Casein, Dextrin, Hydroxypropylmethylcellulose (HPMC), Pullulan, mikrokristalliner Cellulose (MCC), Lactose, Stearinsäure-Magnesium (Stearat-Mg), Stearinsäure-Calcium (Stearat-Ca), PEG 6000, Beta-Cyclodextrin-Pulver, Zeinpulver, Stärkenatriumoctenylsuccinat (SSOC) und Saccharose-Fettsäureestern besteht.

2. Verfahren nach Anspruch 1, wobei, wenn ein 10-facher Wasserextrakt des einzelnen oder komplexen Kräuterarzneimittels in Schritt (a) einen Zuckergehalt von 5,5 oder mehr und weniger als 10,0 Brix aufweist, das Konzentrat für Samen in Schritt (a) durch Konzentrieren des einzelnen oder komplexen Kräuterextrakts auf einen Zuckergehalt von 45-65 Brix angefertigt wird.

3. Verfahren nach Anspruch 1, wobei, wenn ein 10-facher Wasserextrakt des einzelnen oder komplexen Kräuterarzneimittels in Schritt (a) einen Zuckergehalt von 3,0 oder mehr und weniger als 5,5 Brix aufweist, das Konzentrat für Samen in Schritt (a) durch Konzentrieren des einzelnen oder komplexen Kräuterextrakts auf einen Zuckergehalt von 40-60 Brix angefertigt wird.

4. Verfahren nach Anspruch 1, wobei, wenn ein 10-facher Wasserextrakt des einzelnen oder komplexen Kräuterarzneimittels in Schritt (a) einen Zuckergehalt von 0,01 oder mehr und weniger als 3,0 Brix aufweist, das Konzentrat für Samen in Schritt (a) durch Konzentrieren des einzelnen oder komplexen Kräuterextrakts auf einen Zuckergehalt von 30-60 Brix angefertigt wird.

5. Verfahren nach Anspruch 1, wobei, wenn ein 10-facher Wasserextrakt des einzelnen oder komplexen Kräuterarzneimittels in Schritt (a) einen Zuckergehalt von 5,5 oder mehr und weniger als 10,0 Brix aufweist, das Konzentrat für Wirbelschicht-Coating in Schritt (a) durch Konzentrieren des einzelnen oder komplexen Kräuterextrakts auf einen Zuckergehalt von 15-35 Brix angefertigt wird.

6. Verfahren nach Anspruch 1, wobei, wenn ein 10-facher Wasserextrakt des einzelnen oder komplexen Kräuterarzneimittels in Schritt (a) einen Zuckergehalt von 3,0 oder mehr und weniger als 5,5 Brix aufweist, das Konzentrat für Wirbelschicht-Coating in Schritt (a) durch Konzentrieren des einzelnen oder komplexen Kräuterextrakts auf einen Zuckergehalt von 25-45 Brix angefertigt wird.

7. Verfahren nach Anspruch 1, wobei, wenn ein 10-facher Wasserextrakt des einzelnen oder komplexen Kräuterarzneimittels in Schritt (a) einen Zuckergehalt von 0,01 oder mehr und weniger als 3,0 Brix aufweist, das Konzentrat für Wirbelschicht-Coating in Schritt (a) durch Konzentrieren des einzelnen oder komplexen Kräuterextrakts auf einen Zuckergehalt von 20-35 Brix angefertigt wird.

8. Verfahren nach Anspruch 1, wobei Schritt (b) Folgendes umfasst: Leiten eines Gemischs aus dem Konzentrat für Samen und dem Hilfsstoff durch einen Extruder, um ein Extrudat zu schneiden; und Einführen des geschnittenen Extrudats in einen Spheronizer, um das geschnittene Extrudat zu zerkleinern.

9. Verfahren nach Anspruch 1, wobei, wenn ein 10-facher Wasserextrakt des einzelnen oder komplexen Kräuterarzneimittels in Schritt (a) einen Zuckergehalt von 5,5 oder mehr und weniger als 10,0 Brix aufweist, Schritt (b) durch Mischen eines Konzentrats für Samen, das 35-55 Gew.-% von Feststoffen des einzelnen oder komplexen Kräuterextrakts relativ zu dem Gesamttrockengewicht der Samen enthält, mit dem Rest eines Hilfsstoffs durchgeführt wird.

10. Verfahren nach Anspruch 1, wobei, wenn ein 10-facher Wasserextrakt des einzelnen oder komplexen Kräuterarzneimittels in Schritt (a) einen Zuckergehalt von 3,0 oder mehr und weniger als 5,5 Brix aufweist, Schritt (b) durch Mischen eines Konzentrats für Samen, das 45-65 Gew.-% von Feststoffen des einzelnen oder komplexen Kräuterextrakts relativ zu dem Gesamttrockengewicht der Samen enthält, mit dem Rest eines Hilfsstoffs durchgeführt wird.

11. Verfahren nach Anspruch 1, wobei, wenn ein 10-facher Wasserextrakt des einzelnen oder komplexen Kräuterarzneimittels in Schritt (a) einen Zuckergehalt von 0,01 oder mehr und weniger als 3,0 Brix aufweist, Schritt (b) durch ein Konzentrat für Samen, das 50-70 Gew.-% von Feststoffen des einzelnen oder komplexen Kräuterextrakts relativ zu dem Gesamttrockengewicht der Samen enthält, mit dem Rest eines Hilfsstoffs durchgeführt wird.

12. Verfahren nach Anspruch 1, wobei, wenn der einzelne oder komplexe Kräuterextrakt Aromatizität aufweist, das Verfahren ferner Folgendes umfasst: Erhalten eines ätherischen Öls, das während der Extraktion und Konzentrierung verflüchtigt wird; und Einkapseln des ätherischen Öls.

13. Verfahren nach Anspruch 1, wobei, wenn der einzelne oder komplexe Kräuterextrakt Aromatizität aufweist, in Schritt (c) ein Produkt, das durch Einkapseln eines ätherischen Öls des Extrakts, der Aromatizität aufweist, erhalten wird, ferner zu dem Konzentrat für Wirbelschicht-Coating hinzugefügt und damit gemischt wird.

14. Verfahren nach Anspruch 1, ferner zusätzlich umfassend Beschichten der Oberflächen der Samen nach Schritt (c).

15. Verfahren nach Anspruch 1, wobei, wenn ein 10-facher Wasserextrakt des einzelnen oder komplexen Kräuterarzneimittels in Schritt (a) einen Zuckergehalt von 5,5 oder mehr und weniger als 10,0 Brix aufweist, die Pelletformulierung relativ zu dem Gesamttrockengewicht davon 55-80 Gew.-% von Feststoffen des einzelnen oder komplexen Kräuterextrakts enthält.

16. Verfahren nach Anspruch 1, wobei, wenn ein 10-facher Wasserextrakt des einzelnen oder komplexen Kräuterarzneimittels in Schritt (a) einen Zuckergehalt von 3,0 oder mehr und weniger als 5,5 Brix aufweist, die Pelletformulierung relativ zu dem Gesamttrockengewicht davon 60-90 Gew.-% von Feststoffen des einzelnen oder komplexen Kräuterextrakts enthält.

17. Verfahren nach Anspruch 1, wobei, wenn ein 10-facher Wasserextrakt des einzelnen oder komplexen Kräuterarzneimittels in Schritt (a) einen Zuckergehalt von 0,01 oder mehr und weniger als 3,0 Brix aufweist, die Pelletformulierung relativ zu dem Gesamttrockengewicht davon 70-95 Gew.-% von Feststoffen des einzelnen oder komplexen Kräuterextrakts enthält.

**Revendications**

1. Procédé de fabrication d'une formulation de pastilles contenant un extrait d'herbes simple ou complexe, le procédé comprenant :

   (a) la préparation d'un concentré pour graines et d'un concentré pour enrobage en lit fluidisé à partir d'un extrait d'herbes simple ou complexe, dans lequel le concentré pour enrobage en lit fluidisé ayant une teneur en sucre inférieure à celle du concentré pour graines ;
   (b) le moulage d'une graine en mélangeant le concentré pour graines et un excipient ; et
   (c) l'enrobage des surfaces des graines en mélangeant le concentré pour enrobage en lit fluidisé et un excipient,

   et dans lequel l'excipient est au moins un type choisi dans le groupe constitué du carbonate de calcium précipité ($CaCO_3$ précipité), oxyde de magnésium (MgO), silicate de calcium ($CaSiO_3$), silicate de magnésium ($3MgSiO_3$-$5H_2O$), dioxyde de silicium ($SiO_2$), dioxyde de titane ($TiO_2$), bentonite, kaolin, talc, CMC-Ca, caséine, dextrine, hydroxypropylméthylcellulose (HPMC), pullulane, cellulose microcristalline (MCC), lactose, acide stéarique-magnésium (stéarate-Mg), acide stéarique-calcium ( stéarate-Ca), PEG 6000, poudre de bêta-cyclodextrine, poudre de zéine, succinate d'octényle de sodium d'amidon (SSOC) et esters d'acides gras de saccharose.

2. Procédé selon la revendication 1, dans lequel lorsqu'un extrait aqueux 10 fois supérieur de la plante médicinale simple ou complexe a une teneur en sucre de 5,5 ou plus et inférieure à 10,0 Brix à l'étape (a), le concentré pour graines à l'étape (a) est préparé en concentrant l'extrait d'herbes simple ou complexe à une teneur en sucre de 45 à 65 Brix.

3. Procédé selon la revendication 1, dans lequel lorsqu'un extrait aqueux 10 fois supérieur de la plante médicinale simple ou complexe a une teneur en sucre de 3,0 ou plus et inférieure à 5,5 Brix à l'étape (a), le concentré pour graines à l'étape (a) est préparé en concentrant l'extrait d'herbes simple ou complexe à une teneur en sucre de 40 à 60 Brix.

4. Procédé selon la revendication 1, dans lequel lorsqu'un extrait aqueux 10 fois supérieur de la plante médicinale simple ou complexe a une teneur en sucre de 0,01 ou plus et inférieure à 3,0 Brix à l'étape (a), le concentré pour graines à l'étape (a) est préparé en concentrant l'extrait d'herbes simple ou complexe à une teneur en sucre de 30 à 60 Brix.

5. Procédé selon la revendication 1, dans lequel lorsqu'un extrait aqueux 10 fois supérieur de la plante médicinale simple ou complexe a une teneur en sucre de 5,5 ou plus et inférieure à 10,0 Brix à l'étape (a), le concentré pour enrobage en lit fluidisé à l'étape (a) est préparé en concentrant l'extrait d'herbes simple ou complexe à une teneur en sucre de 15 à 35 Brix.

6. Procédé selon la revendication 1, dans lequel lorsqu'un extrait aqueux 10 fois supérieur de la plante médicinale simple ou complexe a une teneur en sucre de 3,0 ou plus et inférieure à 5,5 Brix à l'étape (a), le concentré pour enrobage en lit fluidisé à l'étape (a) est préparé en concentrant l'extrait d'herbes simple ou complexe à une teneur en sucre de 25 à 45 Brix.

7. Procédé selon la revendication 1, dans lequel lorsqu'un extrait aqueux 10 fois supérieur de la plante médicinale simple ou complexe a une teneur en sucre de 0,01 ou plus et inférieure à 3,0 Brix à l'étape (a), le concentré pour enrobage en lit fluidisé à l'étape (a) est préparé en concentrant l'extrait d'herbes simple ou complexe à une teneur en sucre de 20 à 35 Brix.

8. Procédé selon la revendication 1, dans lequel l'étape (b) comprend : le passage d'un mélange du concentré pour graines et de l'excipient à travers une extrudeuse pour couper un extrudat ; et l'introduction de l'extrudat coupé dans un sphéroniseur pour broyer l'extrudat coupé.

9. Procédé selon la revendication 1, dans lequel lorsqu'un extrait aqueux 10 fois supérieur de la plante médicinale simple ou complexe a une teneur en sucre de 5,5 ou plus et inférieure à 10,0 Brix à l'étape (a), l'étape (b) est réalisée en mélangeant un concentré pour graines, qui contient 35 à 55 % en poids de solides d'extraits d'herbes simples ou complexes par rapport au poids sec total des graines, le reste étant un excipient.

10. Procédé selon la revendication 1, dans lequel lorsqu'un extrait aqueux 10 fois supérieur de la plante médicinale simple ou complexe a une teneur en sucre de 3,0 ou plus et inférieure à 5,5 Brix à l'étape (a), l'étape (b) est réalisée en mélangeant un concentré pour graines, qui contient 45 à 65 % en poids de solides d'extraits d'herbes simple ou complexes par rapport au poids sec total des graines, le reste étant un excipient.

11. Procédé selon la revendication 1, dans lequel lorsqu'un extrait aqueux 10 fois supérieur de la plante médicinale simple ou complexe a une teneur en sucre de 0,01 ou plus et inférieure à 3,0 Brix à l'étape (a), l'étape (b) est réalisée par un concentré pour graines, qui contient 50 à 70 % en poids de solides d'extraits d'herbes simples ou complexes par rapport au poids sec total des graines, le reste étant un excipient.

12. Procédé selon la revendication 1, dans lequel lorsque l'extrait d'herbes simple ou complexe présente une aromaticité, le procédé comprend en outre : l'obtention d'une huile essentielle volatilisée pendant l'extraction et la concentration ; et l'encapsulation de l'huile essentielle.

13. Procédé selon la revendication 1, dans lequel lorsque l'extrait d'herbes simple ou complexe présente une aromaticité, un produit obtenu par encapsulation une huile essentielle de l'extrait présentant une aromaticité est en outre ajouté et mélangé au concentré pour enrobage en lit fluidisé, à l'étape (c).

14. Procédé selon la revendication 1, comprenant en outre l'enrobage des surfaces des graines après l'étape (c).

15. Procédé selon la revendication 1, dans lequel lorsqu'un extrait aqueux 10 fois supérieur de la plante médicinale simple ou complexe a une teneur en sucre de 5,5 ou plus et inférieure à 10,0 Brix à l'étape (a), la formulation de pastilles contient, par rapport au poids sec total, 55 à 80 % en poids de solides d'extraits végétaux simples ou complexes.

16. Procédé selon la revendication 1, dans lequel lorsqu'un extrait aqueux 10 fois supérieur de la plante médicinale simple ou complexe a une teneur en sucre de 3,0 ou plus et inférieure à 5,5 Brix à l'étape (a), la formulation de pastilles contient, par rapport au poids sec total, 60 à 90 % en poids de solides d'extraits de plantes simples ou complexes.

17. Procédé selon la revendication 1, dans lequel lorsqu'un extrait aqueux 10 fois supérieur de la plante médicinale simple ou complexe a une teneur en sucre de 0,01 ou plus et inférieure à 3,0 Brix à l'étape (a), la formulation de pastilles contient, par rapport au poids sec total, 70 à 95 % en poids de solides d'extraits végétaux simples ou complexes.

Fig. 1a

* Conventional seeds
being selected after use

* Conventional seeds
being used

Fig. 1b

* Inventive seeds being used

Fig. 2

* Change from rectangle - cylindrical shape

* Change from square - cylindrical shape

Fig. 3

Extrusion hole diameter
1.0, 1.2, 1.5mm

length 1, 1.5, 2cm

Fig. 4a

Fig. 4c

Fig. 5

Fig. 6

Fig. 7

Fig. 8

Fig. 9

Fig. 10

Fig. 11

**REFERENCES CITED IN THE DESCRIPTION**

**Patent documents cited in the description**

- KR 1020040075804
- KR 1020110105351

- KR 101681235

**Non-patent literature cited in the description**

- *J. Pharm. Soc. Korea,* 1998, vol. 42 (4), 422-430
- *J. Korean Med.,* 2017, vol. 38 (3), 170-184
- *J. Korean Neurol. Assoc.,* 2009, vol. 27 (4), 424-427

- **SAIDA IBRAGIC.** Utilisation of a sample and fast HPLC-UV method for separation and quantification of Ephedrine alkaloids in herb of different Ephedra Species. *Research & Reviews: Journal of Pharmacology and Toxicological Studies,* 2017, vol. 5 (2), 7-10